# EUROPEAN PATENT APPLICATION

(11) **EP 4 599 832 A1**
(43) Date of publication of application: **13.08.2025**
(21) Application number: 23874940.2
(22) Date of filing: 05.10.2023
(51) Int. Cl.: A61K 31/47, A61K 31/4709, A61K 31/473, A61K 31/506, A61K 31/5377, A61K 31/5383, A61P 9/00, A61P 9/04, C07D 215/26, C07D 215/28, C07D 401/04, C07D 401/10, C07D 401/12, C07D 401/14, C07D 405/06, C07D 405/12, C07D 413/14, C07D 417/10, C07D 417/12, C07D 455/04, C07D 471/04

(54) **8-HYDROXYQUINOLINE COMPOUND AND USE THEREOF**

(30) Priority: 07.10.2022 JP 2022162292
(71) Applicant: OSAKA UNIVERSITY, Suita-shi, Osaka 565-0871 (JP)
(72) Inventor: TSUKAMOTO, Osamu, Suita-shi, Osaka 565-0871 (JP); TAKASHIMA, Seiji, Suita-shi, Osaka 565-0871 (JP); HARUTA, Junichi, Suita-shi, Osaka 565-0871 (JP); KANAI, Toshio, Suita-shi, Osaka 565-0871 (JP)
(74) Representative: Marks & Clerk LLP
(86) International application number: PCT/JP2023/036369
(87) International publication number: WO 2024/075815

(57) **Abstract**

An object of the present invention is to provide a cMLCK activator that can enhance myocardial contractility without an increase in cellular calcium concentration. This object is achieved by a cMLCK activator containing an 8-hydroxyquinoline compound represented by the following formula (I) or a pharmaceutically acceptable salt thereof, or a solvate thereof, 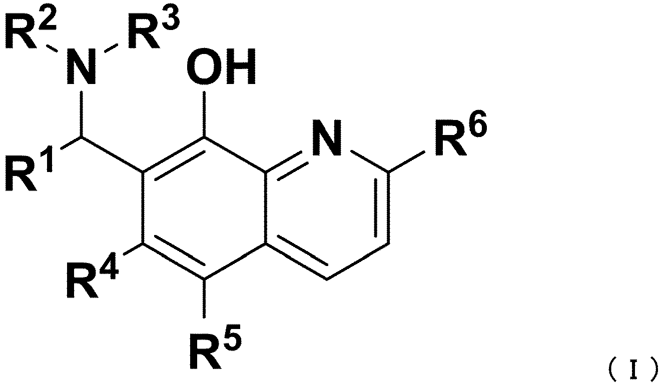 wherein
R¹ represents hydrogen or the like;
R² represents R⁷CO- (R⁷ is C₁₋₆ alkyl or the like) or the like;
R³ represents hydrogen; and
R⁴, R⁵, and R⁶ represent hydrogen or the like.

## Description

### Technical Field

The present invention relates to an 8-hydroxyquinoline compound and use thereof.

### Background Art

The number of patients with heart failure is rapidly increasing worldwide, and is expected to reach 8 million in the United States by 2030. In Japan as well, the number of patients with severe heart failure is steadily increasing due to the aging population as well as an increase in the survival rate of patients with heart disease who could not be previously saved. Furthermore, it is worth noting that the life prognosis of patients with heart failure is comparable to that of cancer patients in terms of mortality rate.

Although inotropic agents play an important role in the treatment of severe heart failure, there are concerns that conventional inotropic agents (catecholamine and PDE3 inhibitors) may worsen prognosis due to side effects (ischemia, arrhythmia, and sudden death) caused by their mechanism of action via an increase in intracellular calcium concentration. Omecamtiv mecarbil (OM), a recently developed direct myocardial myosin activator, can directly enhance myocardial contractility without an increase in intracellular calcium concentration, and also exhibited the effect of prolonging the time to onset of heart failure without worsening prognosis in a phase 3 clinical trial; however, there are still concerns regarding administration of the activator to patients with ischemic heart failure.

Phosphorylation of myosin regulatory light chain (MLC2) is known to be a physiological factor that controls sarcomere contractility.

### Summary of Invention

### Technical Problem

An object of the present invention is to provide a cMLCK activator that can enhance myocardial contractility without an increase in cellular calcium concentration by activating cardiac-specific myosin regulatory light chain kinase (cMLCK), which is specifically expressed in cardiomyocytes, and increasing phosphorylation of its specific substrate, myosin regulatory light chain (MLC2).

### Solution to Problem

The present inventors conducted extensive research to solve the above problems. In the course of the research, the inventors succeeded in synthesizing an 8-hydroxyquinoline compound having a specific structure, and found that the compound can be a compound that can be suitably used for a cMLCK activator. The inventors also found that analogous compounds of the 8-hydroxyquinoline compound are also compounds that can be suitably used for cMLCK activators. The present invention has been accomplished based on these findings.

The present invention includes, for example, the subject matter described in the following items.

### Item 1.

A cMLCK activator comprising an 8-hydroxyquinoline compound represented by the following formula (I) or a pharmaceutically acceptable salt thereof, or a solvate thereof, 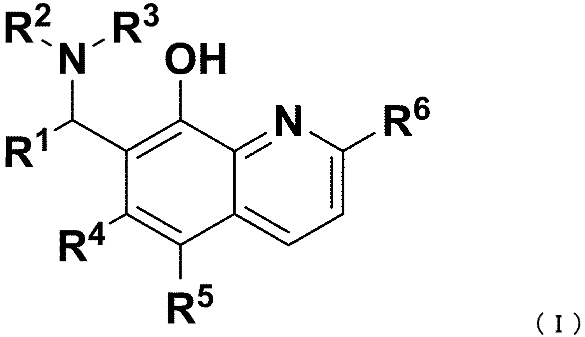 wherein
R¹ represents hydrogen, aryl, a heterocyclic group, C₁₋₆ alkyl optionally substituted with aryl, C₃₋₈ cycloalkyl, C₂₋₆ alkenyl optionally substituted with aryl or heteroaryl, C₃₋₈ cycloalkenyl, or C₂₋₆ alkynyl optionally substituted with aryl;
R² represents hydrogen, R⁷CO-, or heteroaryl;
R³ represents hydrogen;
   wherein R⁷ represents hydrogen, C₁₋₆ alkyl optionally substituted with aryl or heteroaryl, C₁₋₆ alkylamino, C₃₋₈ cycloalkyl that may contain oxygen in a cycloalkyl ring, C₃₋₈ cycloalkylamino, C₁₋₆ alkoxy optionally substituted with aryl, C₃₋₈ cycloalkoxy, C₁₋₃ alkoxy-C₁₋₃ alkyl, C₁₋₄ alkoxycarbonyl C₁₋₄ alkyl, aryl, aryloxy, pyridyl, or amino;
   R¹ and R², taken together with the nitrogen to which they are attached, may form an oxindole ring; and
   R² and R³, taken together with the nitrogen to which they are attached, via -CH₂CH₂X(R⁸)CH₂CH₂-, may form a ring, wherein X represents -CH or nitrogen, and R⁸ represents hydrogen, aryl C₁₋₆ alkyl, or aryl C₁₋ₑ alkoxycarbonyl;
R⁴ represents hydrogen, C₁₋ₑ alkyl, or halogen;
R⁵ represents hydrogen, nitro, halogen, or sulfonyl; and
R⁶ represents hydrogen or C₁₋₆ alkyl.

### Item 2.

A pharmaceutical composition for treating heart disease, comprising the cMLCK activator of Item 1.

### Item 3.

The pharmaceutical composition according to Item 2, for use as a therapeutic agent for heart failure.

### Item 4.

The pharmaceutical composition according to Item 2, for use as an inotropic agent.

### Item 5.

The pharmaceutical composition according to Item 2, for use as a drug for enhancing myocardial contractility.

### Item 6.

The pharmaceutical composition according to Item 2, for use as a drug for improving cardiac function.

### Item 7.

The pharmaceutical composition according to Item 2, for use in promoting organization of a sarcomere structure.

### Item 8.

An 8-hydroxyquinoline compound represented by the following formula (Ia) or a pharmaceutically acceptable salt thereof, or a solvate thereof, 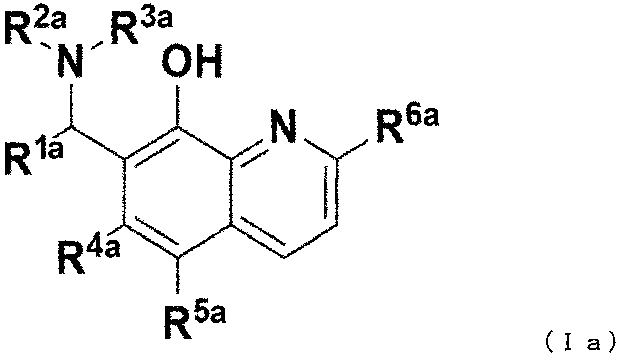 wherein
R^{1a} represents hydrogen, aryl, a heterocyclic group, aryl C₁₋₆ alkyl, C₃₋₈ cycloalkyl, C₂₋₆ alkenyl optionally substituted with aryl or heteroaryl, C₃₋₈ cycloalkenyl, or aryl C₂₋₆ alkynyl;
R^{2a} represents R^{7a}CO- or thiazolyl;
R^{3a} represents hydrogen;
   wherein R^{7a} represents hydrogen, C₁₋₆ alkyl optionally substituted with aryl or heteroaryl, C₁₋₆ alkylamino, C₃₋₈ cycloalkyl that may contain oxygen in a cycloalkyl ring, C₃₋₈ cycloalkylamino, C₁₋₆ alkoxy optionally substituted with aryl, C₃₋₈ cycloalkoxy, C₁₋₃ alkoxy-C₁₋₃ alkyl, C₁₋₄ alkoxycarbonyl C₁₋₄ alkyl, aryloxy, pyridyl, or amino;
   R^{1a} and R^{2a}, taken together with the nitrogen to which they are attached, may form an oxindole ring, and
   R^{2a} and R^{3a}, taken together with the nitrogen to which they are attached, via -CH₂CH₂X(R⁸)CH₂CH₂-, may form a ring, wherein X represents nitrogen, and R⁸ represents aryl C₁₋₆ alkoxycarbonyl;
R^{4a} represents hydrogen, C₁₋₆ alkyl, or halogen;
R^{5a} represents hydrogen, nitro, halogen, or sulfonyl; and
R^{6a} represents hydrogen or C₁₋₆ alkyl.

### Item 9.

Use of an 8-hydroxyquinoline compound represented by the following formula (I) or a pharmaceutically acceptable salt thereof, or a solvate thereof for the production of a cMLCK activator, 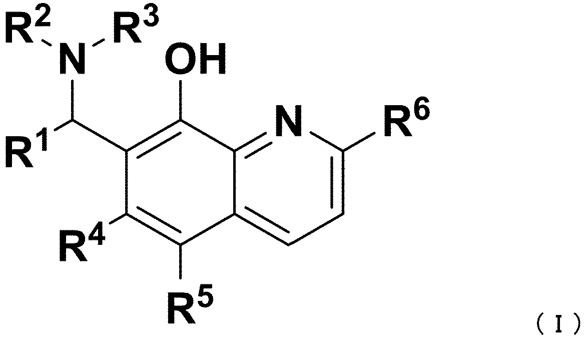 wherein
R¹ represents hydrogen, aryl, a heterocyclic group, C₁₋₆ alkyl optionally substituted with aryl, C₃₋₈ cycloalkyl, C₂₋₆ alkenyl optionally substituted with aryl or heteroaryl, C₃₋₈ cycloalkenyl, or C₂₋₆ alkynyl optionally substituted with aryl;
R² represents hydrogen, R⁷CO-, or heteroaryl;
R³ represents hydrogen;
   wherein R⁷ represents hydrogen, C₁₋₆ alkyl optionally substituted with aryl or heteroaryl, C₁₋₆ alkylamino, C₃₋₈ cycloalkyl that may contain oxygen in a cycloalkyl ring, C₃₋₈ cycloalkylamino, C₁₋₆ alkoxy optionally substituted with aryl, C₃₋₈ cycloalkoxy, C₁₋₃ alkoxy-C₁₋₃ alkyl, C₁₋₄ alkoxycarbonyl C₁₋₄ alkyl, aryl, aryloxy, pyridyl, or amino;
   R¹ and R², taken together with the nitrogen to which they are attached, may form an oxindole ring; and
   R² and R³, taken together with the nitrogen to which they are attached, via -CH₂CH₂X(R⁸)CH₂CH₂-, may form a ring, wherein X represents -CH or nitrogen, and R⁸ represents hydrogen, aryl C₁₋₆ alkyl, or aryl C₁₋₆ alkoxycarbonyl;
R⁴ represents hydrogen, C₁₋₆ alkyl, or halogen;
R⁵ represents hydrogen, nitro, halogen, or sulfonyl; and
R⁶ represents hydrogen or C₁₋₆ alkyl.

### Item 10.

A method of treatment, comprising administering an 8-hydroxyquinoline compound of formula (I) or a pharmaceutically acceptable salt thereof, or a solvate thereof to a subject in need of activation of cMLCK.

### Item 1A.

A method for activating cMLCK, the method comprising administering an 8-hydroxyquinoline compound represented by the following formula (I) or a pharmaceutically acceptable salt thereof, or a solvate thereof to a subject in need of activation of cMLCK or causing a subject in need of activation of cMLCK to ingest an 8-hydroxyquinoline compound represented by the following formula (I) or a pharmaceutically acceptable salt thereof, or a solvate thereof, 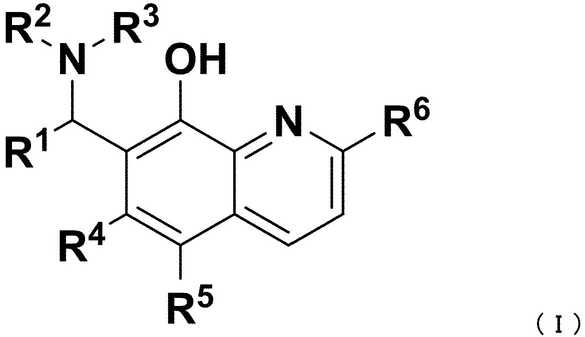 wherein
R¹ represents hydrogen, aryl, a heterocyclic group, C₁₋₆ alkyl optionally substituted with aryl, C₃₋₈ cycloalkyl, C₂₋₆ alkenyl optionally substituted with aryl or heteroaryl, C₃₋₈ cycloalkenyl, or C₂₋₆ alkynyl optionally substituted with aryl;
R² represents hydrogen, R⁷CO-, or heteroaryl;
R³ represents hydrogen;
   wherein R⁷ represents hydrogen, C₁₋₆ alkyl optionally substituted with aryl or heteroaryl, C₁₋₆ alkylamino, C₃₋₈ cycloalkyl that may contain oxygen in a cycloalkyl ring, C₃₋₈ cycloalkylamino, C₁₋₆ alkoxy optionally substituted with aryl, C₃₋₈ cycloalkoxy, C₁₋₃ alkoxy-C₁₋₃ alkyl, C₁₋₄ alkoxycarbonyl C₁₋₄ alkyl, aryl, aryloxy, pyridyl, or amino;
   R¹ and R², taken together with the nitrogen to which they are attached, may form an oxindole ring; and
   R² and R³, taken together with the nitrogen to which they are attached, via -CH₂CH₂X(R⁸)CH₂CH₂-, may form a ring, wherein X represents -CH or nitrogen, and R⁸ represents hydrogen, aryl C₁₋₆ alkyl, or aryl C₁₋ₑ alkoxycarbonyl;
R⁴ represents hydrogen, C₁₋ₑ alkyl, or halogen;
R⁵ represents hydrogen, nitro, halogen, or sulfonyl; and
R⁶ represents hydrogen or C₁₋₆ alkyl.

### Item 2A.

A method for treating heart disease, the method comprising administering the 8-hydroxyquinoline compound or a pharmaceutically acceptable salt thereof, or a solvate thereof of Item 1 to a subject with heart disease or causing a subject with heart disease to ingest the 8-hydroxyquinoline compound or a pharmaceutically acceptable salt thereof, or a solvate thereof of Item 1.

### Item 3A.

A method for treating heart failure, the method comprising administering the 8-hydroxyquinoline compound or a pharmaceutically acceptable salt thereof, or a solvate thereof of Item 1 to a subject with heart failure or causing a subject with heart failure to ingest the 8-hydroxyquinoline compound or a pharmaceutically acceptable salt thereof, or a solvate thereof of Item 1.

### Item 4A.

An inotropic method comprising administering the 8-hydroxyquinoline compound or a pharmaceutically acceptable salt thereof, or a solvate thereof of Item 1 to a subject in need of inotropic support or causing a subject in need of inotropic support to ingest the 8-hydroxyquinoline compound or a pharmaceutically acceptable salt thereof, or a solvate thereof of Item 1.

### Item 5A.

A method for enhancing myocardial contractility, the method comprising administering the 8-hydroxyquinoline compound or a pharmaceutically acceptable salt thereof, or a solvate thereof of Item 1 to a subject in need of enhancement of myocardial contractility or causing a subject in need of enhancement of myocardial contractility to ingest the 8-hydroxyquinoline compound or a pharmaceutically acceptable salt thereof, or a solvate thereof of Item 1.

### Item 6A.

A method for improving cardiac function, the method comprising administering the 8-hydroxyquinoline compound or a pharmaceutically acceptable salt thereof, or a solvate thereof of Item 1 to a subject in need of improvement of cardiac function or causing a subject in need of improvement of cardiac function to ingest the 8-hydroxyquinoline compound or a pharmaceutically acceptable salt thereof, or a solvate thereof of Item 1.

### Item 7A.

A method for promoting organization of a sarcomere structure, the method comprising administering the 8-hydroxyquinoline compound or a pharmaceutically acceptable salt thereof, or a solvate thereof of Item 1 to a subject in need of promotion of organization of a sarcomere structure or causing a subject in need of promotion of organization of a sarcomere structure to ingest the 8-hydroxyquinoline compound or a pharmaceutically acceptable salt thereof, or a solvate thereof of Item 1.

### Item 1B.

An 8-hydroxyquinoline compound represented by the following formula (I) or a pharmaceutically acceptable salt thereof, or a solvate thereof, for use as a cMLCK activator, 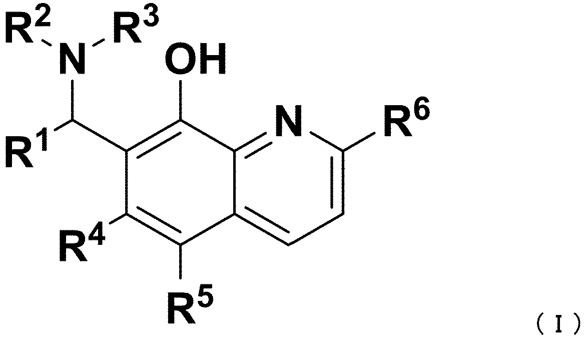 wherein
R¹ represents hydrogen, aryl, a heterocyclic group, C₁₋₆ alkyl optionally substituted with aryl, C₃₋₈ cycloalkyl, C₂₋₆ alkenyl optionally substituted with aryl or heteroaryl, C₃₋₈ cycloalkenyl, or C₂₋₆ alkynyl optionally substituted with aryl;
R² represents hydrogen, R⁷CO-, or heteroaryl;
R³ represents hydrogen;
   wherein R⁷ represents hydrogen, C₁₋₆ alkyl optionally substituted with aryl or heteroaryl, C₁₋₆ alkylamino, C₃₋₈ cycloalkyl that may contain oxygen in a cycloalkyl ring, C₃₋₈ cycloalkylamino, C₁₋₆ alkoxy optionally substituted with aryl, C₃₋₈ cycloalkoxy, C₁₋₃ alkoxy-C₁₋₃ alkyl, C₁₋₄ alkoxycarbonyl C₁₋₄ alkyl, aryl, aryloxy, pyridyl, or amino;
   R¹ and R², taken together with the nitrogen to which they are attached, may form an oxindole ring; and
   R² and R³, taken together with the nitrogen to which they are attached, via -CH₂CH₂X(R⁸)CH₂CH₂-, may form a ring, wherein X represents -CH or nitrogen, and R⁸ represents hydrogen, aryl C₁₋₆ alkyl, or aryl C₁₋ₑ alkoxycarbonyl;
R⁴ represents hydrogen, C₁₋ₑ alkyl, or halogen;
R⁵ represents hydrogen, nitro, halogen, or sulfonyl; and
R⁶ represents hydrogen or C₁₋₆ alkyl.

### Item 2B.

The 8-hydroxyquinoline compound or a pharmaceutically acceptable salt thereof, or a solvate thereof according to Item 1, for use as a pharmaceutical composition for treating heart disease.

### Item 3B.

The 8-hydroxyquinoline compound or a pharmaceutically acceptable salt thereof, or a solvate thereof according to Item 1, for use as a therapeutic agent for heart failure.

### Item 4B.

The 8-hydroxyquinoline compound or a pharmaceutically acceptable salt thereof, or a solvate thereof according to Item 1, for use as an inotropic agent.

### Item 5B.

The 8-hydroxyquinoline compound or a pharmaceutically acceptable salt thereof, or a solvate thereof according to Item 1, for use as a drug for enhancing myocardial contractility.

### Item 6B.

The 8-hydroxyquinoline compound or a pharmaceutically acceptable salt thereof, or a solvate thereof according to Item 1, for use as a drug for improving cardiac function.

### Item 7B.

The 8-hydroxyquinoline compound or a pharmaceutically acceptable salt thereof, or a solvate thereof according to Item 1, for use as a composition for promoting organization of a sarcomere structure.

### Item 2C.

Use of the 8-hydroxyquinoline compound or a pharmaceutically acceptable salt thereof, or a solvate thereof of Item 1, for the production of a pharmaceutical composition for treating heart disease.

### Item 3C.

Use of the 8-hydroxyquinoline compound or a pharmaceutically acceptable salt thereof, or a solvate thereof of Item 1, for the production of a therapeutic agent for heart failure.

### Item 4C.

Use of the 8-hydroxyquinoline compound or a pharmaceutically acceptable salt thereof, or a solvate thereof of Item 1, for the production of an inotropic agent.

### Item 5C.

Use of the 8-hydroxyquinoline compound or a pharmaceutically acceptable salt thereof, or a solvate thereof of Item 1, for the production of a drug for enhancing myocardial contractility.

### Item 6C.

Use of the 8-hydroxyquinoline compound or a pharmaceutically acceptable salt thereof, or a solvate thereof of Item 1, for the production of a drug for improving cardiac function.

### Item 7C.

Use of the 8-hydroxyquinoline compound or a pharmaceutically acceptable salt thereof, or a solvate thereof of Item 1, for the production of a composition for promoting organization of a sarcomere structure.

### Item 1D.

Use of an 8-hydroxyquinoline compound represented by the following formula (I) or a pharmaceutically acceptable salt thereof, or a solvate thereof, as a cMLCK activator, 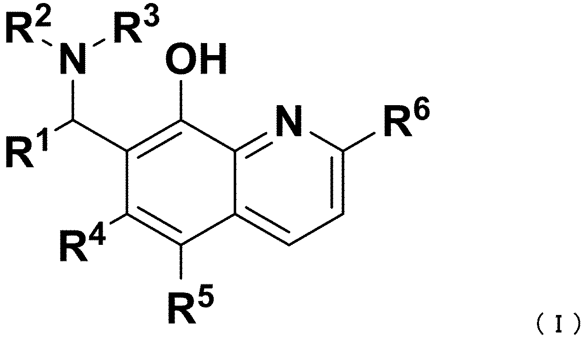 wherein
R¹ represents hydrogen, aryl, a heterocyclic group, C₁₋₆ alkyl optionally substituted with aryl, C₃₋₈ cycloalkyl, C₂₋₆ alkenyl optionally substituted with aryl or heteroaryl, C₃₋₈ cycloalkenyl, or C₂₋₆ alkynyl optionally substituted with aryl;
R² represents hydrogen, R⁷CO-, or heteroaryl;
R³ represents hydrogen;
   wherein R⁷ represents hydrogen, C₁₋₆ alkyl optionally substituted with aryl or heteroaryl, C₁₋₆ alkylamino, C₃₋₈ cycloalkyl that may contain oxygen in a cycloalkyl ring, C₃₋₈ cycloalkylamino, C₁₋₆ alkoxy optionally substituted with aryl, C₃₋₈ cycloalkoxy, C₁₋₃ alkoxy-C₁₋₃ alkyl, C₁₋₄ alkoxycarbonyl C₁₋₄ alkyl, aryl, aryloxy, pyridyl, or amino;
   R¹ and R², taken together with the nitrogen to which they are attached, may form an oxindole ring; and
   R² and R³, taken together with the nitrogen to which they are attached, via -CH₂CH₂X(R⁸)CH₂CH₂-, may form a ring, wherein X represents -CH or nitrogen, and R⁸ represents hydrogen, aryl C₁₋₆ alkyl, or aryl C₁₋₆ alkoxycarbonyl;
R⁴ represents hydrogen, C₁₋₆ alkyl, or halogen;
R⁵ represents hydrogen, nitro, halogen, or sulfonyl; and
R⁶ represents hydrogen or C₁₋₆ alkyl.

### Item 2D.

Use of the 8-hydroxyquinoline compound or a pharmaceutically acceptable salt thereof, or a solvate thereof of Item 1 as a pharmaceutical composition for treating heart disease.

### Item 3D.

Use of the 8-hydroxyquinoline compound or a pharmaceutically acceptable salt thereof, or a solvate thereof of Item 1 as a therapeutic agent for heart failure.

### Item 4D.

Use of the 8-hydroxyquinoline compound or a pharmaceutically acceptable salt thereof, or a solvate thereof of Item 1 as an inotropic agent.

### Item 5D.

Use of the 8-hydroxyquinoline compound or a pharmaceutically acceptable salt thereof, or a solvate thereof of Item 1 as a drug for enhancing myocardial contractility.

### Item 6D.

Use of the 8-hydroxyquinoline compound or a pharmaceutically acceptable salt thereof, or a solvate thereof of Item 1 as a drug for improving cardiac function.

### Item 7D.

Use of the 8-hydroxyquinoline compound or a pharmaceutically acceptable salt thereof, or a solvate thereof of Item 1 as a composition for promoting organization of a sarcomere structure.

### Advantageous Effects of Invention

The present invention provides a cMLCK activator in which an 8-hydroxyquinoline compound represented by formula (I) or a pharmaceutically acceptable salt thereof, or a solvate thereof activates cMLCK and increases phosphorylation of its specific substrate, myosin regulatory light chain (MLC2), thereby enhancing myocardial contractility without an increase in cellular calcium concentration.

The cMLCK activator of the present invention can be a pharmaceutical composition for treating heart disease that is effective as a therapeutic agent for heart failure, an inotropic agent, a drug for enhancing myocardial contractility, a drug for improving cardiac function, etc. Furthermore, the cMLCK activator of the present invention can promote organization of a sarcomere structure.

### Brief Description of Drawings

Fig. 1 shows the results of comparing the cMLCK activity of Synthesis Examples 22 to 28 with the cMLCK activity of Synthesis Example 1 (top), and the results of comparing the cMLCK activity of Synthesis Examples 16, 17, 21, 24, 29, and 30 with the cMLCK activity of Synthesis Example 1 (bottom).
Fig. 2 shows the results of comparing the cMLCK activity of Synthesis Examples 64 to 66 with the cMLCK activity of Synthesis Example 1.
Fig. 3 shows the results of comparing the cMLCK activity of LEU-1128 and Synthesis Examples 84 and 85 with the cMLCK activity of Synthesis Example 1 (top), and the results of comparing the cMLCK activity of Synthesis Examples 86 and 87 with the cMLCK activity of Synthesis Example 1 (bottom).
Fig. 4 shows the reaction velocity of 8-hydroxyquinoline compounds (8HQ compounds) (LEU-1142 and LEU-1170).
Fig. 5 shows the phosphorylation of MLC2v by 8HQ compounds (LEU-1142 and LEU-1170).
Fig. 6 shows the dose-dependent cMLCK activation action of 8HQ compounds (LEU-1142 and LEU-1170).
Fig. 7-1 shows the specificity of 8HQ compounds (LEU-1142 and LEU-1170) for smooth muscle-type MLCK (smMLCK).
Fig. 7-2 shows the specificity of 8HQ compounds (LEU-1142 and LEU-1170) for skeletal muscle-type MLCK (skMLCK).
Fig. 8-1 shows the establishment of (gene-corrected) iPS cardiomyocytes by removing heterozygous cMLCK gene mutation (c1951-1 g>t) using the CRISPR/Cas9 system to normalize iPS cardiomyocytes generated from a patient carrying this mutation.
Fig. 8-2 shows the results of measuring the contraction velocity, relaxation velocity, and developed tension in established (gene-corrected) iPS cardiomyocytes.
Fig. 9-1 shows the results of measuring the dose-dependent activation action, specificity, and enzyme activity of LEU-1154.
Fig. 9-2 shows the results of measuring the contraction velocity, relaxation velocity, and developed tension in (gene-corrected) iPS cardiomyocytes to which LEU-1154 was administered.
Fig. 10 shows the sarcomere structure-improving action of LEU-1154.
Fig. 11 shows the results of measuring calcium dynamics in wild-type human iPS cardiomyocytes treated with LEU-1154. The Y axis of the graphs shows values normalized to the values of the DMSO group (n = 5 in each point).
CTAs: average changes of calcium transient amplitude at systole, PWD50: pulse width duration measured at 50% CTAs

### Description of Embodiments

In the present invention, the 8-hydroxyquinoline compound or a pharmaceutically acceptable salt, or a solvate thereof may be simply referred to as an "8-hydroxyquinoline compound."

In the present specification, the term "comprising" includes "consisting essentially of" and "consisting of." The present specification also encompasses any and all combinations of the technical elements described in the specification.

Embodiments included in the present invention are described in more detail below.

### 1. cMLCK Activator of the Present Invention

The cMLCK activator of the present invention comprises an 8-hydroxyquinoline compound. The 8-hydroxyquinoline compound is represented by the following formula (I): 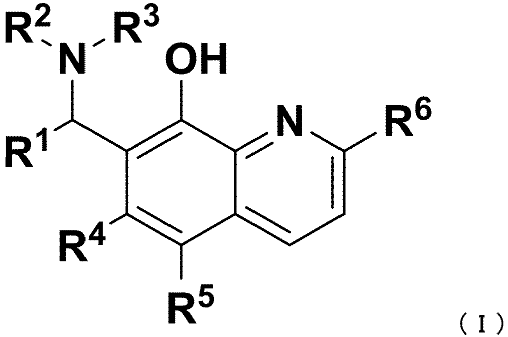 wherein R¹, R², R³, R⁴, R⁵, and R⁶ are as defined above.

Among the compounds represented by formula (I), a compound represented by the following formula (I-A) is preferable, a compound represented by the following formula (I-B) is more preferable, and a compound represented by the following formula (I-C) is particularly preferable. 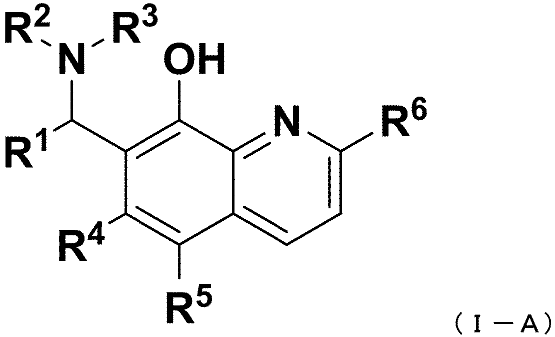 wherein
R¹ represents hydrogen, aryl, a heterocyclic group, aryl C₁₋₆ alkyl, C₃₋₈ cycloalkyl, C₂₋₆ alkenyl optionally substituted with aryl or heteroaryl, C₃₋₈ cycloalkenyl, or C₂₋₆ alkynyl optionally substituted with aryl,
R² represents hydrogen, R⁷CO-, or heteroaryl;
R³ represents hydrogen;
   wherein R⁷ represents hydrogen, C₁₋₆ alkyl optionally substituted with aryl or heteroaryl, C₁₋₆ alkylamino, C₃₋₈ cycloalkyl that may contain oxygen in a cycloalkyl ring, C₃₋₈ cycloalkylamino, C₁₋₆ alkoxy optionally substituted with aryl, C₃₋₈ cycloalkoxy, C₁₋₃ alkoxy-C₁₋₃ alkyl, C₁₋₄ alkoxycarbonyl C₁₋₄ alkyl, aryl, aryloxy, pyridyl, or amino;
   R¹ and R², taken together with the nitrogen to which they are attached, may form an oxindole ring; and
   R² and R³, taken together with the nitrogen to which they are attached, via -CH₂CH₂X(R⁸)CH₂CH₂-, may form a ring, wherein X represents -CH or nitrogen, and R⁸ represents hydrogen, aryl C₁₋₆ alkyl, or aryl C₁₋₆ alkoxycarbonyl;
R⁴ represents hydrogen, C₁₋₆ alkyl, or halogen;
R⁵ represents hydrogen, nitro, halogen, or sulfonyl; and
R⁶ represents hydrogen or C₁₋₆ alkyl. 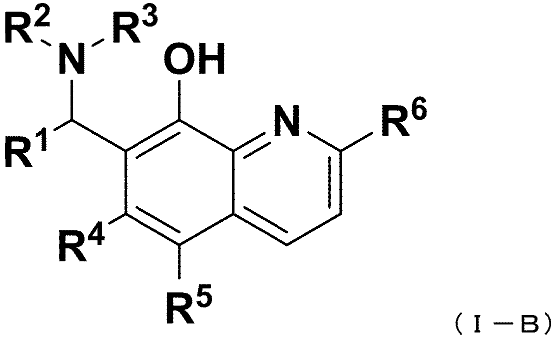
wherein
R¹ represents aryl, a heterocyclic group, aryl C₁₋₆ alkyl, C₂₋₆ alkenyl substituted with aryl or heteroaryl, C₃₋₈ cycloalkenyl, or C₂₋₆ alkynyl optionally substituted with aryl;
R² represents R⁷CO- or heteroaryl;
R³ represents hydrogen;
   wherein R⁷ represents C₁₋₆ alkyl optionally substituted with aryl or heteroaryl, C₁₋₆ alkylamino, C₃₋₈ cycloalkyl that may contain oxygen in a cycloalkyl ring, C₃₋₈ cycloalkylamino, C₁₋₆ alkoxy optionally substituted with aryl, C₃₋₈ cycloalkoxy, C₁₋₃ alkoxy-C₁₋₃ alkyl, aryl, aryloxy, pyridyl, or amino;
   R² and R³, taken together with the nitrogen to which they are attached, via -CH₂CH₂X(R⁸)CH₂CH₂-, may form a ring, wherein X represents nitrogen, and R⁸ represents aryl C₁₋₆ alkoxycarbonyl;
R⁴ represents hydrogen, C₁₋₆ alkyl, or halogen;
R⁵ represents hydrogen, nitro, halogen, or sulfonyl; and
R⁶ represents hydrogen or C₁₋₆ alkyl. 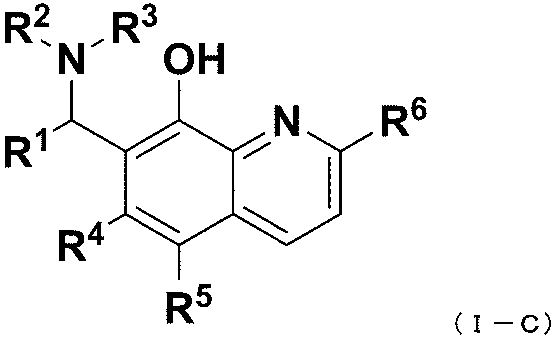
wherein
R¹ represents aryl, a heterocyclic group, aryl C₁₋₆ alkyl, aryl C₂₋₆ alkenyl, C₃₋₈ cycloalkenyl, or C₂₋₆ alkynyl optionally substituted with aryl;
R² represents R⁷CO-;
R³ represents hydrogen;
   wherein R⁷ represents C₁₋₆ alkyl optionally substituted with aryl or heteroaryl, C₁₋₆ alkylamino, C₃₋₈ cycloalkyl, C₁₋₆ alkoxy optionally substituted with aryl, aryl, or aryloxy;
   R² and R³, taken together with the nitrogen to which they are attached, via -CH₂CH₂X(R⁸)CH₂CH₂-, may form a ring, wherein X represents nitrogen, and R⁸ represents aryl C₁₋₆ alkoxycarbonyl;
R⁴ represents hydrogen, C₁₋₆ alkyl, or halogen;
R⁵ represents hydrogen or nitro; and
R⁶ represents hydrogen.

The groups shown in formulas (I), (I-A), (I-B), and (I-C) described above are as follows.

In the present specification, examples of aryl include, but are not particularly limited to, phenyl, naphthyl, and the like.

In the present specification, examples of heterocyclic groups include pyridyl, pyrimidyl, imidazolyl, quinolyl, azaindole, chromenyl, and the like.

In the present specification, the term "C₁₋₆ alkyl" refers to linear or branched alkyl having 1 to 6 carbon atoms, and examples include methyl, ethyl, n-propyl, isopropyl, n-butyl, isobutyl, tert-butyl, sec-butyl, n-pentyl, neopentyl, n-hexyl, isohexyl, 3-methylpentyl, and the like.

In the present specification, the term "C₃₋₈ cycloalkyl" refers to 3-, 4-, 5-, 6-, 7-, or 8-membered cyclic alkyl having 3 to 8 carbon atoms, and examples include cyclopropyl, cyclobutyl, cyclopentyl, cyclohexyl, cycloheptyl, cyclooctyl, 3,4-dimethylcyclopentyl, 3,3-dimethylcyclohexyl, and the like.

In the present specification, examples of heteroaryl include pyridyl, thiazolyl, and the like.

In the present specification, the term "C₂₋₆ alkenyl" refers to linear or branched alkenyl having 2 to 6 carbon atoms and 1 to 3 double bonds, and examples include 2-propenyl, 2-butenyl, 3-butenyl, 4-pentenyl, 3-pentenyl, 5-hexenyl, 4-hexenyl, 3-hexenyl, 1,3-butadienyl, 1,3,5-hexatrienyl, and the like.

In the present specification, the term "C₃₋₈ cycloalkenyl" refers to 3-, 4-, 5-, 6-, 7-, or 8-membered cyclic alkenyl having a double bond and 3 to 8 carbon atoms, and examples include cyclopropenyl, cyclobutenyl, cyclopentenyl, cyclohexenyl, cycloheptenyl, cyclooctenyl, 3,4-dimethylcyclopentenyl, 3,3-dimethylcyclohexenyl, and the like.

In the present specification, the term "C₂₋₆ alkynyl" refers to linear or branched alkynyl having 2 to 6 carbon atoms and 1 to 3 triple bonds, and examples include 2-propynyl, 2-butynyl, 3-butynyl, 4-pentynyl, 3-pentynyl, 5-hexynyl, 4-hexynyl, 3-hexynyl, and the like.

In the present specification, the term "C₁₋₆ alkylamino" refers to linear or branched alkylamino having 1 to 6 carbon atoms, and examples include methylamino, ethylamino, n-propylamino, isopropylamino, n-butylamino, isobutylamino, tert-butylamino, sec-butylamino, n-pentylamino, neopentylamino, n-hexylamino, isohexylamino, 3-methylpentylamino, and the like.

In the present specification, the term "C₃₋₈ cycloalkylamino" refers to 3-, 4-, 5-, 6-, 7-, or 8-membered cyclic alkylamino having 3 to 8 carbon atoms, and examples include cyclopropylamino, cyclobutylamino, cyclopentylamino, cyclohexylamino, cycloheptylamino, cyclooctylamino, 3,4-dimethylcyclopentylamino, 3,3-dimethylcyclohexylamino, and the like.

In the present specification, the term "C₁₋₆ alkoxy" refers to linear or branched alkoxy having 1 to 6 carbon atoms, and examples include methoxy, ethoxy, n-propoxy, isopropoxy, n-butoxy, isobutoxy, tert-butoxy, sec-butoxy, n-pentyloxy, neopentyloxy, n-hexyloxy, isohexyloxy, 3-methylpentyloxy, and the like.

In the present specification, the term "C₃₋₈ cycloalkoxy" refers to C₃₋₈ cycloalkoxy in which the 3-, 4-, 5-, 6-, 7-, or 8-membered cyclic alkyl having 3 to 8 carbon atoms defined above and oxy are bonded to each other, and examples include cyclopropyloxy, cyclobutyloxy, cyclopenthyloxy, cyclohexyloxy, cycloheptyloxy, cyclooctyloxy, and the like.

In the present specification, the term "C₁₋₃ alkoxy" refers to linear or branched alkoxy having 1 to 3 carbon atoms, and examples include methoxy, ethoxy, n-propoxy, isopropoxy, and the like.

In the present specification, the term "C₁₋₃ alkyl" refers to linear or branched alkyl having 1 to 3 carbon atoms, and examples include methyl, ethyl, n-propyl, isopropyl, and the like.

In the present specification, examples of C₁₋₄ alkoxycarbonyl include methoxycarbonyl, ethoxycarbonyl, n-propoxycarbonyl, isopropoxycarbonyl, n-butoxycarbonyl, isobutoxycarbonyl, tert-butoxycarbonyl, sec-butoxycarbonyl, and the like.

In the present specification, the term "C₁₋₄ alkyl" refers to linear or branched alkyl having 1 to 4 carbon atoms, and examples include methyl, ethyl, n-propyl, isopropyl, n-butyl, isobutyl, tert-butyl, sec-butyl, and the like.

In the present specification, examples of aryloxy include phenoxy, naphthyloxy, and the like.

In the present specification, the term "aryl C₁₋₆ alkyl" refers to C₁₋₆ alkyl substituted with aryl, and examples include phenylmethyl, naphthylmethyl, 1-phenylethyl, 2-phenylethyl, 2-naphthylethyl, 1-phenylpropyl, 2-phenylpropyl, 3-phenylpropyl, (2-phenyl-1-methyl)ethyl, 1-phenylbutyl, 2-phenylbutyl, 3-phenylbutyl, 4-phenylbutyl, (3-phenyl-1-methyl)propyl, 5-phenylpentyl, 6-phenylhexyl, and the like.

In the present specification, the term "aryl C₁₋₆ alkoxycarbonyl" refers to C₁₋₆ alkoxycarbonyl substituted with aryl, and examples include phenylmethoxycarbonyl, 1-phenylethoxycarbonyl, 2-phenylethoxycarbonyl, 1-phenylpropoxycarbonyl, 2-phenylpropoxycarbonyl, 3-phenylpropoxycarbonyl, 4-phenylbutoxycarbonyl, 5-phenylpentyloxycarbonyl, 6-phenylhexyloxycarbonyl, naphthylmethylcarbonyl, 2-naphthylethoxycarbonyl, and the like.

In the present specification, examples of halogen include fluorine, chlorine, bromine, and iodine.

In the present invention, the aryl represented by R¹ is not particularly limited, and examples include phenyl, naphthyl, and the like. In the present invention, the aryl is preferably phenyl.

The aryl represented by R¹ described above may be unsubstituted or may have at least one substituent. Examples of substituents include, but are not particularly limited to, oxygen, halogen, hydroxy, amino, nitro, cyano, C₁₋₆ alkyl, halo-C₁₋₆ alkyl, C₁₋₆ alkoxy, halo-C₁₋₆ alkoxy, C₂₋₆ alkenyl, halo-C₂₋₆ alkenyl, C₂₋₆ alkynyl, halo-C₂₋₆ alkynyl, phenyl, phenoxy, pyridyl, pyrazolyl, imidazolyl, triazolyl, thiazolyl, and the like. The number of substituents is generally 1 to 5, preferably 1 to 3, and more preferably 1 or 2.

In the present invention, the aryl represented by R¹ is preferably unsubstituted phenyl or phenyl having at least one substituent selected from the group consisting of halogen, hydroxy, nitro, halo-C₁₋₆ alkyl, C₁₋₆ alkoxy, phenyl, phenoxy, pyridyl, pyrazolyl, imidazolyl, triazolyl, and thiazolyl.

In the present invention, the aryl represented by R¹ is more preferably unsubstituted phenyl or phenyl having at least one substituent selected from the group consisting of halogen, nitro, halo-C₁₋₆ alkyl, C₁₋₆ alkoxy, phenyl, phenoxy, pyridyl, pyrazolyl, imidazolyl, triazolyl, and thiazolyl.

In the present invention, the aryl represented by R¹ is even more preferably phenyl having at least one substituent selected from the group consisting of halogen, halo-C₁₋₆ alkyl, C₁₋₆ alkoxy, phenyl, phenoxy, pyridyl, pyrazolyl, imidazolyl, and triazolyl.

In the present invention, examples of the heterocyclic group represented by R¹ include pyridyl, pyrimidyl, imidazolyl, quinolyl, azaindole, chromenyl, and the like. In the present invention, the heterocyclic group is preferably pyridyl, pyrimidyl, imidazolyl, quinolyl, azaindole, or chromenyl. In the present invention, the heterocyclic group is more preferably pyridyl, pyrimidyl, quinolyl, azaindole, or chromenyl. In the present invention, the heterocyclic group is even more preferably pyridyl, quinolyl, azaindole, or chromenyl.

The heterocyclic group represented by R¹ described above may be unsubstituted or may have at least one substituent. Examples of substituents include, but are not particularly limited to, oxygen, oxo, halogen, hydroxy, amino, nitro, cyano, C₁₋ₑ alkyl, halo-C₁₋₆ alkyl, C₁₋₆ alkoxy, halo-C₁₋₆ alkoxy, C₂₋₆ alkenyl, halo-C₂₋₆ alkenyl, C₂₋₆ alkynyl, halo-C₂₋₆ alkynyl, phenyl, phenoxy, tolyl, pyrazolyl, imidazolyl, morpholino, and the like. The amino may be cyclic amino. The heterocyclic group may have a saturated cyclo ring fused thereto.

In the present invention, the heterocyclic group represented by R¹ is preferably unsubstituted pyridyl or pyridyl having at least one substituent selected from the group consisting of halogen, amino (including cyclic amino), C₁₋₆ alkyl, halo-C₁₋₆ alkyl, C₁₋₆ alkoxy, phenyl, phenoxy, tolyl, pyrazolyl, imidazolyl, and morpholino; unsubstituted pyrimidyl or pyrimidyl substituted with phenyl; unsubstituted imidazolyl; unsubstituted quinolyl; quinolyl in which a saturated cyclo ring is fused and which is substituted with oxo; unsubstituted azaindole; or unsubstituted chromenyl.

In the present invention, the heterocyclic group represented by R¹ is more preferably pyridyl having at least one substituent selected from the group consisting of halogen, amino (including cyclic amino), C₁₋₆ alkyl, halo-C₁₋₆ alkyl, C₁₋₆ alkoxy, phenyl, phenoxy, tolyl, pyrazolyl, imidazolyl, and morpholino; pyrimidyl substituted with phenyl; unsubstituted quinolyl; quinolyl in which a saturated cyclo ring is fused and which is substituted with oxo; unsubstituted azaindole; or unsubstituted chromenyl.

In the present invention, the heterocyclic group represented by R¹ is even more preferably pyridyl having at least one substituent selected from the group consisting of amino (including cyclic amino), C₁₋₆ alkyl, halo-C₁₋₆ alkyl, C₁₋₆ alkoxy, phenyl, phenoxy, tolyl, pyrazolyl, and morpholino; unsubstituted quinolyl; quinolyl in which a saturated cyclo ring is fused and which is substituted with oxo; unsubstituted azaindole; or unsubstituted chromenyl.

In the present invention, when the heterocyclic group represented by R¹ is pyridyl, it is preferably pyridyl having an electron-withdrawing group on the pyridyl ring, from the viewpoint of increasing the activity of the 8-hydroxyquinoline compound of the present invention. Examples of electron-withdrawing groups include trifluoromethyl, trifluoromethylphenyl, chloro, pyrazolyl, phenyl, tolyl, morpholino, imidazolyl, and the like.

The aryl constituting the C₁₋₆ alkyl optionally substituted with aryl, the C₂₋₆ alkenyl optionally substituted with aryl, and the C₂₋₆ alkynyl optionally substituted with aryl, represented by R¹ is not particularly limited, and examples include phenyl, naphthyl, and the like.

The aryl constituting the C₁₋₆ alkyl optionally substituted with aryl, the C₂₋₆ alkenyl optionally substituted with aryl, and the C₂₋₆ alkynyl optionally substituted with aryl, represented by R¹ may be unsubstituted or may have at least one (generally 1 to 5, preferably 1 to 3, more preferably 1 or 2) substituent. Examples of substituents include, but are not particularly limited to, oxygen, halogen, hydroxy, amino, nitro, cyano, C₁₋₆ alkyl, halo-C₁₋₆ alkyl, C₁₋₆ alkoxy, halo-C₁₋₆ alkoxy, phenyl, pyridyl, and the like.

In the present invention, the C₁₋₆ alkyl optionally substituted with aryl represented by R¹ is preferably C₁₋₆ alkyl substituted with aryl. The aryl is preferably phenyl.

In the present invention, the C₂₋₆ alkenyl optionally substituted with aryl or heteroaryl represented by R¹ is preferably C₂₋₆ alkenyl substituted with aryl. The aryl is preferably phenyl.

In the present invention, the C₂₋₆ alkynyl optionally substituted with aryl represented by R¹ is preferably C₂₋₆ alkynyl substituted with aryl. The aryl is preferably phenyl.

The heteroaryl constituting the heteroaryl C₂₋₆ alkenyl represented by R¹ is not particularly limited, and examples include pyridyl and the like.

The heteroaryl constituting the heteroaryl C₂₋₆ alkenyl represented by R¹ may be unsubstituted or may have at least one substituent. Examples of substituents include, but are not particularly limited to, halogen, cyano, nitro, halo-C₁₋₆ alkyl, C₁₋₆ alkoxy, halo-C₁₋₆ alkoxy, phenyl, pyridyl, imidazolyl, and the like.

In the present invention, the heteroaryl C₂₋₆ alkenyl represented by R¹ is preferably unsubstituted pyridylethenyl or pyridylethenyl substituted with imidazolyl.

In the present invention, the heteroaryl C₂₋₆ alkenyl represented by R¹ is more preferably pyridylethenyl substituted with imidazolyl.

In the present invention, among the C₃₋₈ cycloalkyl groups represented by R¹, C₅₋₇ cycloalkyl is more preferable.

In the present invention, among the C₃₋₈ cycloalkenyl groups represented by R¹, C₅₋₇ cycloalkenyl is more preferable.

In the present invention, in R⁷CO- represented by R², the C₁₋₆ alkyl, the C₃₋₈ cycloalkyl, the C₁₋₆ alkylamino, the C₁₋₆ alkoxy, or the arylalkoxy represented by R⁷ is preferably one in which the carbon chain of the alkyl portion is longer, and more preferably one having about 3 to 5 carbon atoms.

In the present invention, the heteroaryl represented by R² is not particularly limited, and examples include pyridyl, thiazolyl, and the like.

In the present invention, the heteroaryl represented by R² is preferably pyridyl or thiazolyl.

The heteroaryl represented by R² described above may be unsubstituted or may have at least one substituent. Examples of substituents include, but are not particularly limited to, oxygen, halogen, hydroxy, amino, nitro, cyano, C₁₋₆ alkyl, halo-C₁₋₆ alkyl, C₁₋₆ alkoxy, halo-C₁₋₆ alkoxy, C₂₋₆ alkenyl, halo-C₂₋₆ alkenyl, C₂₋₆ alkynyl, halo-C₂₋₆ alkynyl, phenyl, phenoxy, pyridyl, pyrazolyl, imidazolyl, triazolyl, thiazolyl, and the like.

In the present invention, the heteroaryl represented by R² is preferably unsubstituted pyridyl or pyridyl substituted with hydroxy; or unsubstituted thiazolyl. In the present invention, the heteroaryl represented by R² is more preferably unsubstituted pyridyl or unsubstituted thiazolyl.

In the present invention, the C₁₋₆ alkyl, the C₁₋₆ alkylamino, the C₃₋₈ cycloalkyl, the C₃₋₈ cycloalkylamino, the C₁₋₆ alkoxy, the C₃₋₈ cycloalkoxy, the C₁₋₃ alkoxy-C₁₋₃ alkyl, the C₁₋₄ alkoxycarbonyl C₁₋₄ alkyl, and the pyridyl represented by R⁷ may be unsubstituted or may have at least one substituent. Examples of substituents include, but are not particularly limited to, oxygen, halogen, hydroxy, amino, nitro, cyano, C₁₋₆ alkyl, halo-C₁₋₆ alkyl, C₁₋₆ alkoxy, halo-C₁₋₆ alkoxy, C₂₋₆ alkenyl, halo-C₂₋₆ alkenyl, C₂₋₆ alkynyl, halo-C₂₋₆ alkynyl, phenyl, phenoxy, pyridyl, pyrazolyl, imidazolyl, triazolyl, thiazolyl, and the like.

In the present invention, the C₁₋₆ alkyl represented by R⁷ is preferably unsubstituted C₁₋₆ alkyl or C₁₋₆ alkyl substituted with halogen or hydroxy. In the present invention, the C₁₋₆ alkyl represented by R⁷ is more preferably unsubstituted C₁₋₆ alkyl or C₁₋₆ alkyl substituted with halogen.

In the present invention, the C₁₋₆ alkylamino represented by R⁷ is preferably unsubstituted C₁₋₆ alkylamino.

In the present invention, the C₃₋₈ cycloalkyl represented by R⁷ is preferably unsubstituted C₃₋₈ cycloalkyl or C₃₋₈ cycloalkyl that contains oxygen in the cycloalkyl ring.

In the present invention, the C₃₋₈ cycloalkyl represented by R⁷ is more preferably unsubstituted C₃₋₈ cycloalkyl.

In the present invention, the C₃₋₈ cycloalkylamino represented by R⁷ is preferably unsubstituted C₃₋₈ cycloalkylamino.

In the present invention, the C₁₋₆ alkoxy represented by R⁷ is preferably unsubstituted C₁₋₆ alkoxy.

In the present invention, the C₃₋₈ cycloalkoxy represented by R⁷ is preferably unsubstituted C₃₋₈ cycloalkoxy.

In the present invention, the C₁₋₃ alkoxy-C₁₋₃ alkyl represented by R⁷ is preferably unsubstituted C₁₋₃ alkoxy-C₁₋₃ alkyl.

In the present invention, the C₁₋₄ alkoxycarbonyl C₁₋₄ alkyl represented by R⁷ is preferably unsubstituted C₁₋₄ alkoxycarbonyl C₁₋₄ alkyl.

In the present invention, the pyridyl represented by R⁷ is preferably unsubstituted pyridyl.

In the present invention, the aryl represented by R⁷ is not particularly limited, and examples include phenyl, naphthyl, and the like. In the present invention, the aryl represented by R⁷ is preferably phenyl.

The aryl represented by R⁷ described above may be unsubstituted or may have at least one (generally 1 to 5, preferably 1 to 3, more preferably 1 or 2) substituent. Examples of substituents include, but are not particularly limited to, oxygen, halogen, hydroxy, amino, nitro, cyano, C₁₋₆ alkyl, halo-C₁₋₆ alkyl, C₁₋₆ alkoxy, halo-C₁₋₆ alkoxy, C₂₋₆ alkenyl, halo-C₂₋₆ alkenyl, C₂₋₆ alkynyl, halo-C₂₋₆ alkynyl, phenyl, phenoxy, pyridyl, pyrazolyl, imidazolyl, triazolyl, thiazolyl, and the like.

In the present invention, the aryl represented by R⁷ is preferably unsubstituted phenyl.

The aryl constituting the aryloxy represented by R⁷ is not particularly limited, and examples include phenyl, naphthyl, and the like.

The aryl constituting the aryloxy represented by R⁷ may be unsubstituted or may have at least one (generally 1 to 5, preferably 1 to 3, more preferably 1 or 2) substituent. Examples of substituents include, but are not particularly limited to, oxygen, halogen, hydroxy, amino, nitro, cyano, C₁₋₆ alkyl, halo-C₁₋₆ alkyl, C₁₋ₑ alkoxy, halo-C₁₋₆ alkoxy, C₂₋₆ alkenyl, halo-C₂₋₆ alkenyl, C₂₋₆ alkynyl, halo-C₂₋₆ alkynyl, phenyl, phenoxy, pyridyl, pyrazolyl, imidazolyl, triazolyl, thiazolyl, and the like.

In the present invention, the aryloxy represented by R⁷ is preferably unsubstituted phenyloxy.

In the present invention, when R² and R³, taken together with the nitrogen to which they are attached, via -CH₂CH₂X (R⁸) CH₂CH₂-, form a ring, it is preferable that when X represents -CH, R⁸ is hydrogen or aryl C₁₋₆ alkyl, and that when X represents nitrogen, R⁸ is aryl C₁₋₆ alkoxycarbonyl.

In the present invention, when R² and R³, taken together with the nitrogen to which they are attached, via -CH₂CH₂X (R⁸) CH₂CH₂-, form a ring, it is more preferable that X is nitrogen, and R⁸ is aryl C₁₋₆ alkoxycarbonyl.

In the present invention, among the C₁₋₆ alkyl groups represented by R⁴, C₁₋₄ alkyl is preferable, and methyl or ethyl is more preferable.

In the present invention, R⁴ is preferably hydrogen.

In the present invention, the sulfonyl represented by R⁵ may be unsubstituted or may have at least one substituent. Examples of substituents include, but are not particularly limited to, oxygen, halogen, hydroxy, amino, nitro, cyano, C₁₋₆ alkyl, halo-C₁₋₆ alkyl, C₁₋₆ alkoxy, halo-C₁₋₆ alkoxy, C₂₋₆ alkenyl, halo-C₂₋₆ alkenyl, C₂₋₆ alkynyl, halo-C₂₋₆ alkynyl, phenyl, phenoxy, pyridyl, pyrazolyl, imidazolyl, triazolyl, thiazolyl, and the like.

In the present invention, the sulfonyl represented by R⁵ is preferably sulfonyl substituted with C₁₋₆ alkyl.

In the present invention, R⁵ is more preferably nitro.

In the present invention, R⁶ is preferably hydrogen or C₁₋₆ alkyl. In the present invention, R⁶ is more preferably hydrogen.

In the present invention, examples of halogen include fluorine, chlorine, bromine, and iodine, with fluorine, chlorine, and bromine being preferable, and fluorine being more preferable.

In the present invention, the term "C₁₋₆ alkyl" refers to linear or branched alkyl having 1 to 6 carbon atoms, and examples include methyl, ethyl, n-propyl, isopropyl, n-butyl, isobutyl, tert-butyl, sec-butyl, n-pentyl, neopentyl, n-hexyl, isohexyl, 3-methylpentyl, and the like.

In the present invention, the term "halo-C₁₋₆ alkyl" refers to the linear or branched alkyl having 1 to 6 carbon atoms defined above that is substituted with 1 to 7 halogen atoms, and examples include fluoromethyl, difluoromethyl, trifluoromethyl, chloromethyl, dichloromethyl, trichloromethyl, bromomethyl, dibromomethyl, dichlorofluoromethyl, 2,2,2-trifluoroethyl, 1,1,2,2,2-pentafluoroethyl, 2-chloroethyl, and the like.

In the present invention, the term "C₁₋₆ alkoxy" refers to linear or branched alkoxy having 1 to 6 carbon atoms, and examples include methoxy, ethoxy, n-propoxy, isopropoxy, n-butoxy, isobutoxy, tert-butoxy, sec-butoxy, and the like.

In the present invention, the term "halo-C₁₋₆ alkoxy" refers to the C₁₋₆ alkoxy defined above that is substituted with 1 to 7 halogen atoms, and examples include fluoromethoxy, chloromethoxy, bromomethoxy, trifluoromethoxy, 2-fluoroethoxy, 2-chloroethoxy, 2,2-difluoroethoxy, 3-fluoropropoxy, and the like.

In the present invention, the term "C₂₋₆ alkenyl" refers to linear or branched alkenyl having 2 to 6 carbon atoms and 1 to 3 double bonds, and examples include 2-propenyl, 2-butenyl, 3-butenyl, 4-pentenyl, 3-pentenyl, 5-hexenyl, 4-hexenyl, 3-hexenyl, 1,3-butadienyl, 1,3,5-hexatrienyl, and the like.

In the present invention, the term "halo-C₂₋₆ alkenyl" refers to the linear or branched alkenyl having 2 to 6 carbon atoms defined above that is substituted with 1 to 7 halogen atoms, and examples include 3-fluoro-1-propenyl, 4-chloro-2-butenyl, 4,4-dichloro-3-butenyl, 4,4-difluoro-3-butenyl, 2,2-dichloro-2-propenyl, and the like.

In the present invention, the term "C₂₋₆ alkynyl" refers to linear or branched alkynyl having 2 to 6 carbon atoms and 1 to 3 triple bonds, and examples include 2-propynyl, 2-butynyl, 3-butynyl, 4-pentynyl, 3-pentynyl, 5-hexynyl, 4-hexynyl, 3-hexynyl, and the like.

In the present invention, the term "halo-C₂₋₆ alkynyl" refers to the linear or branched alkynyl having 2 to 6 carbon atoms defined above that is substituted with 1 to 7 halogen atoms, and examples include 2-chloro-ethynyl, 3-chloro-1-propynyl, 3-chloro-2-propynyl, 4-chloro-1-butynyl, 4,4-dichloro-2-butynyl, 4,4-difluoro-3-butynyl, and the like.

In the present invention, the term "C₃₋₈ cycloalkyl" refers to 3-, 4-, 5-, 6-, 7-, or 8-membered cyclic alkyl having 3 to 8 carbon atoms, and examples include cyclopropyl, cyclobutyl, cyclopentyl, cyclohexyl, cycloheptyl, cyclooctyl, 3,4-dimethylcyclopentyl, 3,3-dimethylcyclohexyl, and the like.

In the present invention, the term "C₃₋₈ cycloalkenyl" refers to 3-, 4-, 5-, 6-, 7-, or 8-membered cyclic alkenyl having a double bond and 3 to 8 carbon atoms, and examples include cyclopropenyl, cyclobutenyl, cyclopentenyl, cyclohexenyl, cycloheptenyl, cyclooctenyl, 3,4-dimethylcyclopentenyl, 3,3-dimethylcyclohexenyl, and the like.

In the present invention, the term "C₃₋₈ cycloalkoxy" refers to C₃₋₈ cycloalkoxy in which the 3-, 4-, 5-, 6-, 7-, or 8-membered cyclic alkyl having 3 to 8 carbon atoms defined above and oxy are bonded to each other, and examples include cyclopropyloxy, cyclobutyloxy, cyclopenthyloxy, cyclohexyloxy, cycloheptyloxy, cyclooctyloxy, and the like.

Examples of the pharmaceutically acceptable salt include inorganic acid salts, such as alkali metal salts (e.g., sodium salts, potassium salts, and lithium salts), hydrofluoric acid salts, hydrochlorides, hydrobromides, hydriodides, sulfates, nitrates, phosphates, and perchlorates; organic acid salts, such as methanesulfonates, ethanesulfonates, benzenesulfonates, toluenesulfonates, acetates, lactates, maleates, fumarates, succinates, citrates, pyruvates, and benzoates; and the like.

Examples of the solvent that constitutes the solvate include water; alcohols, such as methanol, ethanol, n-propanol, isopropanol, and n-butanol; ketones, such as acetone and methyl ethyl ketone; esters, such as ethyl acetate; ethers, such as tetrahydrofuran, diethyl ether, and diisopropyl ether; aromatic hydrocarbons, such as benzene and toluene; halogenated hydrocarbons, such as ethylene chloride and chloroform; polar solvents, such as acetonitrile, dimethylformamide, and dimethyl sulfoxide; and the like.

In the present invention, when the 8-hydroxyquinoline compound includes a compound containing an asymmetric carbon atom, the compound may be an R-form, an L-form, or a racemate thereof. When the 8-hydroxyquinoline compound includes a compound containing a double bond, the compound may be a cis-form or a trans-form.

Among the 8-hydroxyquinoline compounds represented by formula (I), an 8-hydroxyquinoline compound represented by the following formula (Ia) is a novel compound that has not been described in any document and that has been successfully synthesized by the present inventors for the first time.

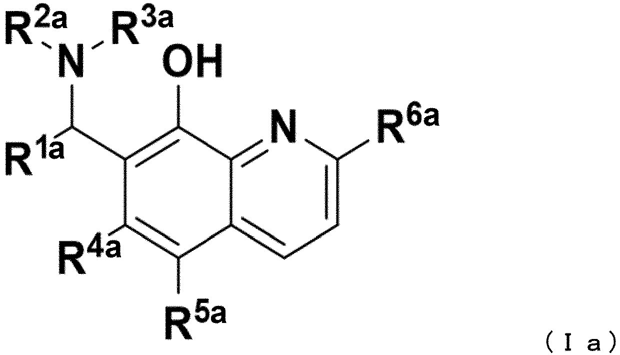

The groups represented by R^{1a}, R^{2a}, R^{3a}, R^{4a}, R^{5a}, R^{6a}, R^{7a}, and R^{8a} in formula (Ia) are respectively synonymous with the groups represented by R¹, R², R³, R⁴, R⁵, R⁶, R⁷, and R⁸ in formula (I).

The 8-hydroxyquinoline compound of the present invention can be produced, for example, by the method shown in the following Reaction Scheme-1.

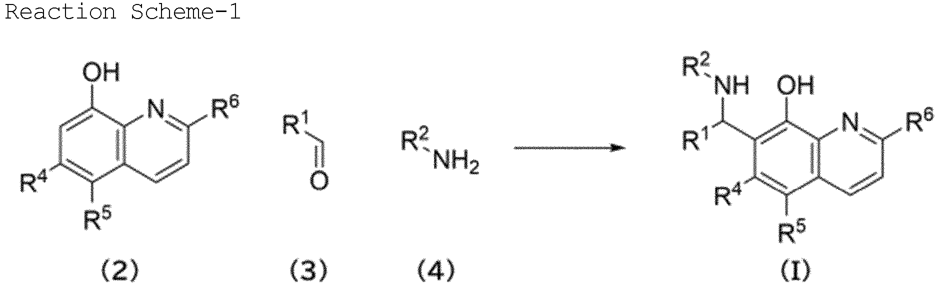

That is, the 8-hydroxyquinoline compound represented by formula (I) is produced by reacting an 8-hydroxyquinoline represented by formula (2), an aldehyde represented by formula (3), and an amine represented by formula (4). Specifically, the 8-hydroxyquinoline compound represented by formula (I) is produced by heating a mixture of the 8-hydroxyquinoline (2), the aldehyde (3), and the amine (4).

The amount of the aldehyde (3) for use is generally about 0.5 to 2 moles, preferably about 0.7 to 1.5 moles, and more preferably about 0.9 to 1.1 moles, per mole of the 8-hydroxyquinoline (2). The amount of the amine (4) for use is generally about 0.5 to 2 moles, preferably about 0.7 to 1.5 moles, and more preferably about 0.9 to 1.1 moles, per mole of the 8-hydroxyquinoline (2).

The heating temperature is equal to or lower than the boiling points of the starting material compounds, i.e., the 8-hydroxyquinoline (2), the aldehyde (3), and the amine (4), preferably 100 to 200°C, and more preferably 130 to 140°C. The heating time varies depending on the heating temperature and cannot be generalized. The heating time is generally about 0.5 to 5 hours.

The above reaction is generally performed without a solvent.

When the 8-hydroxyquinoline (2) is a compound in which R⁵ represents hydrogen, it is preferable to use a sulfonic acid, such as p-toluenesulfonic acid, as a reaction catalyst.

The 8-hydroxyquinoline compound (I) produced by the above method can be easily isolated and purified from the reaction mixture by a known isolation means, such as distillation, drying, washing, or recrystallization and a known purification means, such as column chromatography.

The cMLCK activator or pharmaceutical composition for treating heart disease of the present invention (hereinafter, the cMLCK activator and the pharmaceutical composition for treating heart disease are collectively referred to as "the pharmaceutical composition for treating heart disease") is used as a therapeutic agent for heart failure (in particular, a therapeutic agent for heart failure with reduced contractility, i.e., systolic heart failure), an inotropic agent, a drug for enhancing myocardial contractility, or a agent for improving cardiac function. Moreover, the pharmaceutical composition for treating heart disease is used to promote organization of a sarcomere structure.

In the present invention, "systolic heart failure (HFrEF)" refers to a clinical syndrome in which organic or functional abnormalities of the heart cause poor contraction of the left ventricle and insufficient ejection, resulting in an increase in the left ventricular diastolic volume, an increase in the left ventricular end-diastolic pressure, and a decrease in the left ventricular ejection fraction, whereby dyspnea, fatigue, or an edema occurs, which is accompanied by a decrease in exercise tolerance.

In the present invention, "inotropic agent" refers to a drug that increases myocardial contractility and is used for the treatment of systolic heart failure to improve cardiac pump function.

In the present invention, "drug for enhancing myocardial contractility" refers to a drug that has the action of enhancing myocardial contractility. "Myocardial contractility" refers to the force of contraction of myocardial fibers that make up the heart, and the ability of the heart to contract and pump blood. In the present invention, "myocardial contractility" include the contractile ability and diastolic ability of cardiac muscle, and is also referred to as "cardiac contractility."

In the present invention, "agent for improving cardiac function" refers to a agent that has the action of improving cardiac function etc. "Cardiac function" refers to the function of the heart to supply blood throughout the body at an appropriate or required blood volume and blood pressure.

The present invention is preferably applied to a subject in which improvement in cardiac function is expected by promoting organization of a sarcomere structure. Sarcomere is a structural and functional contraction unit of striated muscle that is composed of thin filaments containing actin as a main component and thick filaments containing myosin as a main component, and is regularly repeated to form striated muscle. By improving disorganization of myocardial sarcomere structures observed in heart failure due to dilated cardiomyopathy, post-myocardial infarction remodeling, or the like, the condition of heart failure can be improved from a structural perspective as well.

In the present invention, examples of diseases requiring improvement of a sarcomere structure and/or promotion of organization include chronic heart failure, ischemic cardiomyopathy, systolic heart failure, dilated phase of hypertrophic cardiomyopathy, and the like.

In the present invention, the subject to which the pharmaceutical composition for treating heart disease is administered is not particularly limited, and may be a human or a non-human mammal. Examples of humans to which the pharmaceutical composition for treating heart disease is administered include patients with diseases such as acute heart failure, chronic heart failure, dilated cardiomyopathy, ischemic cardiomyopathy, systolic heart failure, and dilated phase of hypertrophic cardiomyopathy; and the like. Examples of non-human mammals include mammals raised as pets, livestock, experimental animals, and the like. Examples of such non-human mammals include dogs, cats, monkeys, cattle, horses, sheep, goats, swine, rabbits, mice, rats, guinea pigs, camels, llamas, and the like.

The dosage form, administration method, and dose of the pharmaceutical composition for treating heart disease of the present invention can be appropriately determined according to the purpose of use and various conditions.

Example of dosage forms of the pharmaceutical composition for treating heart disease of the present invention include, but are not particularly limited to, tablets, pills, powders, liquids, suspensions, emulsions, granules, capsules, suppositories, injections (e.g., liquids and suspensions), and the like.

The pharmaceutical composition for treating heart disease of the present invention is prepared using a commonly used diluent or excipient, such as a filler, an extender, a binder, a humectant, a disintegrator, a surfactant, or a lubricant.

When the pharmaceutical composition for treating heart disease of the present invention is prepared in the form of a tablet, a wide range of carriers well known in this field can be used. Examples of usable carriers include excipients, such as lactose, sucrose, sodium chloride, glucose, urea, starch, calcium carbonate, kaolin, crystalline cellulose, and silicic acid; binders, such as water, ethanol, propanol, simple syrups, liquid glucose, liquid starch, gelatin solutions, carboxymethyl cellulose, shellac, methylcellulose, potassium phosphate, and polyvinyl pyrrolidone; disintegrators, such as dry starch, sodium alginate, powdered agar, powdered laminaran, sodium hydrogencarbonate, calcium carbonate, polyoxyethylene sorbitan fatty acid esters, sodium lauryl sulfate, stearic acid monoglyceride, starch, and lactose; disintegration inhibitors, such as sucrose, stearin, cocoa butter, and hydrogenated oils; absorption enhancers, such as quaternary ammonium bases and sodium lauryl sulfate; humectants, such as glycerol and starch; adsorbents, such as starch, lactose, kaolin, bentonite, and colloidal silicic acid; lubricants, such as purified talc, stearic acid salts, powdered boric acid, and polyethylene glycol; and the like.

Tablets may be optionally provided with general coatings to provide sugar-coated tablets, gelatin-coated tablets, enteric-coated tablets, film-coated tablets, double-layer tablets, multi-layer tablets, and the like.

When the pharmaceutical composition for treating heart disease of the present invention is prepared in the form of a pill, a wide range of carriers known in this field can be used. Examples of usable carriers include excipients, such as glucose, lactose, starch, cacao butter, hydrogenated vegetable oils, kaolin, and talc; binders, such as powdered gum arabic, powdered tragacanth, gelatin, and ethanol; disintegrators such as powdered laminaran and powdered agar; and the like.

When the pharmaceutical composition for treating heart disease of the present invention is prepared in the form of a suppository, a wide range of known carriers can be used. Examples include polyethylene glycol, cocoa butter, higher alcohols, esters of higher alcohols, gelatin, semisynthetic glyceride, and the like.

A capsule is generally prepared by mixing the 8-hydroxyquinoline compound of the present invention with one or more carriers mentioned above, and filling a hard gelatin capsule, a soft capsule, or the like with the mixture, according to a usual method.

When the pharmaceutical composition for treating heart disease of the present invention is prepared in the form of an injection, it is preferred that a liquid, an emulsion, or a suspension is sterilized and is isotonic with blood. In preparing the pharmaceutical composition in such a form, any diluent commonly used in this field can be used. Examples of usable diluents include water, ethyl alcohol, macrogol, propylene glycol, ethoxylated isostearyl alcohols, polyoxylated isostearyl alcohols, polyoxyethylene sorbitan fatty acid esters, and the like. In this case, salt, glucose, or glycerol may be incorporated in the pharmaceutical preparation in an amount sufficient to prepare an isotonic solution. General solubilizing agents, buffers, soothing agents, etc. may also be added.

Further, if necessary, the pharmaceutical composition for treating heart disease may contain a coloring agent, a preservative, an aroma component, a flavoring, a sweetener, and other medical drugs.

The amount of the 8-hydroxyquinoline compound contained in the pharmaceutical composition for treating heart disease of the present invention is not particularly limited and may be appropriately selected from a wide range. The amount of the 8-hydroxyquinoline compound in the preparation composition may be generally about 1 to 70 wt%, and preferably about 5 to 50 wt%.

The method for administrating the pharmaceutical composition for treating heart disease of the present invention is not particularly limited. The pharmaceutical composition for treating heart disease is administered by a method according to the dosage form, the patient's age, sex, and other conditions, the severity of the disease, etc. For example, the pharmaceutical composition for treating heart disease of the present invention in the form of a tablet, a pill, a liquid, a suspension, an emulsion, a granule, or a capsule is orally administered. The pharmaceutical composition for treating heart disease of the present invention in the form of an injection is intravenously administered either alone or in mixture with an ordinary fluid replacement such as glucose and amino acid, and if necessary, administered alone intramuscularly, intradermally, subcutaneously, or intraperitoneally. The pharmaceutical composition for treating heart disease of the present invention in the form of a suppository is administered intrarectally.

The dose of the pharmaceutical composition for treating heart disease of the present invention is not particularly limited and is appropriately selected according to the dosage regimen, the patient's age, sex, and other conditions, the severity of the disease, etc. For example, in the case of oral administration to an adult, the daily dose may be, for example, about 0.1 to 1000 mg, and in the case of parenteral administration, the daily dose may be about 0.01 to 200 mg. The frequency of administration may be once or multiple times per day.

### Examples

Hereinafter, embodiments of the present disclosure are described in more detail with reference to Examples. However, the embodiments of the present disclosure are not limited to the following Examples.

### Example 1: Synthesis of 8-Hydroxyquinoline Compound of the Present Invention

### Measurement of Physical Properties of the 8-Hydroxyquinoline Compound of the Present Invention

The physical properties of each of the 8-hydroxyquinoline compounds produced in the following Synthesis Examples were measured according to the following methods. For NMR spectra, a JNM-ECZ400S/L1 spectrometer (JEOL Ltd.) was used. ¹H-NMR was measured at 400 MHz at room temperature using DMSO-d₆ or CDCl₃ as a deuterated solvent. LCMS was performed using an ACQUITY UPLC H-Class/QDa system (Waters) under the conditions of column: ACQUITY UPLC BEH C18 1.7 uM (2.1 x 50 mm), flow rate: 0.5 mL/min, UV detection wavelength: 254 nm, mobile phase: [A] aqueous solution of 0.1% formic acid, [B] acetonitrile; linear gradient was performed for 3 minutes (linear gradient from 90% of [A] and 10% of [B] to 5% of [A] and 95% of [B]), followed by maintaining the condition of 5% of [A] and 95% of [B] for 0.5 minutes. RT indicates the retention time of LC under the above conditions.

### Synthesis Example 1: Synthesis of LEU-0992

A mixture of 8-hydroxy-5-nitroquinoline (190 mg, 1 mmol), p-anisaldehyde (163 mg, 1.2 mmol), and valeramide (106 mg, 1.05 mmol) was heated at 135°C for 3 hours. The reaction mixture was cooled to room temperature, and suspended and washed with ethyl acetate to give N-[(8-hydroxy-5-nitroquinolin-7-yl)(4-methoxyphenyl)methyl]pentanamide (LEU-0992) (amount: 336 mg, yield: 82%). 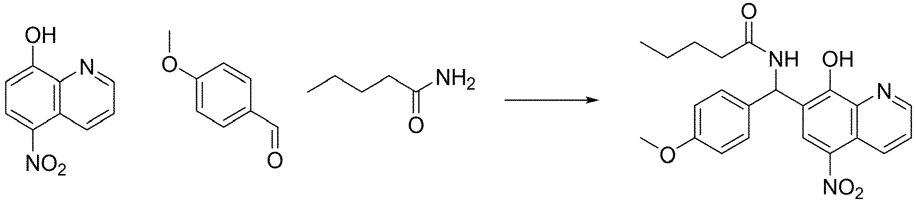
¹H-NMR (400 MHz, DMSO-d₆) δppm
9.16 (dd, J = 8.9, 1.6 Hz, 1H), 8.99 (dd, J = 4.0, 1.4 Hz, 1H), 8.83 (d, J = 8.2 Hz, 1H), 8.66 (s, 1H), 7.87 (dd, J = 8.7, 4.0 Hz, 1H), 7.17 (d, J = 8.7 Hz, 2H), 6.87 (d, J = 8.7 Hz, 2H), 6.57 (d, J = 8.7 Hz, 1H), 3.70 (s, 3H), 2.21 (t, J = 7.6 Hz, 2H), 1.50 (tt, J = 7. 6, 7. 6 Hz, 2H), 1.25 (qt, J = 7.6, 7.6 Hz, 2H), 0.84 (t, J = 7.6 Hz, 3H)

### Synthesis Example 2: Synthesis of LEU-0989

N-[(8-hydroxy-5-nitroquinolin-7-yl)(4-methoxyphenyl)methyl]cyclohexanecarboxamide (LEU-0989) was obtained in the same manner as in Synthesis Example 1 by using corresponding starting material compounds. 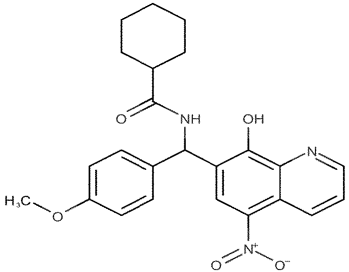
¹H-NMR (400 MHz, DMSO-d₆) δppm
9.17 (dd, J = 8.9, 1.6 Hz, 1H), 8.97 (d, J = 3.9, 1.4 Hz, 1H), 8.73 (d, J = 8.7 Hz, 1H), 8.66 (s, 1H), 7.85 (dd, J = 8.8, 3.8 Hz, 1H), 7.16 (d, J = 8.7 Hz, 2H), 6.86 (d, J = 8.7 Hz, 2H), 6.54 (d, J = 8.7 Hz, 1H), 3.69 (s, 3H), 2.26-2.32 (m, 1H), 1.59-1.70 (m, 5H), 1.11-1.39 (m, 5H)

### Synthesis Example 3: Synthesis of LEU-1034

1-butyl-3-[(8-hydroxy-5-nitroquinolin-7-yl) (4-methoxyphenyl)methyl]urea (LEU-1034) was obtained in the same manner as in Synthesis Example 1 by using corresponding starting material compounds. 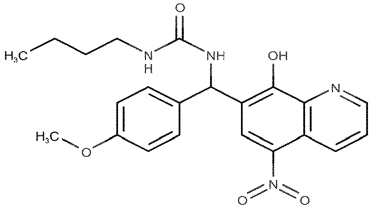
¹H-NMR (400 MHz, DMSO-d₆) δppm
9.17 (dd, J = 9.1, 1.4 Hz, 1H), 8.98 (dd, J = 4.0, 1.4 Hz, 1H), 8.63 (s, 1H), 7.86 (dd, J = 8.7, 4.0 Hz, 1H), 7.17 (d, J = 8.7 Hz, 2H), 6.93 (d, J = 8.2 Hz, 1H), 6.86 (d, J = 8.7 Hz, 2H), 6.31 (d, J = 8.2 Hz, 1H), 5.96 (t, J = 5.7 Hz, 1H), 3.69 (s, 3H), 2.98 (td, J = 7.6 Hz, 5.7, 2H), 1.32 (qt, J = 7.6, 7.6 Hz, 2H), 1.25 (tt, J = 7.6, 7.6 Hz, 2H), 0.84 (t, J = 7.6 Hz, 3H)

### Synthesis Example 4: Synthesis of LEU-1035

Butyl [(8-hydroxy-5-nitroquinolin-7-yl)(4-methoxyphenyl)methyl]carbamate (LEU-1035) was obtained in the same manner as in Synthesis Example 1 by using corresponding starting material compounds. 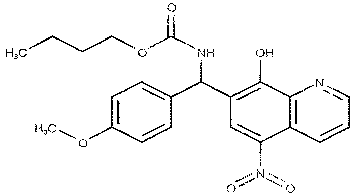
¹H-NMR (400 MHz, DMSO-d₆) δppm
9.16 (dd, J = 8.9, 1.6 Hz, 1H), 8.99 (dd, J = 4.0, 1.4 Hz, 1H), 8.75 (s, 1H), 8.38 (d, J = 9.1 Hz, 1H), 7.87 (dd, J = 8.7, 4.0 Hz, 1H), 7.21 (d, J = 8.7 Hz, 2H), 6.86 (d, J = 8.7 Hz, 2H), 6.38 (d, J = 9.1 Hz, 1H), 3.96 (t, J = 6.6 Hz, 2H), 3.69 (s, 3H), 1.52 (tt, J = 7. 6, 6.6 Hz, 2H), 1. 33 (qt, J = 7.6, 7.6 Hz, 2H), 0.86 (t, J = 7.6 Hz, 3H)

### Synthesis Example 5: Synthesis of LEU-1049

N-[(3-bromophenyl)(8-hydroxy-5-nitroquinolin-7-yl)methyl]pentanamide (LEU-1049) was obtained in the same manner as in Synthesis Example 1 by using corresponding starting material compounds. 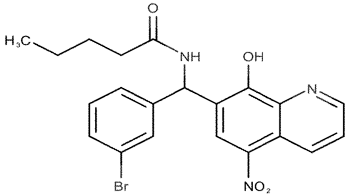
¹H-NMR (400 MHz, CDCl₃) δppm
9.34 (dd, J = 8.7, 1.4 Hz, 1H), 8.89 (dd, J = 4.0, 1.4 Hz, 1H), 8.63 (s, 1H), 7.75 (dd, J = 8.7, 4.0 Hz, 1H), 7.46 (t, J = 1.6 Hz, 1H), 7.42 (dt, J = 7.8, 1.6 Hz, 1H), 7.25 (d, J = 8.0, 1H), 7.22 (dd, J = 8.0, 7.6 Hz, 1H), 6.64 (d, J = 8.0 Hz, 1H), 6.60 (d, J = 8.0 Hz, 1H), 2.33 (t, J = 7.6, Hz, 2H), 1.67 (tt, J = 7.6, 7.6 Hz, 2H), 1.38 (qt, J = 7.6, 7.3 Hz, 2H), 0.93 (t, J = 7.3 Hz, 3H)

### Synthesis Example 6: Synthesis of LEU-1050

N-[(4-bromophenyl)(8-hydroxy-5-nitroquinolin-7-yl)methyl]pentanamide (LEU-1050) was obtained in the same manner as in Synthesis Example 1 by using corresponding starting material compounds. 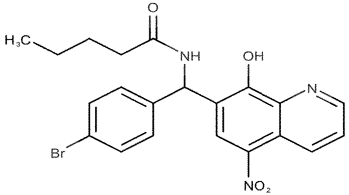
¹H-NMR (400 MHz, CDCl₃) δppm
9.33 (dd, J = 9.1, 1.4 Hz, 1H), 8.88 (dd, J = 4.0, 1.4 Hz, 1H), 8.63 (s, 1H), 7.74 (dd, J = 8.7, 4.0 Hz, 1H), 7.46 (dt, J = 9.0, 2.3 Hz, 2H), 7.20 (d, J = 9.0, 2H), 6.57-6.62 (m, 2H), 2.32 (t, J = 7.5 Hz, 2H), 1.67 (tt, J = 7.6, 7.6 Hz, 2H), 1.37 (qt, J = 7.6, 7.3 Hz, 2H), 0.92 (t, J = 7.3 Hz, 3H)

### Synthesis Example 7: Synthesis of LEU-1057

N-[(1,1'-biphenyl)-3-yl(8-hydroxy-5-nitroquinolin-7-yl)methyl]pentanamide (LEU-1057) was obtained in the same manner as in Synthesis Example 1 by using corresponding starting material compounds. 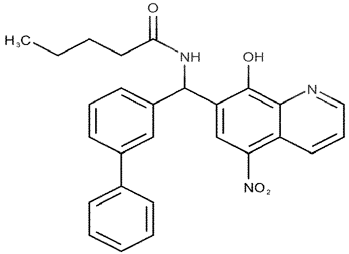
¹H-NMR (400 MHz, CDCl₃) δppm
9.32 (dd, J = 8.7, 1.4 Hz, 1H), 8.86 (dd, J = 4.0, 1.4 Hz, 1H), 8.70 (s, 1H), 7.71 (dd, J = 8.7, 4.0 Hz, 1H), 7.49-7.54 (m, 4H), 7.38-7.43 (m, 3H), 7.27-7.35 (m, 2H), 6.68 (d, J = 7.8 Hz, 1H), 6.63 (d, J = 7.8 Hz, 1H), 2.32 (t, J = 7.5 Hz, 2H), 1.67 (tt, J = 7.6, 7.5 Hz, 2H), 1.37 (qt, J = 7.6, 7.3 Hz, 2H), 0.91 (t, J = 7.3 Hz, 3H)

### Synthesis Example 8: Synthesis of LEU-1061

N-[(1,1'-biphenyl)-4-yl(8-hydroxy-5-nitroquinolin-7-yl)methyl]pentanamide (LEU-1061) was obtained in the same manner as in Synthesis Example 1 by using corresponding starting material compounds. 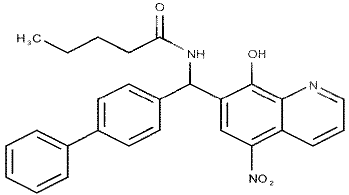
¹H-NMR (400 MHz, CDCl₃) δppm
9.34 (dd, J = 8.7, 1.4 Hz, 1H), 8.88 (dd, J = 4.0, 1.4 Hz, 1H), 8.71 (s, 1H), 7.73 (dd, J = 8.7, 4.0 Hz, 1H), 7.52-7.57 (m, 4H), 7.39-7.44 (m, 4H), 7.32-7.36 (m, 1H), 6.68 (d, J = 7.6 Hz, 1H), 6.65 (d, J = 7.6 Hz, 1H), 2.34 (t, J = 7.5 Hz, 2H), 1.69(tt, J = 7.6, 7.5 Hz, 2H), 1.39 (qt, J = 7.6, 7.3 Hz, 2H), 0.93 (t, J = 7.3 Hz, 3H)

### Synthesis Example 9: Synthesis of LEU-1080

N-[(8-hydroxy-5-nitroquinolin-7-yl)(4-methoxyphenyl)methyl]hexanamide (LEU-1080) was obtained in the same manner as in Synthesis Example 1 by using corresponding starting material compounds. 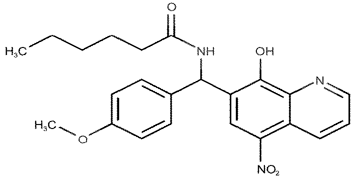
¹H-NMR (400 MHz, CDCl₃) δppm
9.32 (dd, J = 8.7, 1.4 Hz, 1H), 8.86 (dd, J = 4.0, 1.4 Hz, 1H), 8.67 (s, 1H), 7.71 (dd, J = 8.7, 4.0 Hz, 1H), 7.24 (d, J = 8.7 Hz, 2H), 6.86 (dt, J = 8.7 Hz, 2H), 6.57 (d, J = 7.3 Hz, 1H), 6.52 (d, J = 7.8 Hz, 1H), 3.78 (s, 3H), 2.30 (t, J = 7.5 Hz, 2H), 1.68 (tt, J = 7.6, 7.5 Hz, 2H), 1.35-1.29 (m, 4H), 0.87 (t, J = 7.1 Hz, 3H)

### Synthesis Example 10: Synthesis of LEU-1081

Benzyl [(8-hydroxy-5-nitroquinolin-7-yl) (4-methoxyphenyl)methyl]carbamate (LEU-1081) was obtained in the same manner as in Synthesis Example 1 by using corresponding starting material compounds. 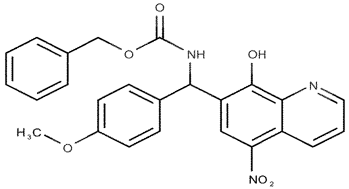
¹H-NMR (400 MHz, CDCl₃) δppm
9.32 (dd, J = 8.9, 1.6 Hz, 1H), 8.86 (dd, J = 4.0, 1.4 Hz, 1H), 8.70 (s, 1H), 7.71 (dd, J = 8.7, 4.0 Hz, 1H), 7.41-7.30 (m, 5H), 7.27 (d, J = 8.4, 2H), 6.85 (d, J = 8.4, 2H), 6.35 (d, J = 7.3 Hz, 1H), 5.86 (bs, 1H), 5.17-5.09 (m, 2H), 3.77 (s, 3H)

### Synthesis Example 11: Synthesis of LEU-1100

N-[(4-fluorophenyl)(8-hydroxy-5-nitroquinolin-7-yl)methyl]pentanamide (LEU-1100) was obtained in the same manner as in Synthesis Example 1 by using corresponding starting material compounds. 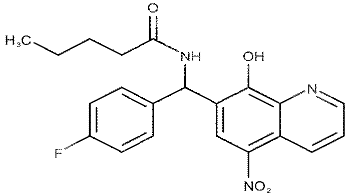
¹H-NMR (400 MHz, DMSO-d₆) δppm
9.18 (dd, J = 8.7, 1.4 Hz, 1H), 9.00 (dd, J = 4.0, 1.4 Hz, 1H), 8.89 (d, J = 8.2 Hz, 1H), 8.65 (s, 1H), 7.88 (dd, J = 8.7, 4.0 Hz, 1H), 7.29 (dd, J = 8.7, 5.5 Hz, 2H), 7.14 (dd, J = 8.9, 8.7 Hz, 2H), 6.62 (d, J = 8.2 Hz, 1H), 2.22 (t, J = 7.6 Hz, 2H), 1.50 (tt, J = 7.6, 7.6 Hz, 2H), 1.25 (qt, J = 7.6, 7.3 Hz, 2H), 0.85 (t, J = 7.3 Hz, 3H)

### Synthesis Example 12: Synthesis of LEU-1101

N-{(8-hydroxy-5-nitroquinolin-7-yl) [4-(trifluoromethyl)phenyl]methyl}pentanamide (LEU-1101) was obtained in the same manner as in Synthesis Example 1 by using corresponding starting material compounds. 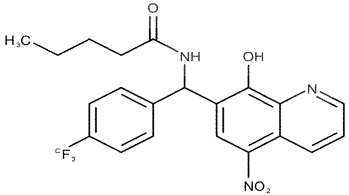
¹H-NMR (400 MHz, DMSO-d₆) δppm
9.19 (dd, J = 9.0, 1.4 Hz, 1H), 9.00 (dd, J = 4.0, 1.4 Hz, 1H), 8.98 (d, J = 8.2 Hz, 1H), 8.66 (s, 1H), 7.90 (dd, J = 9.0, 4.0 Hz, 1H), 7.70 (d, J = 8.2 Hz, 2H), 7.49 (d, J = 8.2 Hz, 2H), 6.72 (d, J = 8.2 Hz, 1H), 2.24 (t, J = 7.5 Hz, 2H), 1.51 (tt, J = 7.6, 7.5 Hz, 2H), 1.26 (qt, J = 7.6, 7.3 Hz, 2H), 0.85 (t, J = 7.3 Hz, 3H)

### Synthesis Example 13: Synthesis of LEU-1106

N-[(8-hydroxy-5-nitroquinolin-7-yl)(2-methoxyphenyl)methyl]pentanamide (LEU-1106) was obtained in the same manner as in Synthesis Example 1 by using corresponding starting material compounds. 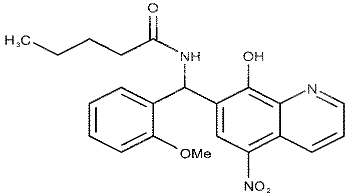
¹H-NMR (400 MHz, DMSO-d₆) δppm
9.17 (dd, J = 9.0, 1.4 Hz, 1H), 8.98 (dd, J = 4.0, 1.4 Hz, 1H), 8.51 (d, J = 7.8 Hz, 1H), 8.45 (s, 1H), 7.87 (dd, J = 9.0, 4.0 Hz, 1H), 7.27 (td, J = 7.5, 1.5 Hz, 1H), 7.11 (dd, J = 7.5, 1.6 Hz, 1H), 7.00 (d, J = 7.5 Hz, 1H), 6.90 (td, J = 7.5, 0.9 Hz, 1H), 6.76 (d, J = 7.8 Hz, 1H), 3.72 (s, 3H), 2.17 (t, J = 7.5 Hz, 2H), 1.49 (tt, J = 7.6, 7.5 Hz, 2H), 1.26 (qt, J = 7.6, 7.3 Hz, 2H), 0.85 (t, J = 7.3 Hz, 3H)

### Synthesis Example 14: Synthesis of LEU-1114

N-[(3-fluorophenyl)(8-hydroxy-5-nitroquinolin-7-yl)methyl]pentanamide (LEU-1114) was obtained in the same manner as in Synthesis Example 1 by using corresponding starting material compounds. 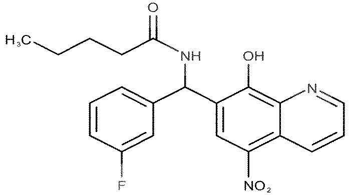
¹H-NMR (400 MHz, DMSO-d₆) δppm
9.19 (dd, J = 8.7, 1.4 Hz, 1H), 9.00 (dd, J = 4.0, 1.4 Hz, 1H), 8.92 (d, J = 8.2 Hz, 1H), 8.64 (s, 1H), 7.88 (dd, J = 8.7, 4.0 Hz, 1H), 7.34-7.39 (m, 1H), 7.07-7.12 (m, 3H), 6.66 (d, J = 8.2 Hz, 1H), 2.23 (t, J = 7.6 Hz, 2H), 1.51 (tt, J = 7.6, 7.5 Hz, 2H), 1.26 (qt, J = 7.6, 7.3 Hz, 2H), 0.85 (t, J = 7.3 Hz, 3H)

### Synthesis Example 15: Synthesis of LEU-1115

N-{(8-hydroxy-5-nitroquinolin-7-yl)[4-(pyridin-4-yl)phenyl]methyl}pentanamide (LEU-1115) was obtained in the same manner as in Synthesis Example 1 by using corresponding starting material compounds. 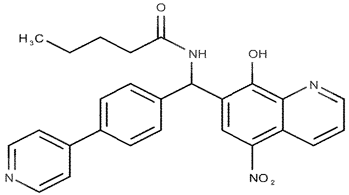
¹H-NMR (400 MHz, DMSO-d₆) δppm
9.19 (dd, J = 8.7, 1.4 Hz, 1H), 9.00 (dd, J = 4.0, 1.4 Hz, 1H), 8.96 (d, J = 8.7 Hz, 1H), 8.70 (s, 1H), 8.60 (d, J = 6.2 Hz, 2H), 7.89 (dd, J = 8.7, 4.0 Hz, 1H), 7.76 (d, J = 8.4 Hz, 2H), 7.66 (d, J = 6.2 Hz, 2H), 7.41 (d, J = 8.4 Hz, 2H), 6.71 (d, J = 8.7 Hz, 1H), 2.25 (t, J = 7.5 Hz, 2H), 1.52 (tt, J = 7.6, 7.5 Hz, 2H), 1.27 (qt, J = 7.6, 7.3 Hz, 2H), 0.86 (t, J = 7.3 Hz, 3H)

### Synthesis Example 16: Synthesis of LEU-1116

N-{(8-hydroxy-5-nitroquinolin-7-yl)[4-(pyridin-3-yl)phenyl]methyl}pentanamide (LEU-1116) was obtained in the same manner as in Synthesis Example 1 by using corresponding starting material compounds. 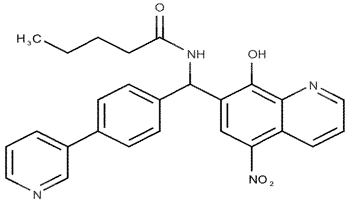
¹H-NMR (400 MHz, DMSO-d₆) δppm
9.19 (dd, J = 8.8, 1.4 Hz, 1H), 9.01 (dd, J = 4.0, 1.4 Hz, 1H), 8.95 (d, J = 8.7 Hz, 1H), 8.85 (d, J = 1.8 Hz, 1H), 8.71 (s, 1H), 8.54 (dd, J = 4.6, 1.8 Hz, 1H), 8.03 (dt, J = 8.0, 1.8 Hz, 1H), 7.89 (dd, J = 8.8, 4.0 Hz, 1H), 7.68 (d, J = 8.6 Hz, 2H), 7.46 (dd, J = 8.0, 4.6 Hz, 1H), 7.39 (d, J = 8.6 Hz, 2H), 6.70 (d, J = 8.7 Hz, 1H), 2.25 (t, J = 7.5 Hz, 2H), 1.52 (tt, J = 7.6, 7.5 Hz, 2H), 1.27 (qt, J = 7.6, 7.3 Hz, 2H), 0.86 (t, J = 7.3 Hz, 3H)

### Synthesis Example 17: Synthesis of LEU-1117

N-[(8-hydroxy-5-nitroquinolin-7-yl) (quinolin-6-yl)methyl]pentanamide (LEU-1117) was obtained in the same manner as in Synthesis Example 1 by using corresponding starting material compounds. 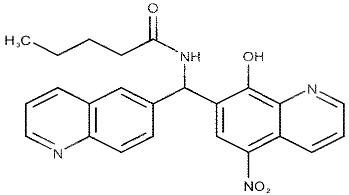
¹H-NMR (400 MHz, DMSO-d₆) δppm
9.21 (dd, J = 8.7, 1.4 Hz, 1H), 9.04-8.98 (m, 2H), 8.86 (dd, J = 4.0, 1.4 Hz, 1H), 8.72 (s, 1H), 8.31 (d, J = 6.9 Hz, 1H), 7.99 (d, J = 9.0 Hz, 1H), 7.89 (dd, J = 8.6, 4.0 Hz, 1H), 7.77 (s, 1H), 7.69 (dd, J = 9.0, 2.1 Hz, 1H), 7.49 (dd, J = 8.5, 4.0 Hz, 1H), 6.82 (d, J = 8.2 Hz, 1H), 2.26 (t, J = 7.5 Hz, 2H), 1.53 (tt, J = 7.6, 7.5 Hz, 2H), 1.28 (qt, J = 7.6, 7.3 Hz, 2H), 0.86 (t, J = 7.3 Hz, 3H)

### Synthesis Example 18: Synthesis of LEU-1118

N-[(2-fluorophenyl)(8-hydroxy-5-nitroquinolin-7-yl)methyl]pentanamide (LEU-1118) was obtained in the same manner as in Synthesis Example 1 by using corresponding starting material compounds. 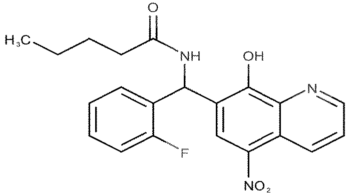
¹H-NMR (400 MHz, DMSO-d₆) δppm
9.17 (dd, J = 8.9, 1.6 Hz, 1H), 8.96 (dd, J = 4.0, 1.4 Hz, 1H), 8.78 (d, J = 8.2 Hz, 1H), 8.53 (s, 1H), 7.86 (dd, J = 8.8, 4.0 Hz, 1H), 7.28-7.34 (m, 1H), 7.10-7.21 (m, 3H), 6.77 (d, J = 7.8 Hz, 1H), 2.18 (td, J = 7.3, 2.9 Hz, 2H), 1.47 (tt, J = 7.3, 7.3 Hz, 2H), 1.23 (qt, J = 7.3, 7.3 Hz, 2H), 0.82 (t, J = 7.3 Hz, 3H)

### Synthesis Example 19: Synthesis of LEU-1119

N-[(4-chlorophenyl)(8-hydroxy-5-nitroquinolin-7-yl)methyl]pentanamide (LEU-1119) was obtained in the same manner as in Synthesis Example 1 by using corresponding starting material compounds. 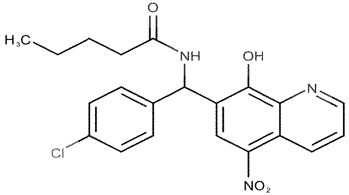
¹H-NMR (400 MHz, DMSO-d₆) δppm
9.17 (dd, J = 9.0, 1.4 Hz, 1H), 8.99 (dd, J = 4.0, 1.4 Hz, 1H), 8.91 (d, J = 8.2 Hz, 1H), 8.64 (s, 1H), 7.87 (dd, J = 9.0, 4.0 Hz, 1H), 7.38 (d, J = 7.6 Hz, 2H), 7.27 (d, J = 7.6 Hz, 2H), 6.63 (d, J = 8.2 Hz, 1H), 2.22 (t, J = 7.3 Hz, 2H), 1.49 (tt, J = 7.3, 7.3 Hz, 2H), 1.26 (qt, J = 7.3, 7.3 Hz, 2H), 0.85 (d, J = 7.3 Hz, 3H)

### Synthesis Example 20: Synthesis of LEU-1120

N-[(3,4-dimethoxyphenyl)(8-hydroxy-5-nitroquinolin-7-yl)methyl]pentanamide (LEU-1120) was obtained in the same manner as in Synthesis Example 1 by using corresponding starting material compounds. 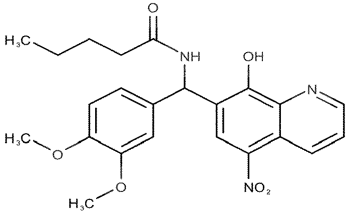
¹H-NMR (400 MHz, DMSO-d₆) δppm
9.17 (d, J = 8.8, 1.4 Hz, 1H), 8.99 (dd, J = 4.0, 1.4 Hz, 1H), 8.81 (d, J = 8.2 Hz, 1H), 8.65 (s, 1H), 7.87 (dd, J = 8.8, 4.0 Hz, 1H), 6. 93 (s, 1H), 6.86 (d, J = 8.4 Hz, 1H), 6.72 (d, J = 8.4 Hz, 1H), 6.58 (d, J = 8.2 Hz, 1H), 3.69 (s, 6H), 2.21 (t, J = 7.3 Hz, 2H), 1.50 (tt, J = 7.3, 7.3 Hz, 2H), 1.26 (qt, J = 7.3, 7.3 Hz, 2H), 0.85 (t, J = 7.3 Hz, 3H)

### Synthesis Example 21: Synthesis of LEU-1127

N-{(8-hydroxy-5-nitroquinolin-7-yl) [6-(trifluoromethyl)pyridin-3-yl]methyl}pentanamide (LEU-1127) was obtained in the same manner as in Synthesis Example 1 by using corresponding starting material compounds. 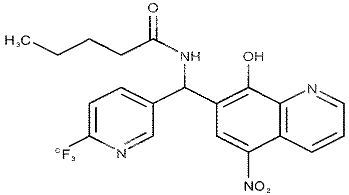
¹H-NMR (400 MHz, DMSO-d₆) δppm
9.24 (dd, J = 8.9, 1.4 Hz, 1H), 9.05 (d, J = 8.2 Hz, 1H), 9.01 (dd, J = 4.0, 1.4 Hz, 1H), 8.74 (d, J = 1.8 Hz, 1H), 8.71 (s, 1H), 7.87-7.96 (m, 3H), 6.73 (d, J = 8.2 Hz, 1H), 2.27 (t, J = 7.3 Hz, 2H), 1.54 (tt, J = 7.3, 7.3 Hz, 2H), 1.28 (qt, J = 7.3, 7.3 Hz, 2H), 0.87 (t, J = 7.3 Hz, 3H)

### Synthesis Example 22: Synthesis of LEU-1129

N-{(8-hydroxy-5-nitroquinolin-7-yl) [5-(trifluoromethyl)pyridin-2-yl]methyl}pentanamide (LEU-1129) was obtained in the same manner as in Synthesis Example 1 by using corresponding starting material compounds. 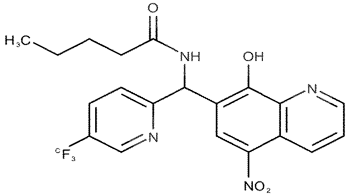
¹H-NMR (400 MHz, DMSO-d₆) δppm
9.19 (dd, J = 8.8, 1.4 Hz, 1H), 9.04 (d, J = 8.2 Hz, 1H), 9.00 (dd, J = 4.0, 1.4 Hz, 1H), 8.90 (s, 1H), 8.66 (s, 1H), 8.23 (dd, J = 8.2, 2.3 Hz, 1H), 7.90 (dd, J = 8.8, 4.0 Hz, 1H), 7.69 (d, J = 8.2 Hz, 1H), 6.78 (d, J = 8.2 Hz, 1H), 2.27 (td, J = 7.4, 3.5 Hz, 2H), 1.52 (tt, J = 7.5, 7.4 Hz, 2H), 1.27 (qt, J = 7.5, 7.3 Hz, 2H), 0.86 (t, J = 7.3 Hz, 3H)

### Synthesis Example 23: Synthesis of LEU-1140

N-[(8-hydroxy-5-nitroquinolin-7-yl)(4-methoxyphenyl)methyl]benzamide (LEU-1140) was obtained in the same manner as in Synthesis Example 1 by using corresponding starting material compounds. 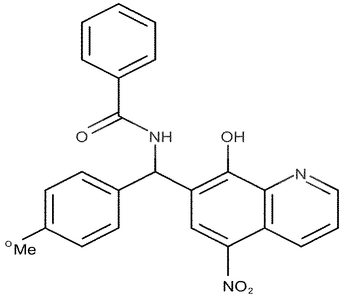
¹H-NMR (400 MHz, DMSO-d₆) δppm
9.37 (d, J = 8.4 Hz, 1H), 9.19 (dd, J = 8.8, 1.4 Hz, 1H), 9.02 (dd, J = 4.0, 1.4 Hz, 1H), 8.79 (s, 1H), 7.93-7.88 (m, 2H), 7.89 (dd, J = 8.8, 4.0 Hz, 1H), 7.58-7.45 (m, 3H), 7.27 (d, J = 8.5 Hz, 2H), 6.92 (d, J = 8.5, 2H), 6.91 (d, J = 8.4 Hz, 1H), 3.73 (s, 3H)

### Synthesis Example 24: Synthesis of LEU-1142

N-{[4-(1H-imidazol-1-yl)phenyl] (8-hydroxy-5-nitroquinolin-7-yl)methyl}pentanamide (LEU-1142) was obtained in the same manner as in Synthesis Example 1 by using corresponding starting material compounds. 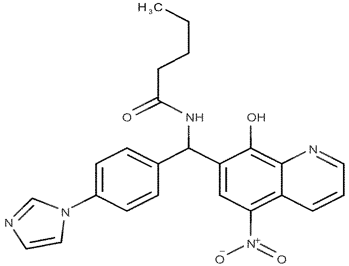
¹H-NMR (400MHz, DMSO-d₆) δppm
9.21 (dd, J = 8.7, 1.4 Hz, 1H), 8.99 (q, J = 1.8 Hz, 1H), 8.94 (d, J = 8.7 Hz, 1H), 8.69 (s, 1H), 8.22 (s, 1H), 7.88 (q, J = 4.4 Hz, 1H), 7.70 (t, J = 1.1 Hz, 1H), 7.60 (d, J = 8.7 Hz, 2H), 7.41 (d, J = 8.7 Hz, 2H), 7.09 (s, 1H), 6.68 (d, J = 8.2 Hz, 1H), 2.25 (t, J = 7.3 Hz, 2H), 1.53 (tt, J = 7.3, 7.3 Hz, 2H), 1.28 (qt, J = 7.5, 7.3 Hz, 2H), 0.87 (t, J = 7.5 Hz, 3H)
LCMS (ESI): m/z = 446.2 [M+H], RT = 2.14

### Synthesis Example 25: Synthesis of LEU-1144

N-[(8-hydroxy-5-nitroquinolin-7-yl)(4-methoxyphenyl)methyl]-2-(4-hydroxyphenyl)acetamide (LEU-1144) was obtained in the same manner as in Synthesis Example 1 by using corresponding starting material compounds. 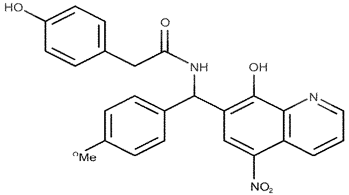
¹H-NMR (400 MHz, DMSO-d₆) δppm
9.19 (s, 1H), 9.17 (dd, J = 8.8, 1.4 Hz, 1H), 9.05 (d, J = 8.2 Hz, 1H), 9.00 (dd, J = 4.0, 1.4 Hz, 1H), 8.69 (s, 1H), 7.88 (dd, J = 8.8, 4.0 Hz, 1H), 7.18 (d, J = 8.7 Hz, 2H), 7.07 (d, J = 8.7 Hz, 2H), 6.88 (d, J = 9.1 Hz, 2H), 6.65 (d, J = 9.1 Hz, 2H), 6.55 (d, J = 8.2 Hz, 1H), 3.70 (s, 3H)

### Synthesis Example 26: Synthesis of LEU-1146

N-{[4-(1H-1,2,4-triazol-1-yl)phenyl] (8-hydroxy-5-nitroquinolin-7-yl)methyl}pentanamide (LEU-1146) was obtained in the same manner as in Synthesis Example 1 by using corresponding starting material compounds. 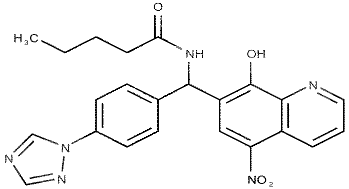
¹H-NMR (400 MHz, DMSO-d₆) δppm
9.21 (s, 1H), 9.16 (dd, J = 8.8, 1.4 Hz, 1H), 8.98 (dd, J = 4.0, 1.4 Hz, 1H), 8.93 (d, J = 8.2 Hz, 1H), 8.65 (s, 1H), 8.17 (s, 1H), 7.86 (dd, J = 8.8, 4.0 Hz, 1H), 7.77 (d, J = 8.7 Hz, 2H), 7.42 (d, J = 8.7 Hz, 2H), 6.67 (d, J = 8.2 Hz, 1H), 2.22 (t, J = 7.3 Hz, 2H), 1.49 (tt, J = 7.3, 7.3 Hz, 2H), 1.24 (qt, J = 7.3, 7.3 Hz, 2H), 0.83 (t, J = 7.3 Hz, 3H)

### Synthesis Example 27: Synthesis of LEU-1154

N-{(8-hydroxy-5-nitroquinolin-7-yl)[1H-pyrrolo(2,3,-b)pyridin-3-yl]methyl}pentanamide (LEU-1154) was obtained in the same manner as in Synthesis Example 1 by using corresponding starting material compounds. 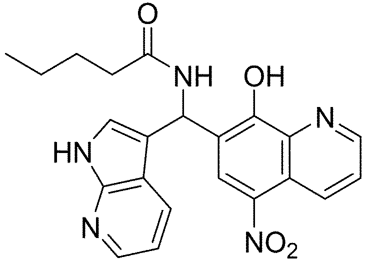
¹H-NMR (400 MHz, DMSO-d₆) δppm
11.52 (s, 1H), 9.21 (dd, J = 9.0, 1.4 Hz, 1H), 9.01 (d, J = 3.2 Hz, 1H), 8.87 (d, J = 8.5 Hz, 1H), 8.77 (s, 1H), 8.21 (dd, J = 4.6, 1.6 Hz, 1H), 7.89 (dd, J = 9.0, 4.0 Hz, 1H), 7.84 (dd, J = 8.0, 1.4 Hz, 1H), 7.06 (dd, J = 8.0, 4.6 Hz, 1H), 7.02 (d, J = 1.6 Hz, 1H), 6.89 (d, J = 8.5 Hz, 1H), 2.21 (t, J = 7.3 Hz, 2H), 1.52 (tt, J = 7.3, 7.3 Hz, 2H), 1.26 (qt, J = 7.3, 7.3 Hz, 2H), 0.85 (t, J = 7.3 Hz, 3H)

### Synthesis Example 28: Synthesis of LEU-1155

N-[(8-hydroxy-5-nitroquinolin-7-yl) (quinolin-8-yl)methyl]pentanamide (LEU-1155) was obtained in the same manner as in Synthesis Example 1 by using corresponding starting material compounds. 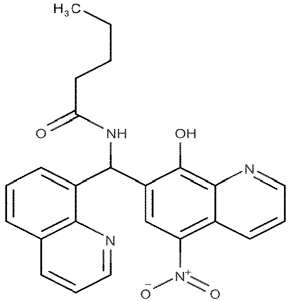
¹H-NMR (400 MHz, DMSO-d₆) δppm
9.18 (dd, J = 8.9, 1.4 Hz, 1H), 8.98 (dd, J = 4.0, 1.4 Hz, 1H), 8.91 (dd, J = 4.2, 2.0 Hz, 1H), 8.67 (d, J = 7.3 Hz, 1H), 8.49 (s, 1H), 8.40 (dd, J = 8.7, 2.0 Hz, 1H), 7.95 (dd, J = 8.0, 1.6 Hz, 1H), 7.86 (dd, J = 8.8, 4.0 Hz, 1H), 7.58-7.65 (m, 3H), 7.56 (dd, J = 8.2, 4.1 Hz, 1H), 2.16-2.24 (m, 2H), 1.48-1.55 (m, 2H), 1.28 (qt, J = 7.3, 7.3 Hz, 2H), 0.86 (t, J = 7.3 Hz, 3H)

### Synthesis Example 29: Synthesis of LEU-1168

N-{(8-hydroxy-5-nitroquinolin-7-yl)[4-(pyridin-2-yl)phenyl]methyl}pentanamide (LEU-1168) was obtained in the same manner as in Synthesis Example 1 by using corresponding starting material compounds. 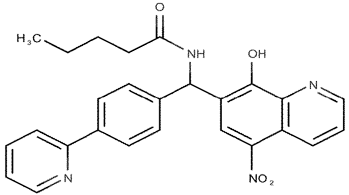
¹H-NMR (400 MHz, DMSO-d₆) δppm
9.15 (dd, J = 8.7, 1.4 Hz, 1H), 8.98 (dd, J = 4.0, 1.4 Hz, 1H), 8.93 (d, J = 8.2 Hz, 1H), 8.67 (s, 1H), 8.60 (td, J = 2.5, 0.9 Hz, 1H), 8.00 (d, J = 8.7 Hz, 2H), 7.80-7.89 (m, 3H), 7.35 (d, J = 8.2 Hz, 2H), 7.29 (ddd, J = 7.3, 4.8, 1.1 Hz, 1H), 6.68 (d, J = 8.7 Hz, 1H), 2.22 (t, J = 7.3 Hz, 2H), 1.49 (tt, J = 7.3, 7.3 Hz, 2H), 1.24 (qt, J = 7.3, 7.3 Hz, 2H), 0.83 (t, J = 7.3 Hz, 3H)

### Synthesis Example 30: Synthesis of LEU-1173

N-{[6-(1H-pyrazol-1-yl)pyridin-3-yl](8-hydroxy-5-nitroquinolin-7-yl)methyl}pentanamide (LEU-1173) was obtained in the same manner as in Synthesis Example 1 by using corresponding starting material compounds. 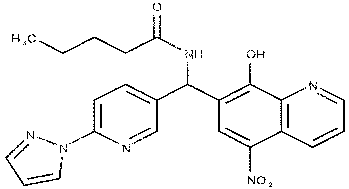
¹H-NMR (400 MHz, DMSO-d₆) δppm
9.22 (dd, J = 8.9, 1.6 Hz, 1H), 9.00-9.03 (m, 2H), 8.73 (s, 1H), 8.58 (d, J = 2.7 Hz, 1H), 8.41 (d, J = 1.8 Hz, 1H), 7.83-7.93 (m, 3H), 7.80 (d, J = 0.9 Hz, 1H), 6.68 (d, J = 8.2 Hz, 1H), 6.56 (q, J = 2.5, 1.8 Hz, 1H), 2.27 (t, J = 7.3 Hz, 2H), 1.54 (tt, J = 7.3, 7.3 Hz, 2H), 1.29 (qt, J = 7.3, 7.3 Hz, 2H), 0.87 (t, J = 7.3 Hz, 3H)

### Synthesis Example 31: Synthesis of LEU-1199

N-[(8-hydroxy-5-nitroquinolin-7-yl)(4-methoxyphenyl)methyl]cyclopropanecarboxamide (LEU-1199) was obtained in the same manner as in Synthesis Example 1 by using corresponding starting material compounds. 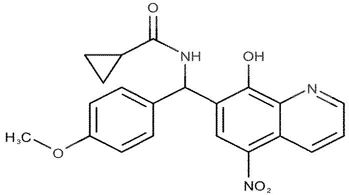
¹H-NMR (400 MHz, DMSO-d₆) δppm
9.18 (dd, J = 8.8, 1.4 Hz, 1H), 9.10 (d, J = 8.2 Hz, 1H), 8.97 (dd, J = 4.0, 1.4 Hz, 1H), 8.68 (s, 1H), 7.86 (dd, J = 8.8, 4.0 Hz, 1H), 7.19 (d, J = 8.2 Hz, 2H), 6.88 (d, J = 9.1 Hz, 2H), 6.61 (d, J = 8.7 Hz, 1H), 3.70 (s, 3H), 1.72-1.79 (m, 1H), 0.61-0.72 (m, 4H)

### Synthesis Example 32: Synthesis of LEU-1208

N-[(8-hydroxy-5-nitroquinolin-7-yl) (6-phenoxypyridin-3-yl)methyl]pentanamide (LEU-1208) was obtained in the same manner as in Synthesis Example 1 by using corresponding starting material compounds. 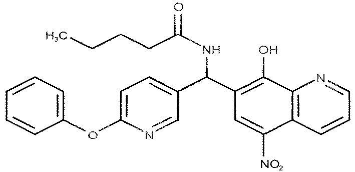
¹H-NMR (400 MHz, DMSO-d₆) δppm
9.20 (dd, J = 8.8, 1.4 Hz, 1H), 9.00 (dd, J = 4.0, 1.4 Hz, 1H), 8.95 (d, J = 8.2 Hz, 1H), 8.71 (s, 1H), 8.05 (s, 1H), 7.90 (dd, J = 8.8, 4.0 Hz, 1H), 7.73 (d, J = 8.2 Hz, 1H), 7.39 (t, J = 7.3 Hz, 2H), 7.19 (t, J = 6.9 Hz, 1H), 7.09 (d, J = 7.8 Hz, 2H), 6.99 (d, J = 8.2 Hz, 1H), 6.60 (d, J = 8.2 Hz, 1H), 2.24 (t, J = 7.3 Hz, 2H), 1.52 (tt, J = 7.3, 7.3 Hz, 2H), 1.27 (qt, J = 7.3, 7.3 Hz, 2H), 0.86 (t, J = 7.3 Hz, 3H)

### Synthesis Example 33: Synthesis of LEU-1214

N-{(8-hydroxy-5-nitroquinolin-7-yl) [3-(pyridin-4-yl)phenyl]methyl}pentanamide (LEU-1214) was obtained in the same manner as in Synthesis Example 1 by using corresponding starting material compounds. 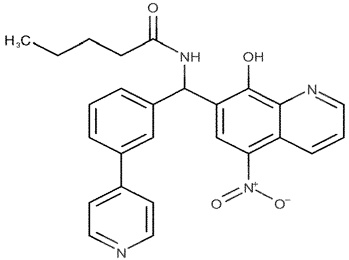
¹H-NMR (400 MHz, DMSO-d₆) δppm
9.20 (dd, J = 8.9, 1.6 Hz, 1H), 8.97-9.01 (m, 2H), 8.72 (s, 1H), 8.63 (dd, J = 4.6, 1.4 Hz, 2H), 7.89 (dd, J = 8.8, 4.0 Hz, 1H), 7.77 (d, J = 4.1 Hz, 1H), 7.70 (d, J = 7.8 Hz, 1H), 7.66 (dd, J = 4.4, 1.6 Hz, 2H), 7.49 (t, J = 7.8 Hz, 1H), 7.39 (d, J = 7.8 Hz, 1H), 6.74 (d, J = 8.2 Hz, 1H), 2.27 (t, J = 7.3 Hz, 2H), 1.53 (tt, J = 7.3, 7.3 Hz, 2H), 1.28 (qt, J = 7.3, 7.3 Hz, 2H), 0.86 (t, J = 7.3 Hz, 3H)
LCMS (ESI): m/z = 456.9 [M+H], RT = 2.44

### Synthesis Example 34: Synthesis of LEU-1216

N-{[3-(1H-pyrazol-1-yl)phenyl] (8-hydroxy-5-nitroquinolin-7-yl)methyl}pentanamide (LEU-1216) was obtained in the same manner as in Synthesis Example 1 by using corresponding starting material compounds. 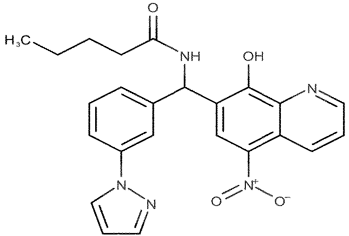
¹H-NMR (400 MHz, DMSO-d₆) δppm
9.20 (dd, J = 8.8, 1.4 Hz, 1H), 9.01 (dd, J = 4.0, 1.4 Hz, 1H), 8.99 (d, J = 8.2 Hz, 1H), 8.70 (s, 1H), 8.47 (d, J = 2.3 Hz, 1H), 7.90 (dd, J = 8.8, 4.0 Hz, 1H), 7.81 (s, 1H), 7.71-7.75 (m, 2H), 7.45 (t, J = 8.0 Hz, 1H), 7.22 (dd, J = 7.6, 0.8 Hz, 1H), 6.72 (d, J = 8.7 Hz, 1H), 6.53 (dd, J = 2.4, 1.6 Hz, 1H), 2.26 (t, J = 7.3 Hz, 2H), 1.53 (qt, J = 7.3, 7.3 Hz, 2H), 1.28 (qt, J = 7.3, 7.3 Hz, 2H), 0.86 (t, J = 7.3 Hz, 3H)
LCMS (ESI): m/z = 445.9 [M+H], RT = 3.32

### Synthesis Example 35: Synthesis of LEU-1252

N-[(8-hydroxy-5-nitroquinolin-7-yl)(4-phenoxyphenyl)methyl]pentanamide (LEU-1252) was obtained in the same manner as in Synthesis Example 1 by using corresponding starting material compounds. 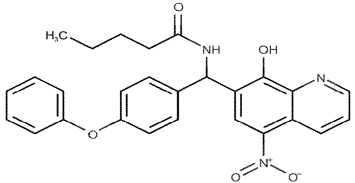
¹H-NMR (400 MHz, DMSO-d₆) δppm
9.19 (dd, J = 8.8, 1.4 Hz, 1H), 9.00 (dd, J = 4.0, 1.4 Hz, 1H), 8.91 (d, J = 8.2 Hz, 1H), 8.68 (s, 1H), 7.88 (dd, J = 8.8, 4.0 Hz, 1H), 7.37 (t, J = 8.0 Hz, 12), 7.28 (d, J = 8.7 Hz, 2H), 7.12 (t, J = 7.3 Hz, 1H), 6.96-6.99 (m, 4H), 6.63 (d, J = 8.2 Hz, 1H), 2.23 (t, J = 7.3 Hz, 2H), 1.52 (tt, J = 7.3, 7.3 Hz, 2H), 1.26 (qt, J = 7.3, 7.3 Hz, 2H), 0.86 (t, J = 7.3 Hz, 3 H)
LCMS (ESI): m/z = 472.1 [M+H], RT = 3.60

### Synthesis Example 36: Synthesis of LEU-1263

N-{(8-hydroxy-5-nitroquinolin-7-yl)[6-(o-tolyl)pyridin-3-yl]methyl}pentanamide (LEU-1263) was obtained in the same manner as in Synthesis Example 1 by using corresponding starting material compounds. 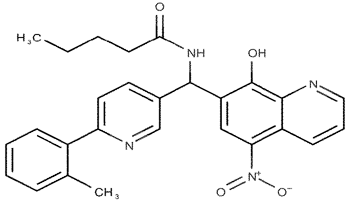
¹H-NMR (400MHz, DMSO-d₆) δppm
9.23 (dd, J = 8.8, 1.4 Hz, 1H), 9.06 (d, J = 8.2 Hz, 1H), 9.00 (dd, J = 4.0, 1.4 Hz, 1H), 8.76 (s, 1H), 8.60 (d, J = 2.3 Hz, 1H), 7.90 (dd, J = 8.8, 4.0 Hz, 1H), 7.73 (dd, J = 8.7, 2.3 Hz, 1H), 7.49 (d, J = 8.0 Hz, 1H), 7.24-7.36 (m, 4H), 6.71 (d, J = 8.2 Hz, 1H), 2.30 (s, 3 H), 2.28 (t, J = 7.3 Hz, 2H), 1.54 (qt, J = 7.3, 7.3 Hz, 2H), 1.29 (qt, J = 7.3, 7.3 Hz, 2H), 0.87 (t, J = 7.3 Hz, 3H)
LCMS (ESI): m/z = 471.1 [M+H], RT = 3.00

### Synthesis Example 37: Synthesis of LEU-1276

Phenyl[(8-hydroxy-5-nitroquinolin-7-yl)(4-methoxyphenyl)methyl]carbamate (LEU-1276) was obtained in the same manner as in Synthesis Example 1 by using corresponding starting material compounds. 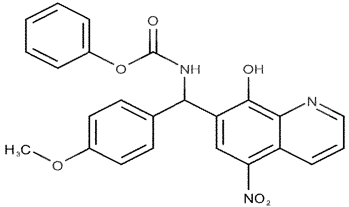
¹H-NMR (400 MHz, DMSO-d₆) δppm
9.20 (dd, J = 8.8, 1.4 Hz, 1H), 9.05 (d, J = 9.1 Hz, 1H), 9.02 (dd, J = 4.0, 1.4 Hz, 1H), 8.84 (s, 1H), 7.90 (dd, J = 8.8, 4.0 Hz, 1H), 7.37 (t, J = 7.8 Hz, 2H), 7.30 (d, J = 8.7 Hz, 2H), 7.20 (t, J = 7.3 Hz, 1H), 7.13 (d, J = 7.8 Hz, 2H), 6.92 (d, J = 9.1 Hz, 2H), 6.46 (d, J = 9.1 Hz, 1H), 3.73 (s, 3H)

### Synthesis Example 38: Synthesis of LEU-1280

N-[(8-hydroxy-5-nitroquinolin-7-yl)(4-methoxyphenyl)methyl]-2-(1,3,5-trimethyl-1H-pyrazol-4-yl)acetamide (LEU-1280) was obtained in the same manner as in Synthesis Example 1 by using corresponding starting material compounds. 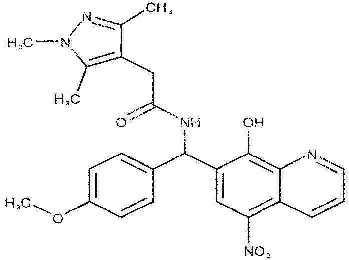
¹H-NMR (400 MHz, DMSO-d₆) δppm
9.18 (dd, J = 8.8, 1.4 Hz, 1H), 9.00 (dd, J = 4.0, 1.4 Hz, 1H), 8.96 (d, J = 8.2 Hz, 1H), 8.69 (s, 1H), 7.88 (dd, J = 8.8, 4.0 Hz, 1H), 7.18 (d, J = 8.7 Hz, 2H), 6.89 (d, J = 8.7 Hz, 2H), 6.54 (d, J = 8.2 Hz, 1H), 3.72 (s, 3H), 3.57 (s, 3H), 3.27 (d, J = 4.6 Hz, 2H), 2.13 (s, 3H), 2.02 (s, 3H)

### Synthesis Example 39: Synthesis of LEU-1288

N-{(8-hydroxy-5-nitroquinolin-7-yl)[5-(o-tolyl)pyridin-2-yl]methyl}pentanamide (LEU-1288) was obtained in the same manner as in Synthesis Example 1 by using corresponding starting material compounds. 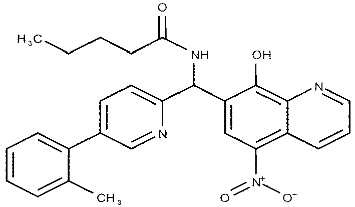
¹H-NMR (400 MHz, DMSO-d₆) δppm
9.18 (dd, J = 8.8, 1.4 Hz, 1H), 9.04 (d, J = 8.2 Hz, 1H), 8.99 (dd, J = 4.0, 1.4 Hz, 1H), 8.71 (s, 1H), 8.47 (d, J = 1.4 Hz, 1H), 7.87 (dd, J = 8.8, 4.0 Hz, 1H), 7.81 (dd, J = 8.2, 2.3 Hz, 1H), 7.51 (d, J = 8.2 Hz, 1H), 7.24-7.32 (m, 3H), 7.20 (d, J = 6.9 Hz, 1H), 6.75 (d, J = 8.2 Hz, 1H), 2.26 (t, J = 7.3 Hz, 2H), 2.21 (s, 3H), 1.51 (qt, J = 7.3, 7.3 Hz, 2H), 1.26 (qt, J = 7.3, 7.3 Hz, 2H), 0.84 (t, J = 7.3 Hz, 3H)

### Synthesis Example 40: Synthesis of LEU-1295

Tert-butyl[(8-hydroxy-5-nitroquinolin-7-yl)(4-methoxyphenyl)methyl]carbamate (LEU-1295) was obtained in the same manner as in Synthesis Example 1 by using corresponding starting material compounds. 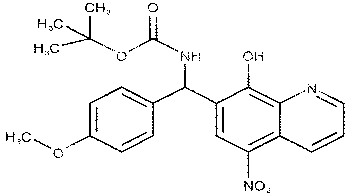
¹H-NMR (400 MHz, DMSO-d₆) δppm
9.16 (dd, J = 8.8, 1.4 Hz, 1H), 9.00 (dd, J = 4.0, 1.4 Hz, 1H), 8.79 (s, 1H), 8.14 (d, J = 9.1 Hz, 1H), 7.87 (dd, J = 8.8, 4.0 Hz, 1H), 7.22 (d, J = 8.7 Hz, 2H), 6.86 (d, J = 8.7 Hz, 2H), 6.37 (d, J = 9.1 Hz, 1H), 3.70 (s, 3H), 1.40 (s, 9H)

### Synthesis Example 41: Synthesis of LEU-1307

N-[(8-hydroxy-5-nitroquinolin-7-yl) {6-[2-(trifluoromethyl)phenyl]pyridin-3-yl}methyl]pentanamide (LEU-1307) was obtained in the same manner as in Synthesis Example 1 by using corresponding starting material compounds. 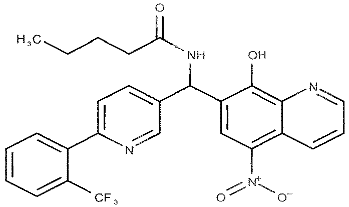
¹H-NMR (400 MHz, DMSO-d₆) δppm
9.22 (dd, J = 9.1, 1.4 Hz, 1H), 9.06 (d, J = 8.7 Hz, 1H), 8.98 (d, J = 3.7 Hz, 1H), 8.73 (s, 1H), 8.57 (d, J = 2.3 Hz, 1H), 7.88 (dd, J = 8.9, 4.3 Hz, 1H), 7.83 (d, J = 7.8 Hz, 1H), 7.75 (dd, J = 8.2, 2.3 Hz, 1H), 7.73 (t, J = 6.9 Hz, 1H), 7.64 (t, J = 7.5 Hz, 1H), 7.49 (d, J = 7.3 Hz, 1H), 7.46 (d, J = 8.2 Hz, 1H), 6.72 (d, J = 8.7 Hz, 1H), 2.26 (t, J = 7.3 Hz, 2H), 1.53 (tt, J = 7.3, 7.3 Hz, 2H), 1.27 (qt, J = 7.3, 7.3 Hz, 2H), 0.86 (t, J = 7.3 Hz, 3H)
LCMS (ESI): m/z = 525.2 [M+H], RT = 2.84

### Synthesis Example 42: Synthesis of LEU-1396

4,4-difluoro-N-[(8-hydroxy-5-nitroquinolin-7-yl) (4-methoxyphenyl)methyl]pentanamide (LEU-1396) was obtained in the same manner as in Synthesis Example 1 by using corresponding starting material compounds. 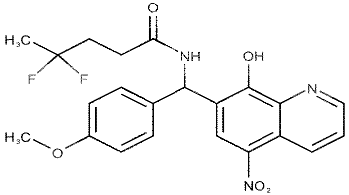
¹H-NMR (400 MHz, DMSO-d₆) δppm
9.17 (dd, J = 8.7, 1.4 Hz, 1H), 8.96-8.98 (m, 2H), 8.63 (s, 1H), 7.86 (dd, J = 8.8, 4.0 Hz, 1H), 7.18 (d, J = 8.7 Hz, 2H), 6.87 (d, J = 8.7 Hz, 2H), 6.58 (d, J = 8.2 Hz, 1H), 2.38-2.42 (m, 2H), 2.06-2.18 (m, 2H), 1.57 (t, J = 19.0 Hz, 3H)

### Synthesis Example 43: Synthesis of LEU-1408

N-[(8-hydroxy-6-methyl-5-nitroquinolin-7-yl)(4-methoxyphenyl)methyl]pentanamide (LEU-1408) was obtained in the same manner as in Synthesis Example 1 by using corresponding starting material compounds. 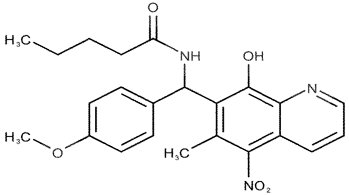
¹H-NMR (400 MHz, DMSO-d₆) δppm
11.00 (bs, 1H), 8.97 (dd, J = 4.0, 1.4 Hz, 1H), 8.42 (d, J = 8.2 Hz, 1H), 8.14 (dd, J = 8.7, 1.4 Hz, 1H), 7.76 (dd, J = 8.8, 4.0 Hz, 1H), 7.09 (d, J = 8.2 Hz, 2H), 6.88 (d, J = 8.7 Hz, 2H), 6.80 (d, J = 6.9 Hz, 1H), 3.71 (s, 3H), 2.36 (s, 3H), 2.18-2.35 (m, 5H), 1.44-1.56 (m, 2H), 1.25 (qt, J = 7.3, 7.3 Hz, 2H), 0.84 (t, J = 7.3 Hz, 3H)

### Synthesis Example 44: Synthesis of LEU-1423

(E)-N-[1-(8-hydroxy-5-nitroquinolin-7-yl)-3-phenylallyl]pentanamide (LEU-1423) was obtained in the same manner as in Synthesis Example 1 by using corresponding starting material compounds. 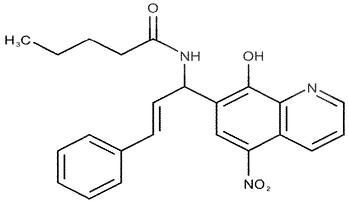
¹H-NMR (400 MHz, DMSO-d₆) δppm
9.20 (dd, J = 8.8, 1.4 Hz, 1H), 9.02 (dd, J = 4.0, 1.4 Hz, 1H), 8.77 (d, J = 8.2 Hz, 1H), 8.66 (s, 1H), 7.89 (dd, J = 8.8, 4.0 Hz, 1H), 7.41 (d, J = 7.8 Hz, 2H), 7.30 (t, J = 7.5 Hz, 2H), 7.23 (t, J = 7.3 Hz, 1H), 6.42-6.52 (m, 2H), 6.17 (dd, J = 8.2, 4.6 Hz, 1H), 2.22 (t, J = 7.3 Hz, 2H), 1.51 (tt, J = 7.3, 7.3 Hz, 2H), 1.27 (qt, J = 7.3, 7.3 Hz, 2H), 0.86 (t, J = 7.3 Hz, 3H)

### Synthesis Example 45: Synthesis of LEU-1430

N-{[4-(1H-pyrazol-1-yl)phenyl] (8-hydroxy-5-nitroquinolin-7-yl)methyl}pentanamide (LEU-1430) was obtained in the same manner as in Synthesis Example 1 by using corresponding starting material compounds. 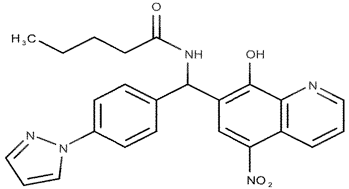
¹H-NMR (400 MHz, DMSO-d₆) δppm
9.20 (dd, J = 8.7, 1.4 Hz, 1H), 9.02 (dd, J = 4.0, 1.4 Hz, 1H), 8.96 (d, J = 8.2 Hz, 1H), 8.70 (s, 1H), 8.45 (d, J = 2.3 Hz, 1H), 7.90 (dd, J = 8.8, 4.0 Hz, 1H), 7.79 (d, J = 8.7 Hz, 2H), 7.72 (d, J = 1.8 Hz, 1H), 7.38 (d, J = 8.7 Hz, 2H), 6.69 (d, J = 8.2 Hz, 1H), 6.52 (dd, J = 2.7, 1.8 Hz, 1H), 2.25 (t, J = 7.3 Hz, 2H), 1.53 (tt, J = 7.3, 7.3 Hz, 2H), 1.28 (qt, J = 7.3, 7.3 Hz, 2H), 0.87 (t, J = 7.3 Hz, 3H)

### Synthesis Example 46: Synthesis of LEU-1436

N-[1-(8-hydroxy-5-nitroquinolin-7-yl)-3-phenylprop-2-en-1-yl]pentanamide (LEU-1436) was obtained in the same manner as in Synthesis Example 1 by using corresponding starting material compounds. 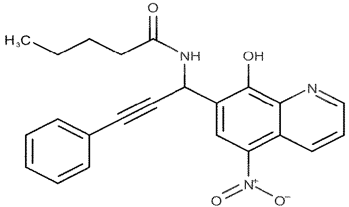
¹H-NMR (400 MHz, DMSO-d₆) δppm
9.25 (d, J = 8.7 Hz, 1H), 9.06 (d, J = 7.8 Hz, 1H), 9.02 (d, J = 4.0 Hz, 1H), 8.85 (s, 1H), 7.92 (dd, J = 8.7, 4.1 Hz, 1H), 7.36-7.44 (m, 5H), 6.48 (d, J = 7.8 Hz, 1H), 2.18 (t, J = 7.3 Hz, 2H), 1.50 (tt, J = 7.3, 7.3 Hz, 2H), 1.26 (qt, J = 7.3, 7.3 Hz, 2H), 0.85 (t, J = 7.3 Hz, 3H)
LCMS (ESI): m/z = 404.2 [M+H], RT = 3.14

### Synthesis Example 47: Synthesis of LEU-1437

N-[(6-chloro-8-hydroxy-5-nitroquinolin-7-yl)(4-methoxyphenyl)methyl]pentanamide (LEU-1437) was obtained in the same manner as in Synthesis Example 1 by using corresponding starting material compounds. 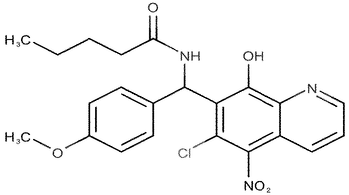
¹H-NMR (400 MHz, DMSO-d₆) δppm
9.04 (dd, J = 4.1, 1.4 Hz, 1H), 8.48 (d, J = 8.2 Hz, 1H), 8.21 (dd, J = 8.7, 1.4 Hz, 1H), 7.83 (dd, J = 8.7, 4.1 Hz, 1H), 7.18 (d, J = 8.2 Hz, 2H), 6.84-6.94 (m, 3H), 3.71 (s, 3H), 2.34-2.18 (m, 2H), 1.44-1.53 (m, 2H), 1.24 (qt, J = 7.3, 7.3 Hz, 2H), 0.84 (t, J = 7.3 Hz, 3H)

### Synthesis Example 48: Synthesis of LEU-1440

N-[1-(8-hydroxy-5-nitroquinolin-7-yl)-3-phenylpropyl]pentanamide (LEU-1440) was obtained in the same manner as in Synthesis Example 1 by using corresponding starting material compounds. 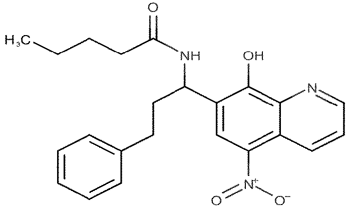
¹H-NMR (400 MHz, DMSO-d₆) δppm
9.17 (d, J = 8.7 Hz, 1H), 8.99 (d, J = 4.1 Hz, 1H), 8.60 (s, 1H), 8.57 (d, J = 8.2 Hz, 1H), 7.85 (dd, J = 8.2, 3.7 Hz, 1H), 7.14-7.27 (m, 5H), 5.39-5.32 (m, 1H), 2.78-2.56 (m, 2H), 2.20 (t, J = 7.3 Hz, 2H), 2.07-1.96 (m, 2H), 1.51 (tt, J = 7.3, 7.3 Hz, 2H), 1.27 (qt, J = 7.3, 7.3 Hz, 2H), 0.87 (t, J = 7.3 Hz, 3H)
LCMS (ESI): m/z = 408.1 [M+H], RT = 3.28

### Synthesis Example 49: Synthesis of LEU-1462

N-{[4-(1H-imidazol-1-yl)phenyl](8-hydroxy-6-methyl-5-nitroquinolin-7-yl)methyl}pentanamide (LEU-1462) was obtained in the same manner as in Synthesis Example 1 by using corresponding starting material compounds. 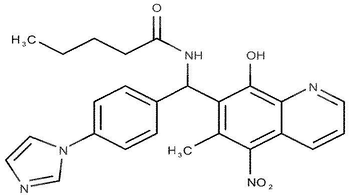
¹H-NMR (400 MHz, DMSO-d₆) δppm
8.97 (dd, J = 4.0, 1.4 Hz, 1H), 8.55 (d, J = 7.8 Hz, 1H), 8.32 (s, 1H), 8.21 (s, 1H), 8.16 (dd, J = 8.8, 1.4 Hz, 1H), 7.77 (dd, J = 8.8, 4.0 Hz, 1H), 7.70 (s, 1H), 7.59 (d, J = 8.7 Hz, 2H), 7.30 (d, J = 8.2 Hz, 2H), 7.08 (s, 1H), 6.88 (d, J = 7.3 Hz, 1H), 2.40 (s, 3H), 2.22-2.40 (m, 2H), 1.59-1.46 (m, 2H), 1.27 (qt, J = 7.3, 7.3 Hz, 2H), 0.86 (t, J = 7.3 Hz, 3H)

### Synthesis Example 50: Synthesis of LEU-1507

N-[(8-hydroxy-5-nitroquinolin-7-yl) (2-methoxypyridin-4-yl)methyl]pentanamide (LEU-1507) was obtained in the same manner as in Synthesis Example 1 by using corresponding starting material compounds. 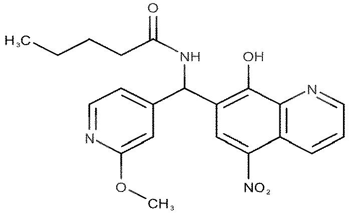
¹H-NMR (400 MHz, DMSO-d₆) δppm
9.20 (dd, J = 9.1, 1.4 Hz, 1H), 9.02 (dd, J = 4.1, 1.4 Hz, 1H), 8.95 (d, J = 8.7 Hz, 1H), 8.61 (s, 1H), 8.10 (d, J = 5.0 Hz, 1H), 7.91 (dd, J = 8.9, 4.3 Hz, 1H), 6.90 (dd, J = 5.5, 1.4 Hz, 1H), 6.68 (d, J = 1.4 Hz, 1H), 6.64 (d, J = 8.7 Hz, 1H), 3.82 (s, 3H), 2.25 (t, J = 7.3 Hz, 2H), 1.52 (tt, J = 7.3, 7.3 Hz, 2H), 1.27 (qt, J = 7.3, 7.3 Hz, 2H), 0.86 (t, J = 7.3 Hz, 3H)

### Synthesis Example 51: Synthesis of LEU-1817

N-{[6-(dimethylamino)pyridin-3-yl](8-hydroxy-5-nitroquinolin-7-yl)methyl}pentanamide (LEU-1817) was obtained in the same manner as in Synthesis Example 1 by using corresponding starting material compounds. 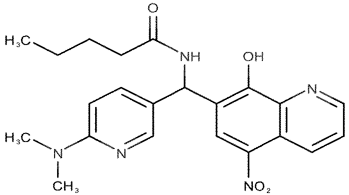
¹H-NMR (400 MHz, DMSO-d₆) δppm
9.19 (d, J = 8.7 Hz, 1H), 9.00 (t, J = 1.8 Hz, 1H), 8.82 (d, J = 7.8 Hz, 1H), 8.70 (d, J = 2.3 Hz, 1H), 7.97 (s, 1H), 7.87-7.89 (m, 1H), 7.38 (d, J = 9.1 Hz, 1H), 6.59 (d, J = 8.7 Hz, 1H), 6.47 (d, J = 8.2 Hz, 1H), 2.97 (s, 6H), 2.22 (t, J = 7.1 Hz, 2H), 1.51 (tt, J = 7.3, 7.3 Hz, 2H), 1.27 (qt, J = 7.3, 7.3 Hz, 2H), 0.86 (t, J = 7.3, 3H)

### Synthesis Example 52: Synthesis of LEU-1823

N-{[(1R,5S)-6,6-dimethylbicyclo(3.1.1)hept-2-en-2-yl] (8-hydroxy-5-nitroquinolin-7-yl)methyl}pentanamide (LEU-1823) was obtained in the same manner as in Synthesis Example 1 by using corresponding starting material compounds. 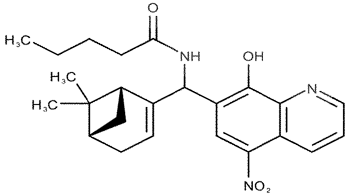
¹H-NMR (400 MHz, DMSO-d₆) δppm
9.17 (d, J = 8.7 Hz, 1H), 8.99 (d, J = 2.7 Hz, 1H), 8.62 (s, 1H), 8.50 (d, J = 9.1 Hz, 1H), 7.86 (dd, J = 8.7, 3.7 Hz, 1H), 5.96 (d, J = 8.2 Hz, 1H), 5.13 (s, 1H), 1.98-2.35 (m, 6H), 1.13-1.50 (m, 9H), 0.83 (t, J = 7.3, 3H), 0.70 (s, 3H)

### Synthesis Example 53: Synthesis of LEU-1850

N-[cyclohex-1-en-1-yl(8-hydroxy-5-nitroquinolin-7-yl)methyl]pentanamide (LEU-1850) was obtained in the same manner as in Synthesis Example 1 by using corresponding starting material compounds. 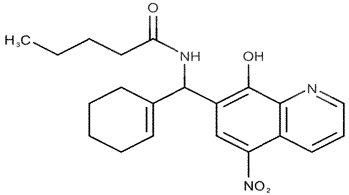
¹H-NMR (400 MHz, CDCl₃) δppm
9.30 (d, J = 8.7 Hz, 1H), 8.86 (s, 1H), 8.52 (s, 1H), 7.70 (d, J = 5.5 Hz, 1H), 7.26 (s, 1H), 6.37 (d, J = 7.8 Hz, 1H), 5.89 (d, J = 7.8 Hz, 1H), 5.54 (s, 1H), 2.25 (t, J = 7.1 Hz, 2H), 1.95-2.10 (m, 4H), 1.55-1.64 (m, 6H), 1.32 (qt, J = 7.3, 7.3 Hz, 2H), 0.90 (t, J = 6.9 Hz, 3H)

### Synthesis Example 54: Synthesis of LEU-1868

N-{(8-hydroxy-5-nitroquinolin-7-yl)[(S)-4-(prop-1-en-2-yl)cyclohex-1-en-1-yl]methyl}pentanamide (LEU-1868) was obtained in the same manner as in Synthesis Example 1 by using corresponding starting material compounds. 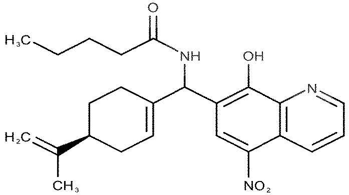
¹H-NMR (400 MHz, DMSO-d₆) δppm
9.15 (d, J = 8.7 Hz, 1H), 9.00 (d, J = 3.2 Hz, 1H), 8.55 (d, J = 9.1 Hz, 1H), 8.51 (d, J = 8.7 Hz, 1H), 7.87 (dd, J = 8.7, 4.1 Hz, 1H), 5.91 (d, J = 8.2 Hz, 1H), 5.40 (s, 1H), 4.67 (s, 2H), 2.17 (td, J = 7.2, 3.0 Hz, 2H), 1.68-2.08 (m, 6H), 1.66 (s, 3H), 1.47 (tt, J = 7.3, 7.3 Hz, 2H), 1.37-1.39 (m, 1H), 1.23 (qt, J = 7.3, 7.3 Hz, 2H), 0.83 (t, J = 7.3 Hz, 3H)

### Synthesis Example 55: Synthesis of LEU-1869

(E)-N-[1-(8-hydroxy-5-nitroquinolin-7-yl)-2-phenylbut-2-en-1-yl]pentanamide (LEU-1869) was obtained in the same manner as in Synthesis Example 1 by using corresponding starting material compounds. 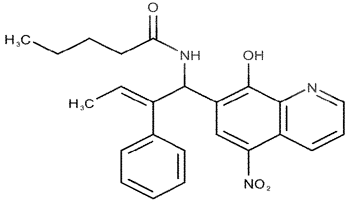
¹H-NMR (400 MHz, DMSO-d₆) δppm
9.19 (d, J = 8.7 Hz, 1H), 9.00 (d, J = 4.1 Hz, 1H), 8.64 (s, 1H), 8.54 (d, J = 8.2 Hz, 1H), 8.32 (s, 1H), 7.88 (dd, J = 8.7, 4.1 Hz, 1H), 7.19-7.39 (m, 6H), 6.30 (d, J = 8.2 Hz, 1H), 5.43 (q, J = 6.7 Hz, 1H), 2.09 (t, J = 7.3 Hz, 2H), 1.50 (d, J = 6.9 Hz, 3H), 1.39 (tt, J = 7.3, 7.3 Hz, 2H), 1.12 (qt, J = 7.3, 7.3 Hz, 2H), 0.80 (t, J = 7.3 Hz, 3H)

### Synthesis Example 56: Synthesis of LEU-1905

N-[{6-[8-oxa-3-azabicyclo(3.2.1)octan-3-yl]pyridin-3-yl}(8-hydroxy-5-nitroquinolin-7-yl)methyl]pentanamide (LEU-1905) was obtained in the same manner as in Synthesis Example 1 by using corresponding starting material compounds. 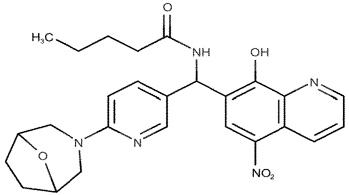
¹H-NMR (400 MHz, CDCl₃) δppm
9.30 (d, J = 8.7 Hz, 1H), 8.85 (d, J = 3.7 Hz, 1H), 8.64 (s, 1H), 8.10 (s, 1H), 7.71 (dd, J = 8.7, 3.7 Hz, 1H), 7.44 (d, J = 6.9 Hz, 1H), 6.61 (d, J = 7.3 Hz, 1H), 6.48-6.52 (m, 2H), 4.46 (s, 2H), 3.73 (d, J = 11.9 Hz, 2H), 3.07 (d, J = 11.9 Hz, 2H), 2.26 t, J = 7.3 Hz, 2H), 1.86-1.93 (m, 2H), 1.79-1.81 (m, 2H), 1.65 (tt, J = 7.3, 7.3 Hz, 2H), 1.36 (qt, J = 7.3, 7.3 Hz, 2H), 0.91 (t, J = 7.3 Hz, 3H)

### Synthesis Example 57: Synthesis of LEU-1908

N-[(2H-chromen-3-yl)(8-hydroxy-5-nitroquinolin-7-yl)methyl]pentanamide (LEU-1908) was obtained in the same manner as in Synthesis Example 1 by using corresponding starting material compounds. 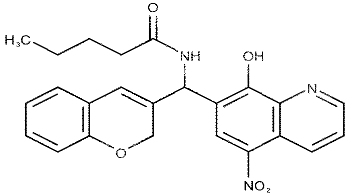
¹H-NMR (400 MHz, DMSO-d₆) δppm
9.23 (dd, J = 8.7, 1.4 Hz, 1H), 9.04 (dd, J = 4.3, 1.6 Hz, 1H), 8.81 (d, J = 8.7 Hz, 1H), 8.67 (s, 1H), 7.92 (dd, J = 8.7, 4.1 Hz, 1H), 7.09 (td, J = 7.7, 1.5 Hz, 1H), 7.01 (dd, J = 7.5, 1.6 Hz, 1H), 6.83 (td, J = 7.3, 0.9 Hz, 1H), 6.77 (d, J = 7.8 Hz, 1H), 6.15 (s, 1H), 6.11 (d, J = 8.7 Hz, 1H), 4.71 (q, J = 14.5 Hz, 2H), 2.23 (td, J = 7.3, 2.3 Hz, 2H), 1.52 (tt, J = 7.3, 7.3 Hz, 2H), 1.27 (qt, J = 7.3, 7.3 Hz, 2H), 0.87 (t, J = 7.3 Hz, 3H)

### Synthesis Example 58: Synthesis of LEU-1910

N-{ (8-hydroxy-5-nitroquinolin-7-yl) [6- (2,2, 6, 6-tetramethylmorpholino)pyridin-3-yl]methyl}pentanamide (LEU-1910) was obtained in the same manner as in Synthesis Example 1 by using corresponding starting material compounds. 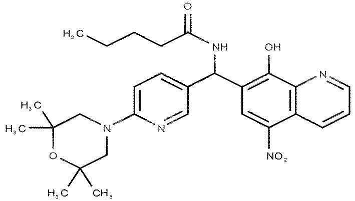
¹H-NMR (400 MHz, CDCl₃) δppm
9.31 (d, J = 8.7 Hz, 1H), 8.86 (d, J = 4.1 Hz, 1H), 8.65 (s, 1H), 8.07 (d, J = 2.3 Hz, 1H), 7.72 (dd, J = 8.9, 3.9 Hz, 1H), 7.44 (dd, J = 8.9, 2.5 Hz, 1H), 6.50-6.60 (m, 3H), 3.38 (s, 4H), 2.30 (t, J = 7.3 Hz, 2H), 1.66 (tt, J = 7.3, 7.3 Hz, 2H), 1.36 (qt, J = 7.3, 7.3 Hz, 2H), 1.24 (s, 12H), 0.91 (t, J = 7.3 Hz, 3H)

### Synthesis Example 59: Synthesis of LEU-1918

N-{(8-hydroxy-5-nitroquinolin-7-yl)[5-oxo-2,3-dihydro-1H,5H-pyrido(3,2,1-ij)quinolin-6-yl]methyl}pentanamide (LEU-1918) was obtained in the same manner as in Synthesis Example 1 by using corresponding starting material compounds. 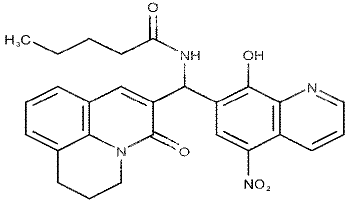
¹H-NMR (400 MHz, CDCl₃) δppm
9.21 (dd, J = 8.7, 1.4 Hz, 1H), 8.96 (d, J = 2.3 Hz, 1H), 8.75 (t, J = 1.8 Hz, 1H), 8.27 (d, J = 9.1 Hz, 1H), 8.13 (s, 1H), 7.61 (ddd, J = 8.9, 4.1, 2.1 Hz, 1H), 7.50 (d, J = 7.8 Hz, 1H), 7.29 (d, J = 7.3 Hz, 1H), 7.16 (t, J = 7.5 Hz, 1H), 6.76 (d, J = 9.1 Hz, 1H), 4.07-4.18 (m, 2H), 2.88-2.98 (m, 2H), 2.38 (t, J = 7.3 Hz, 2H), 2.01-2.17 (m, 2H), 1.70 (tt, J = 7.3, 7.3 Hz, 2H), 1.40 (qt, J = 7.3, 7.3 Hz, 2H), 0.95 (t, J = 7.3 Hz, 3H)

### Synthesis Example 60: Synthesis of LEU-1045

N-[(8-hydroxy-5-nitroquinolin-7-yl)(4-methoxyphenyl)methyl]tetrahydro-2H-pyran-4-carboxamide (LEU-1045) was obtained in the same manner as in Synthesis Example 1 by using corresponding starting material compounds. 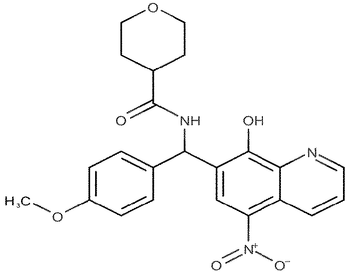
¹H-NMR (400 MHz, DMSO-d₆) δppm
9.18 (d, J = 8.7 Hz, 1H), 9.00 (t, J = 2.3 Hz, 1H), 8.84 (d, J = 8.2 Hz, 1H), 8.67 (s, 1H), 7.88 (dd, J = 8.7, 4.1 Hz, 1H), 7.19 (d, J = 8.7 Hz, 2H), 6.88 (d, J = 8.7 Hz, 2H), 6.58 (d, J = 8.2 Hz, 1H), 3.82-3.89 (m, 2H), 3.71 (s, 3H), 3.35-3.25 (m, 2H), 1.51-1.65 (m, 5H)
LCMS (ESI): m/z = 437.9 [M+H], RT = 2.24

### Synthesis Example 61: Synthesis of LEU-1046

2-ethoxy-N-[(8-hydroxy-5-nitroquinolin-7-yl) (4-methoxyphenyl)methyl]acetamide (LEU-1046) was obtained in the same manner as in Synthesis Example 1 by using corresponding starting material compounds. 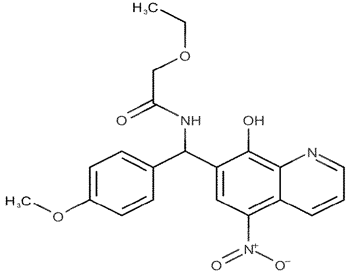
¹H-NMR (400 MHz, DMSO-d₆) δppm
9.20 (d, J = 8.7 Hz, 1H), 9.00 (dd, J = 4.1, 1.4 Hz, 1H), 8.82 (d, J = 8.7 Hz, 1H), 7.89 (dd, J = 8.9, 4.3 Hz, 1H), 7.22 (d, J = 8.7 Hz, 2H), 6.89 (d, J = 8.7 Hz, 2H), 6.62 (d, J = 8.7 Hz, 1H), 3.96 (d, J = 2.3 Hz, 2H), 3.71 (s, 3H), 3.51 (q, J = 7.0 Hz, 2H), 1.15 (t, J = 7.1 Hz, 3H)
LCMS (ESI): m/z = 411.9 [M+H], RT = 2.49

### Synthesis Example 62: Synthesis of LEU-1064

1-[(8-hydroxy-5-nitroquinolin-7-yl)(4-methoxyphenyl)methyl]urea (LEU-1064) was obtained in the same manner as in Synthesis Example 1 by using corresponding starting material compounds. 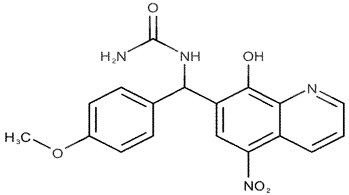
¹H-NMR (400 MHz, DMSO-d₆) δppm
9.18 (d, J = 8.7 Hz, 1H), 8.98 (dd, J = 4.1, 1.4 Hz, 1H), 8.64 (d, J = 1.4 Hz, 1H), 7.86 (dd, J = 8.9, 4.3 Hz, 1H), 7.18 (d, J = 8.7 Hz, 2H), 7.09 (d, J = 8.2 Hz, 1H), 6.86 (d, J = 8.7 Hz, 2H), 6.29 (d, J = 8.2 Hz, 1H), 5.63 (s, 2H), 3.69 (s, 3H)

### Synthesis Example 63: Synthesis of LEU-1099

Cyclohexyl [(8-hydroxy-5-nitroquinolin-7-yl) (4-methoxyphenyl)methyl]carbamate (LEU-1099) was obtained in the same manner as in Synthesis Example 1 by using corresponding starting material compounds. 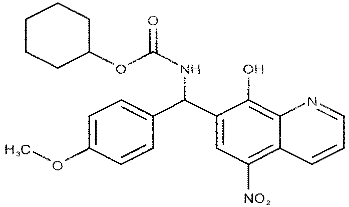
¹H-NMR (400 MHz, DMSO-d₆) δppm
9.12 (dd, J = 8.9, 1.6 Hz, 1H), 8.96 (dd, J = 4.1, 1.8 Hz, 1H), 8.74 (s, 1H), 8.31 (d, J = 9.1 Hz, 1H), 7.84 (dd, J = 8.7, 4.1 Hz, 1H), 7.18 (d, J = 8.7 Hz, 2H), 6.83 (dd, J = 9.1, 2.3 Hz, 2H), 6.36 (d, J = 9.1 Hz, 1H), 4.41-4.53 (m, 1H), 3.66 (s, 3H), 1.13-1.78 (m, 10H)

### Synthesis Example 64: Synthesis of LEU-1166

N-[(8-hydroxy-2-methyl-5-nitroquinolin-7-yl)(4-methoxyphenyl)methyl]pentanamide (LEU-1166) was obtained in the same manner as in Synthesis Example 1 by using corresponding starting material compounds. 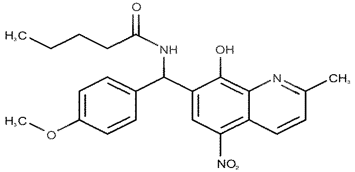
¹H-NMR (400 MHz, CDCl₃) δppm
9.19 (d, J = 9.1 Hz, 1H), 8.58 (s, 1H), 7.57 (d, J = 8.7 Hz, 1H), 7.23 (d, J = 8.7 Hz, 2H), 6.85 (d, J = 8.7 Hz, 2H), 6.55 (s, 2H), 3.78 (s, 3H), 2.75 (s, 3H), 2.30 (t, J = 7.3 Hz, 2H), 1.67 (tt, J = 7.3, 7.3 Hz, 2H), 1.37 (qt, J = 7.3, 7.3 Hz, 2H), 0.92 (t, J = 7.3 Hz, 3H)

### Synthesis Example 65: Synthesis of LEU-1170

A mixture of 8-hydroxy-5-nitroquinoline (83 mg, 0.437 mmol), 6-morpholinonicotinaldehyde (84 mg, 0.437 mmol), and valeramide (44.2 mg, 0.437 mmol) was heated at 135°C for 3 hours. The reaction mixture was cooled to room temperature and recrystallized with ethyl acetate to give N-[(8-hydroxy-5-nitroquinolin-7-yl) (6-morpholinopyridin-3-yl)methyl]pentanamide (LEU-1170) (amount: 82.2 mg, yield: 40%). 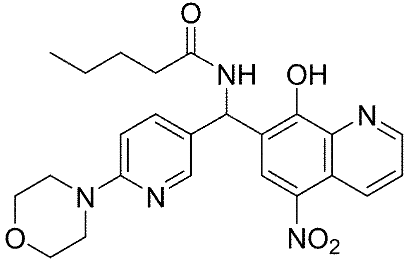
¹H-NMR (400 MHz, DMSO-d₆) δppm
9.19 (d, J = 9.1 Hz, 1H), 9.00 (d, J = 3.2 Hz, 1H), 8.84 (d, J = 8.2 Hz, 1H), 8.70 (s, 1H), 8.03 (d, J = 2.7 Hz, 1H), 7.88 (dd, J = 8.7, 4.1 Hz, 1H), 7.44 (dd, J = 8.7, 2.3 Hz, 1H), 6.79 (d, J = 8.7 Hz, 1H), 6.50 (d, J = 8.2 Hz, 1H), 3.65-3.69 (m, 4H), 3.37-3.39 (m, 4H), 2.22 (td, J = 7.3, 2.4 Hz, 2H), 1.50 (tt, J = 7.3, 7.3 Hz, 2H), 1.27 (qt, J = 7.3, 7.3 Hz, 2H), 0.86 (t, J = 7.3 Hz, 3H)

### Synthesis Example 66: Synthesis of LEU-1171

N-[(6-chloropyridin-3-yl)(8-hydroxy-5-nitroquinolin-7-yl)methyl]pentanamide (LEU-1171) was obtained in the same manner as in Synthesis Example 1 by using corresponding starting material compounds. 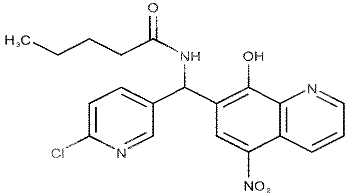
¹H-NMR (400 MHz, DMSO-d₆) δppm
9.22 (dd, J = 8.7, 1.4 Hz, 1H), 8.98-9.02 (m, 2H), 8.69 (s, 1H), 8.36 (d, J = 2.7 Hz, 1H), 7.91 (dd, J = 8.9, 4.3 Hz, 1H), 7.74 (dd, J = 8.5, 2.5 Hz, 1H), 7.49 (d, J = 8.2 Hz, 1H), 6.63 (d, J = 8.2 Hz, 1H), 2.25 (t, J = 7.3 Hz, 2H), 1.53 (tt, J = 7.3, 7.3 Hz, 2H), 1.27 (qt, J = 7.3, 7.3 Hz, 2H), 0.86 (t, J = 7.3 Hz, 3H)

### Synthesis Example 67: Synthesis of LEU-1175

N-[(8-hydroxy-5-nitroquinolin-7-yl)(4-methoxyphenyl)methyl]nicotinamide (LEU-1175) was obtained in the same manner as in Synthesis Example 1 by using corresponding starting material compounds. 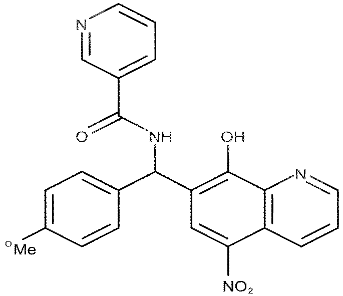
¹H-NMR (400 MHz, DMSO-d₆) δppm
9.56 (d, J = 8.2 Hz, 1H), 9.19 (dd, J = 8.7, 1.4 Hz, 1H), 9.07 (d, J = 2.3 Hz, 1H), 9.03 (dd, J = 4.1, 1.4 Hz, 1H), 8.76 (s, 1H), 8.73 (dd, J = 4.8, 1.6 Hz, 1H), 8.26 (dt, J = 7.9, 1.9 Hz, 1H), 7.90 (dd, J = 9.1, 4.1 Hz, 1H), 7.51-7.55 (m, 1H), 7.29 (d, J = 8.7 Hz, 2H), 6.90-6.94 (m, 3H), 3.73 (s, 3H)

### Synthesis Example 68: Synthesis of LEU-1176

N-[(8-hydroxy-5-nitroquinolin-7-yl)(4-methoxyphenyl)methyl]isonicotinamide (LEU-1176) was obtained in the same manner as in Synthesis Example 1 by using corresponding starting material compounds. 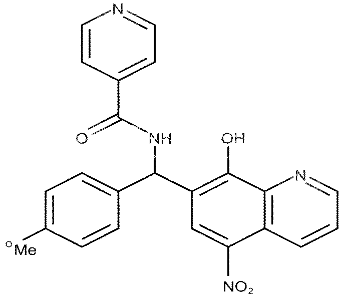
¹H-NMR (400 MHz, DMSO-d₆) δppm
9.64 (d, J = 8.7 Hz, 1H), 9.19 (d, J = 8.7 Hz, 1H), 9.03 (d, J = 3.7 Hz, 1H), 8.73-8.76 (m, 3H), 7.91 (dd, J = 9.1, 4.1 Hz, 1H), 7.83 (d, J = 5.5 Hz, 2H), 7.28 (d, J = 8.7 Hz, 2H), 6.93 (d, J = 8.7 Hz, 2H), 6.90 (d, J = 8.7 Hz, 1H), 3.74 (s, 3H)

### Synthesis Example 69: Synthesis of LEU-1198

N-{[6-(1H-imidazol-1-yl)pyridin-3-yl](8-hydroxy-5-nitroquinolin-7-yl)methyl}pentanamide (LEU-1198) was obtained in the same manner as in Synthesis Example 1 by using corresponding starting material compounds. 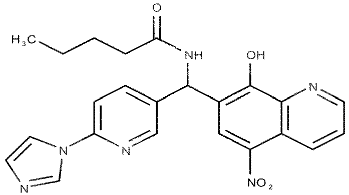
¹H-NMR (400 MHz, DMSO-d₆) δppm
9.23 (d, J = 8.7 Hz, 1H), 9.00-9.02 (m, 2H), 8.72 (s, 1H), 8.51 (s, 1H), 8.44 (d, J = 1.8 Hz, 1H), 7.88-7.92 (m, 3H), 7.78 (d, J = 8.7 Hz, 1H), 7.12 (s, 1H), 6.68 (d, J = 8.2 Hz, 1H), 2.27 (t, J = 7.3 Hz, 2H), 1.54 (tt, J = 7.3, 7.3 Hz, 2H), 1.29 (qt, J = 7.3, 7.3 Hz, 2H), 0.87 (t, J = 7.3 Hz, 3H)

### Synthesis Example 70: Synthesis of LEU-1217

N-{(8-hydroxy-5-nitroquinolin-7-yl) [4-(thiazol-2-yl)phenyl]methyl}pentanamide (LEU-1217) was obtained in the same manner as in Synthesis Example 1 by using corresponding starting material compounds. 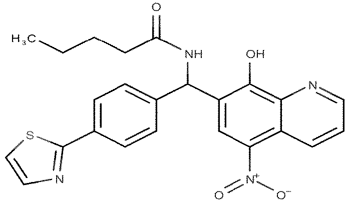
¹H-NMR (400 MHz, DMSO-d₆) δppm
9.20 (dd, J = 9.1, 1.4 Hz, 1H), 9.02 (dd, J = 4.3, 1.6 Hz, 1H), 8.98 (d, J = 8.2 Hz, 1H), 8.69 (s, 1H), 7.88-7.95 (m, 4H), 7.77 (d, J = 3.2 Hz, 1H), 7.40 (d, J = 8.2 Hz, 2H), 6.70 (d, J = 8.7 Hz, 1H), 2.26 (t, J = 7.3 Hz, 2H), 1.52 (tt, J = 7.3, 7.3 Hz, 2H), 1.29 (qt, J = 7.3, 7.3 Hz, 2H), 0.87 (t, J = 7.3 Hz, 4H)

### Synthesis Example 71: Synthesis of LEU-1219

N-[(8-hydroxy-5-nitroquinolin-7-yl) (2-phenylpyrimidin-5-yl)methyl]pentanamide (LEU-1219) was obtained in the same manner as in Synthesis Example 1 by using corresponding starting material compounds. 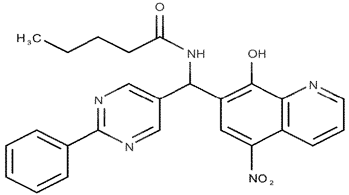
¹H-NMR (400 MHz, DMSO-d₆) δppm
9.25 (dd, J = 8.7, 1.4 Hz, 1H), 9.07 (d, J = 8.2 Hz, 1H), 9.00 (dd, J = 4.3, 1.6 Hz, 1H), 8.83 (s, 2H), 8.75 (s, 1H), 8.34-8.37 (m, 2H), 7.92 (dd, J = 8.9, 4.3 Hz, 1H), 7.50-7.54 (m, 3H), 6.64 (d, J = 8.2 Hz, 1H), 2.29 (t, J = 7.3 Hz, 2H), 1.56 (tt, J = 7.3, 7.3 Hz, 2H), 1.30 (qt, J = 7.3, 7.3 Hz, 2H), 0.88 (t, J = 7.3 Hz, 3H)

### Synthesis Example 72: Synthesis of LEU-1221

1-cyclopropyl-3-[(8-hydroxy-5-nitroquinolin-7-yl) (4-methoxyphenyl)methyl]urea (LEU-1221) was obtained in the same manner as in Synthesis Example 1 by using corresponding starting material compounds. 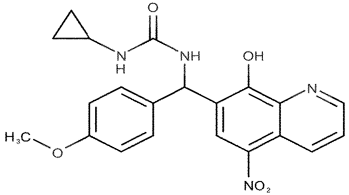
¹H-NMR (400 MHz, DMSO-d₆) δppm
9.18 (dd, J = 8.9, 1.6 Hz, 1H), 9.00 (dd, J = 4.1, 1.4 Hz, 1H), 8.67 (s, 1H), 7.88 (dd, J = 8.7, 4.1 Hz, 1H), 7.20 (d, J = 8.2 Hz, 2H), 7.00 (d, J = 8.2 Hz, 1H), 6.87 (dt, J = 9.3, 2.5 Hz, 2H), 6.37 (d, J = 8.2 Hz, 1H), 6.31 (s, 1H), 3.70 (s, 3H), 2.44-2.49 (m, 1H), 0.53-0.60 (m, 2H), 0.31-0.38 (m, 2H)

### Synthesis Example 73: Synthesis of LEU-1222

1-cyclobutyl-3-[(8-hydroxy-5-nitroquinolin-7-yl) (4-methoxyphenyl)methyl]urea (LEU-1222) was obtained in the same manner as in Synthesis Example 1 by using corresponding starting material compounds. 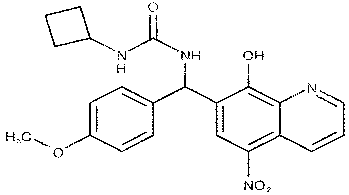
¹H-NMR (400 MHz, DMSO-d₆) δppm
9.18 (dd, J = 8.9, 1.6 Hz, 1H), 8.99 (dd, J = 4.1, 1.8 Hz, 1H), 8.63 (s, 1H), 7.88 (dd, J = 8.9, 4.3 Hz, 1H), 7.17 (dd, J = 9.1, 2.3 Hz, 2H), 6.85-6.92 (m, 3H), 6.30 (dd, J = 22.2, 8.5 Hz, 2H), 4.00-4.07 (m, 1H), 3.70 (s, 3H), 2.09-2.17 (m, 2H), 1.72-1.83 (m, 2H), 1.48-1.61 (m, 2H)

### Synthesis Example 74: Synthesis of LEU-1298

N-[(6-ethoxypyridin-3-yl)(8-hydroxy-5-nitroquinolin-7-yl)methyl]pentanamide (LEU-1298) was obtained in the same manner as in Synthesis Example 1 by using corresponding starting material compounds. 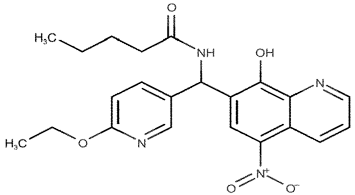
¹H-NMR (400 MHz, DMSO-d₆) δppm
9.19 (dd, J = 8.7, 1.4 Hz, 1H), 8.97 (dd, J = 4.1, 1.4 Hz, 1H), 8.89 (d, J = 8.2 Hz, 1H), 8.68 (s, 1H), 8.03 (d, J = 2.7 Hz, 1H), 7.87 (dd, J = 8.7, 4.1 Hz, 1H), 7.56 (dd, J = 8.7, 2.7 Hz, 1H), 6.73 (d, J = 8.7 Hz, 1H), 6.54 (d, J = 8.2 Hz, 1H), 4.24 (q, J = 7.0 Hz, 2H), 2.22 (td, J = 7.3, 2.0 Hz, 2H), 1.50 (tt, J = 7.3, 7.3 Hz, 2H), 1.22-1.30 (m, 5H), 0.85 (t, J = 7.3 Hz, 3H)

### Synthesis Example 75: Synthesis of LEU-1367

N-{[8-hydroxy-5-(methylsulfonyl)quinolin-7-yl](4-methoxyphenyl)methyl}-2-(4-hydroxyphenyl)acetamide (LEU-1367) was obtained in the same manner as in Synthesis Example 1 by using corresponding starting material compounds. 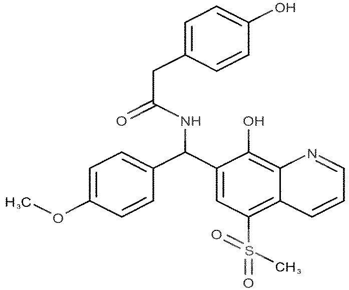
¹H-NMR (400 MHz, DMSO-d₆) δppm
9.19 (s, 1H), 9.09 (d, J = 8.2 Hz, 1H), 9.00 (d, J = 3.2 Hz, 1H), 8.94 (dd, J = 8.7, 1.4 Hz, 1H), 8.31 (s, 1H), 7.79 (dd, J = 8.7, 4.1 Hz, 1H), 7.16 (d, J = 8.2 Hz, 2H), 7.07 (d, J = 8.7 Hz, 2H), 6.88 (dt, J = 9.5, 2.5 Hz, 2H), 6.65 (dt, J = 9.1, 2.4 Hz, 2H), 6.56 (d, J = 8.2 Hz, 1H), 3.71 (s, 3H), 3.41 (dd, J = 39.3, 13.7 Hz, 2H), 3.25 (s, 3H)

### Synthesis Example 76: Synthesis of LEU-1442

(E)-N-{3-[6-(1H-imidazol-1-yl)pyridin-3-yl]-1-(8-hydroxy-5-nitroquinolin-7-yl)aryl}pentanamide (LEU-1442) was obtained in the same manner as in Synthesis Example 1 by using corresponding starting material compounds. 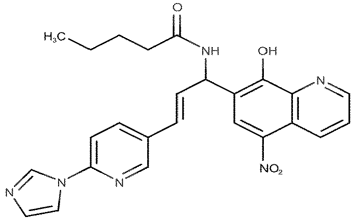
¹H-NMR (400 MHz, DMSO-d₆) δppm
9.23 (d, J = 8.7 Hz, 1H), 8.98 (s, 1H), 8.78 (d, J = 7.8 Hz, 1H), 8.64 (d, J = 10.1 Hz, 1H), 8.51 (d, J = 10.1 Hz, 2H), 8.13 (d, J = 8.7 Hz, 1H), 7.95 (d, J = 1.4 Hz, 1H), 7.87 (dd, J = 8.7, 4.1 Hz, 1H), 7.78 (d, J = 8.7 Hz, 1H), 7.11 (s, 1H), 6.66 (dd, J = 16.0, 5.9 Hz, 1H), 6.52 (d, J = 16.0 Hz, 1H), 6.20 (t, J = 6.9 Hz, 1H), 2.23 (t, J = 7.3 Hz, 2H), 1.52 (tt, J = 7.3, 7.3 Hz, 2H), 1.27 (qt, J = 7.3, 7.3 Hz, 2H), 0.87 (t, J = 7.3 Hz, 3H)

### Synthesis Example 77: Synthesis of LEU-1461

N-{[4-(1H-imidazol-1-yl)phenyl][8-hydroxy-5-(methylsulfonyl)quinolin-7-yl]methyl}pentanamide (LEU-1461) was obtained in the same manner as in Synthesis Example 1 by using corresponding starting material compounds. 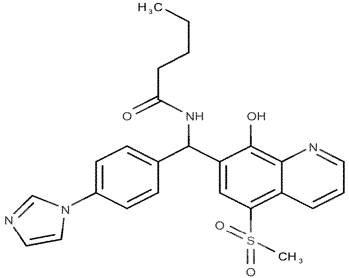
¹H-NMR (400 MHz, DMSO-d₆) δppm
8.92-8.99 (m, 3H), 8.20 (s, 1H), 7.69 (t, J = 1.4 Hz, 1H), 7.59 (d, J = 8.7 Hz, 2H), 7.39 (d, J = 8.7 Hz, 2H), 7.08-7.09 (m, 1H), 2.24 (t, J = 7.3 Hz, 2H), 1.51 (tt, J = 7.3, 7.3 Hz, 2H), 1.27 (qt, J = 7.3, 7.3 Hz, 2H), 0.87 (t, J = 7.3 Hz, 3H)
LCMS (ESI): m/z = 479.2 [M+H], RT = 1.85

### Synthesis Example 78: Synthesis of LEU-1474

N-{(8-hydroxy-5-nitroquinolin-7-yl) [6-(trifluoromethyl)pyridin-3-yl]methyl}-2-(1,3,5-trimethyl-1H-pyrazol-4-yl)acetamide (LEU-1474) was obtained in the same manner as in Synthesis Example 1 by using corresponding starting material compounds. 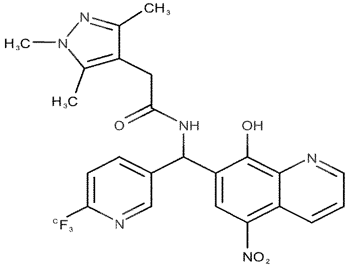
¹H-NMR (400 MHz, DMSO-d₆) δppm
9.25 (dd, J = 8.7, 1.4 Hz, 1H), 9.15 (d, J = 8.2 Hz, 1H), 9.00 (dd, J = 4.1, 1.4 Hz, 1H), 8.74 (d, J = 1.8 Hz, 1H), 8.73 (s, 1H), 7.88-7.96 (m, 3H), 6.70 (d, J = 8.2 Hz, 1H), 3.58 (s, 3H), 3.32 (dd, J = 17.4, 15.6 Hz, 2H), 2.14 (s, 3H), 2.03 (s, 3H)

### Synthesis Example 79: Synthesis of LEU-1475

N-{[4-(1H-pyrazol-1-yl)phenyl](8-hydroxy-5-nitroquinolin-7-yl)methyl}-2-(1,3,5-trimethyl-1H-pyrazol-4-yl)acetamide (LEU-1475) was obtained in the same manner as in Synthesis Example 1 by using corresponding starting material compounds. 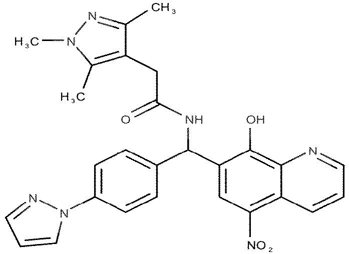
¹H-NMR (400 MHz, DMSO-d₆) δppm
9.21 (dd, J = 8.7, 1.4 Hz, 1H), 9.07 (d, J = 8.2 Hz, 1H), 9.00 (dd, J = 4.1, 1.4 Hz, 1H), 8.72 (s, 1H), 8.25 (s, 1H), 7.89 (dd, J = 8.7, 4.1 Hz, 1H), 7.72 (t, J = 1.4 Hz, 1H), 7.62 (d, J = 8.7 Hz, 2H), 7.41 (d, J = 8.2 Hz, 2H), 7.11 (t, J = 1.1 Hz, 1H), 6.65 (d, J = 8.2 Hz, 1H), 3.57 (s, 3H), 3.30 (dd, J = 17.4, 15.6 Hz, 2H), 2.14 (s, 3H), 2.03 (s, 3H)

### Synthesis Example 80: Synthesis of LEU-1009

7-[cyclohexyl(thiazol-2-ylamino)methyl]quinolin-8-ol (LEU-1009) was obtained in the same manner as in Synthesis Example 1 by using corresponding starting material compounds. 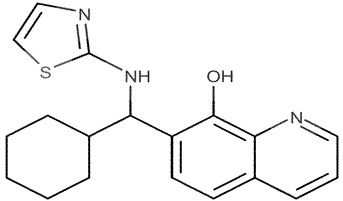
¹H-NMR (400 MHz, DMSO-d₆) δppm
9.75 (s, 1H), 8.83 (dd, J = 4.1, 1.8 Hz, 1H), 8.26 (dd, J = 8.2, 1.8 Hz, 1H), 8.07 (d, J = 8.2 Hz, 1H), 7.49-7.53 (m, 2H), 7.35 (d, J = 8.2 Hz, 1H), 6.89 (d, J = 3.2 Hz, 1H), 6.47 (d, J = 3.7 Hz, 1H), 5.04 (t, J = 8.2 Hz, 1H), 1.07-1.93 (m, 11H)
LCMS (ESI): m/z = 339.9 [M+H], RT = 2.19

### Synthesis Example 81: Synthesis of LEU-1059

N-[(5-chloro-8-hydroxyquinolin-7-yl)(4-methoxyphenyl)methyl]-2-ethoxyacetamide (LEU-1059) was obtained in the same manner as in Synthesis Example 1 by using corresponding starting material compounds. 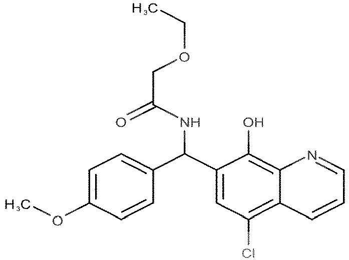
¹H-NMR (400 MHz, CDCl₃) δppm
8.82 (dd, J = 4.3, 1.6 Hz, 1H), 8.50 (dd, J = 8.5, 1.6 Hz, 1H), 8.05 (d, J = 8.7 Hz, 1H), 7.56 (dd, J = 8.5, 4.3 Hz, 1H), 7.55 (s, 1H), 7.28 (d, J = 9.1 Hz, 2H), 6.85 (d, J = 9.1 Hz, 2H), 6.54 (d, J = 8.7 Hz, 1H), 4.02 (dd, J = 28.1, 15.3 Hz, 2H), 3.77 (s, 3H), 3.60 (q, J = 7.0 Hz, 2H), 1.26 (t, J = 6.9 Hz, 3H)
LCMS (ESI): m/z = 400.8 [M+H], RT = 2.81

### Synthesis Example 82: Synthesis of LEU-1102

N-[(8-hydroxy-5-nitroquinolin-7-yl)(pyridin-3-yl)methyl]pentanamide (LEU-1102) was obtained in the same manner as in Synthesis Example 1 by using corresponding starting material compounds. 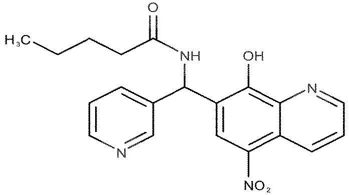
¹H-NMR (400 MHz, DMSO-d₆)
δppm 9.20 (dd, J = 8.7, 1.4 Hz, 1H), 8.96-9.00 (m, 2H), 8.69 (s, 1H), 8.51 (d, J = 2.3 Hz, 1H), 8.46 (dd, J = 4.8, 1.6 Hz, 1H), 7.89 (dd, J = 9.1, 4.1 Hz, 1H), 7.65 (dt, J = 8.1, 1.7 Hz, 1H), 7.35 (dd, J = 8.2, 5.0 Hz, 1H), 6.65 (d, J = 8.2 Hz, 1H), 2.24 (t, J = 7.1 Hz, 2H), 1.51 (tt, J = 7.3, 7.3 Hz, 2H), 1.27 (qt, J = 7.3, 7.3 Hz, 2H), 0.85 (t, J = 7.3 Hz, 3H)

### Synthesis Example 83: Synthesis of LEU-1107

2-ethoxy-N-[(8-hydroxy-5-nitroquinolin-7-yl)(pyridin-3-yl)methyl]acetamide (LEU-1107) was obtained in the same manner as in Synthesis Example 1 by using corresponding starting material compounds. 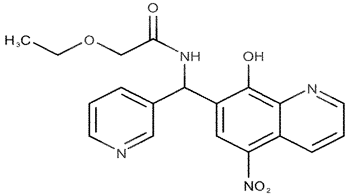
¹H-NMR (400 MHz, DMSO-d₆) δppm
9.24 (d, J = 9.1 Hz, 1H), 9.03 (d, J = 8.7 Hz, 1H), 8.94 (s, 1H), 8.75 (s, 1H), 8.53 (d, J = 2.3 Hz, 1H), 8.44 (d, J = 4.6 Hz, 1H), 7.85-7.88 (m, 1H), 7.69 (d, J =7.8 Hz, 1H), 7.34 (dd, J = 7.8, 5.0 Hz, 1H), 6.65 (s, 1H), 3.97 (dd, J = 20.1, 15.1 Hz, 2H), 3.51 (q, J = 7.0 Hz, 2H), 1.15 (t, J = 6.9 Hz, 3H)

### Synthesis Example 84: Synthesis of LEU-1143

N-[(8-hydroxy-5-nitroquinolin-7-yl) (1H-imidazol-2-yl)methyl]pentanamide (LEU-1143) was obtained in the same manner as in Synthesis Example 1 by using corresponding starting material compounds. 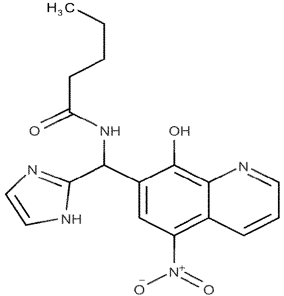
¹H-NMR (400 MHz, DMSO-d₆) δppm
9.24 (dd, J = 8.9, 1.6 Hz, 1H), 8.88-8.90 (m, 2H), 8.63 (s, 1H), 7.79 (dd, J = 8.7, 4.1 Hz, 1H), 7.09 (s, 2H), 6.59 (d, J = 7.8 Hz, 1H), 2.17-2.26 (m, 2H), 1.46-1.53 (m, 2H), 1.25 (qt, J = 7.3, 7.3 Hz, 2H), 0.84 (t, J = 7.3 Hz, 3H)
LCMS (ESI): m/z = 369.9 [M+H], RT = 1.69

### Synthesis Example 85: Synthesis of LEU-1145

N-[(8-hydroxy-5-nitroquinolin-7-yl)(pyridin-2-yl)methyl]pentanamide (LEU-1145) was obtained in the same manner as in Synthesis Example 1 by using corresponding starting material compounds. 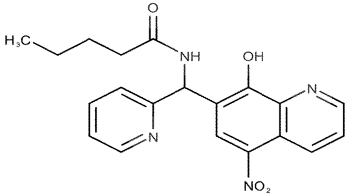
¹H-NMR (400 MHz, DMSO-d₆) δppm
9.17 (dd, J = 8.7, 1.4 Hz, 1H), 9.00 (dd, J = 4.1, 1.8 Hz, 1H), 8.96 (d, J = 8.2 Hz, 1H), 8.67 (s, 1H), 8.50 (ddd, J = 4.6, 1.8, 0.9 Hz, 1H), 7.88 (dd, J = 8.7, 4.1 Hz, 1H), 7.80 (td, J = 7.8, 1.8 Hz, 1H), 7.45 (d, J = 7.8 Hz, 1H), 7.29 (ddd, J = 7.5, 4.8, 0.9 Hz, 1H), 6.70 (d, J = 8.2 Hz, 1H), 2.26 (qt, J = 14.0, 7.3 Hz, 2H), 1.47-1.54 (m, 2H), 1.26 (qt, J = 7.3, 7.3 Hz, 2H), 0.85 (t, J = 7.3 Hz, 3H)

### Synthesis Example 86: Synthesis of LEU-1159

N-[(8-hydroxy-5-nitroquinolin-7-yl)(4-methoxyphenyl)methyl]-2-(pyridin-3-yl)acetamide (LEU-1159) was obtained in the same manner as in Synthesis Example 1 by using corresponding starting material compounds. 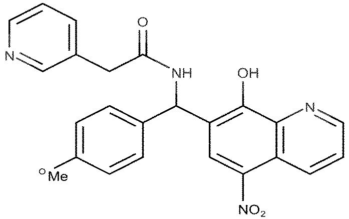
¹H-NMR (400 MHz, DMSO-d₆) δppm
9.21 (d, J = 8.2 Hz, 1H), 9.18 (dd, J = 8.7, 1.4 Hz, 1H), 9.00 (dd, J = 4.1, 1.4 Hz, 1H), 8.65 (s, 1H), 8.46 (d, J = 1.8 Hz, 1H), 8.42 (dd, J = 4.8, 1.6 Hz, 1H), 7.88 (dd, J = 8.7, 4.1 Hz, 1H), 7.69 (dt, J = 7.8, 1.8 Hz, 1H), 7.29-7.32 (m, 1H), 7.20 (d, J = 8.7 Hz, 2H), 6.89 (d, J = 8.7 Hz, 2H), 6.56 (d, J = 8.7 Hz, 1H), 3.71 (s, 3H), 3.62 (d, J = 2.7 Hz, 2H)

### Synthesis Example 87: Synthesis of LEU-1162

N-[(8-hydroxy-5-nitroquinolin-7-yl)(4-methoxyphenyl)methyl]-3-(pyridin-3-yl)propanamide (LEU-1162) was obtained in the same manner as in Synthesis Example 1 by using corresponding starting material compounds. 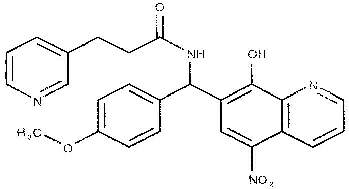
¹H-NMR (400 MHz, DMSO-d₆) δppm
9.17 (dd, J = 8.9, 1.6 Hz, 1H), 9.01 (dd, J = 4.1, 1.4 Hz, 1H), 8.89 (d, J = 8.7 Hz, 1H), 8.56 (s, 1H), 8.41 (d, J = 1.8 Hz, 1H), 8.35 (dd, J = 4.6, 1.8 Hz, 1H), 7.88 (dd, J = 9.1, 4.1 Hz, 1H), 7.60 (dt, J = 7.9, 2.1 Hz, 1H), 7.23 (dd, J = 7.8, 4.6 Hz, 1H), 7.07 (d, J = 8.7 Hz, 2H), 6.85 (d, J = 9.1 Hz, 2H), 6.59 (d, J = 8.7 Hz, 1H), 3.71 (s, 3H), 2.86 (t, J = 7.3 Hz, 2H), 2.57 (t, J = 7.3 Hz, 2H)

### Synthesis Example 88: Synthesis of LEU-1174

N-[(8-hydroxy-5-nitroquinolin-7-yl) (6-methoxypyridin-3-yl)methyl]pentanamide (LEU-1174) was obtained in the same manner as in Synthesis Example 1 by using corresponding starting material compounds. 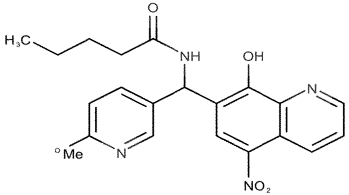
¹H-NMR (400 MHz, DMSO-d₆) δppm
9.20 (dd, J = 8.9, 1.6 Hz, 1H), 9.00 (dd, J = 4.3, 1.6 Hz, 1H), 8.91 (d, J = 8.2 Hz, 1H), 8.70 (s, 1H), 8.07 (d, J = 2.7 Hz, 1H), 7.89 (dd, J = 8.7, 4.1 Hz, 1H), 7.59 (dd, J = 8.5, 2.5 Hz, 1H), 6.78 (d, J = 8.7 Hz, 1H), 6.57 (d, J = 8.2 Hz, 1H), 3.81 (s, 3H), 2.23 (td, J = 7.3, 2.1 Hz, 2H), 1.52 (tt, J = 7.3, 7.3 Hz, 2H), 1.27 (qt, J = 7.3, 7.3 Hz, 2H), 0.86 (t, J = 7.3 Hz, 3H)

### Synthesis Example 89: Synthesis of LEU-1215

N-[(8-hydroxy-5-nitroquinolin-7-yl) (pyrimidin-5-yl)methyl]pentanamide (LEU-1215) was obtained in the same manner as in Synthesis Example 1 by using corresponding starting material compounds. 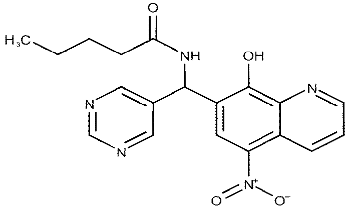
¹H-NMR (400 MHz, DMSO-d₆) δppm
9.25 (dd, J = 9.1, 1.4 Hz, 1H), 9.10 (s, 1H), 9.04 (d, J = 8.2 Hz, 1H), 8.99 (dd, J = 4.3, 1.6 Hz, 1H), 8.74 (s, 2H), 8.71 (s, 1H), 7.91 (dd, J = 9.1, 4.1 Hz, 1H), 6.61 (d, J = 8.2 Hz, 1H), 2.27 (t, J = 7.3 Hz, 2H), 1.54 (tt, J = 7.3, 7.3 Hz, 2H), 1.28 (qt, J = 7.3, 7.3 Hz, 2H), 0.87 (t, J = 7.3 Hz, 3H)

### Synthesis Example 90: Synthesis of LEU-1272

N-{[8-hydroxy-5-(methylsulfonyl)quinolin-7-yl](4-methoxyphenyl)methyl}pentanamide (LEU-1272) was obtained in the same manner as in Synthesis Example 1 by using corresponding starting material compounds. 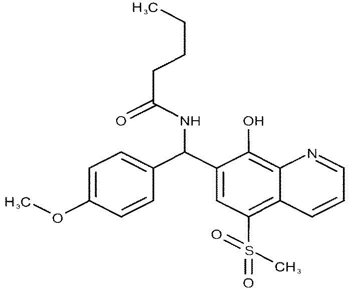
¹H-NMR (400 MHz, DMSO-d₆) δppm
9.00 (s, 1H), 8.95 (d, J = 7.8 Hz, 1H), 8.84 (d, J = 6.9 Hz, 1H), 8.27 (s, 1H), 7.80 (d, J = 5.0 Hz, 1H), 7.16 (d, J = 8.7 Hz, 2H), 6.88 (d, J = 8.7 Hz, 2H), 6.58 (d, J = 7.3 Hz, 1H), 3.71 (s, 3H), 3.27 (s, 3H), 2.21 (t, J = 7.3 Hz, 2H), 1.50 (tt, J = 7.3, 7.3 Hz, 2H), 1.26 (qt, J = 7.3, 7.3 Hz, 2H), 0.86 (t, J = 7.3 Hz, 3H)

### Synthesis Example 91: Synthesis of LEU-1277

Methyl4-{[(8-hydroxy-5-nitroquinolin-7-yl)(4-methoxyphenyl)methyl]amino}-4-oxobutanoate (LEU-1277) was obtained in the same manner as in Synthesis Example 1 by using corresponding starting material compounds. 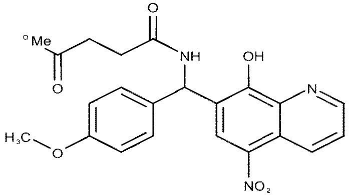
¹H-NMR (400 MHz, DMSO-d₆) δppm
9.17 (dd, J = 8.9, 1.6 Hz, 1H), 9.01 (dd, J = 4.3, 1.6 Hz, 1H), 8.95 (d, J = 8.7 Hz, 1H), 8.67 (s, 1H), 7.88 (dd, J = 9.1, 4.1 Hz, 1H), 7.19 (d, J = 8.7 Hz, 2H), 6.88 (d, J = 8.7 Hz, 2H), 6.59 (d, J = 8.7 Hz, 1H), 3.71 (s, 3H), 3.54 (s, 3H), 2.53-2.53 (m, 4H)

### Synthesis Example 92: Synthesis of LEU-1279

2-hydroxy-N-[(8-hydroxy-5-nitroquinolin-7-yl) (4-methoxyphenyl)methyl]-2-methylpropanamide (LEU-1279) was obtained in the same manner as in Synthesis Example 1 by using corresponding starting material compounds. 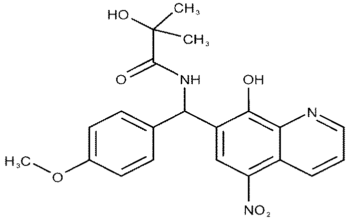
¹H-NMR (400 MHz, DMSO-d₆) δppm
9.19 (dd, J = 8.9, 1.6 Hz, 1H), 8.98 (dd, J = 4.1, 1.4 Hz, 1H), 8.75 (s, 1H), 8.52 (d, J = 8.7 Hz, 1H), 7.88 (dd, J = 8.9, 4.3 Hz, 1H), 7.24 (d, J = 8.7 Hz, 2H), 6.88 (d, J = 8.7 Hz, 2H), 6.46 (d, J = 8.7 Hz, 1H), 5.54 (s, 1H), 3.71 (s, 3H), 1.29 (d, J = 16.0 Hz, 6H)

### Synthesis Example 93: Synthesis of LEU-1282

Methyl5-{[(8-hydroxy-5-nitroquinolin-7-yl)(4-methoxyphenyl)methyl]amino}-5-oxopentanoate (LEU-1282) was obtained in the same manner as in Synthesis Example 1 by using corresponding starting material compounds. 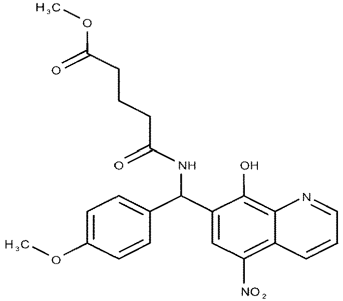
¹H-NMR (400 MHz, DMSO-d₆) δppm
9.18 (dd, J = 8.7, 1.4 Hz, 1H), 9.00 (dd, J = 4.3, 1.6 Hz, 1H), 8.88 (d, J = 8.7 Hz, 1H), 8.65 (s, 1H), 7.88 (dd, J = 8.7, 4.1 Hz, 1H), 7.19 (d, J = 8.7 Hz, 2H), 6.88 (d, J = 8.7 Hz, 2H), 6.59 (d, J = 8.2 Hz, 1H), 3.71 (s, 3H), 3.57 (s, 3H), 2.24-2.32 (m, 4H), 1.77 (tt, J = 7.3, 7.3 Hz, 2H)

### Synthesis Example 94: Synthesis of LEU-1353

N-{[4-(1H-pyrazol-1-yl)phenyl][8-hydroxy-5-(methylsulfonyl)quinolin-7-yl]methyl}pentanamide (LEU-1353) was obtained in the same manner as in Synthesis Example 1 by using corresponding starting material compounds. 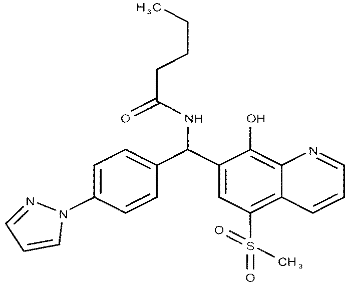
¹H-NMR (400 MHz, DMSO-d₆) δppm
8.94-9.02 (m, 3H), 8.45 (d, J = 2.7 Hz, 1H), 8.27 (s, 1H), 7.77-7.83 (m, 3H), 7.72 (d, J = 1.8 Hz, 1H), 7.36 (d, J = 8.2 Hz, 2H), 6.68 (d, J = 8.2 Hz, 1H), 6.52 (t, J = 2.1 Hz, 1H), 3.27 (s, 3H), 2.24 (t, J = 7.3 Hz, 2H), 1.52 (tt, J = 7.3, 7.3 Hz, 2H), 1.27 (qt, J = 7.3, 7.3 Hz, 2H), 0.87 (t, J = 7.3 Hz, 3H)

### Synthesis Example 95: Synthesis of LEU-1354

N-[(5-fluoro-8-hydroxyquinolin-7-yl)(4-methoxyphenyl)methyl]pentanamide (LEU-1354) was obtained in the same manner as in Synthesis Example 1 by using corresponding starting material compounds. 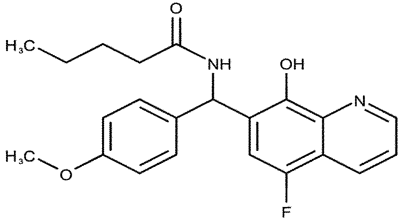
¹H-NMR (400 MHz, DMSO-d₆) δppm
9.93 (s, 1H), 8.94 (dd, J = 4.1, 1.8 Hz, 1H), 8.66 (d, J = 8.7 Hz, 1H), 8.42 (dd, J = 8.5, 1.6 Hz, 1H), 7.65 (dd, J = 8.5, 4.3 Hz, 1H), 7.41 (d, J = 11.0 Hz, 1H), 7.15 (d, J = 8.7 Hz, 2H), 6.87 (d, J = 8.7 Hz, 2H), 6.65 (d, J = 8.7 Hz, 1H), 3.70 (s, 3H), 2.21 (t, J = 7.3 Hz, 2H), 1.50 (tt, J = 7.3, 7.3 Hz, 2H), 1.26 (qt, J = 7.3, 7.3 Hz, 2H), 0.86 (t, J = 7.3 Hz, 3H)

### Synthesis Example 96: Synthesis of LEU-1355

N-[(5-bromo-8-hydroxyquinolin-7-yl)(4-methoxyphenyl)methyl]pentanamide (LEU-1355) was obtained in the same manner as in Synthesis Example 1 by using corresponding starting material compounds. 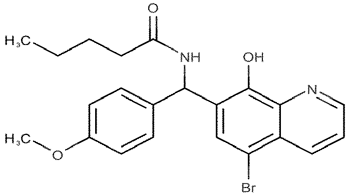
¹H-NMR (400 MHz, DMSO-d₆) δppm
10.28 (bs, 1H), 8.93 (dd, J = 4.1, 1.4 Hz, 1H), 8.70 (d, J = 8.7 Hz, 1H), 8.41 (dd, J = 8.5, 1.6 Hz, 1H), 7.87 (s, 1H), 7.72 (dd, J = 8.7, 4.1 Hz, 1H), 7.15 (d, J = 8.7 Hz, 2H), 6.87 (d, J = 8.7 Hz, 2H), 6.62 (d, J = 8.7 Hz, 1H), 3.70 (s, 3H), 2.21 (t, J = 7.5 Hz, 2H), 1.51 (tt, J = 7.3, 7.3 Hz, 2H), 1.26 (qt, J = 7.3, 7.3 Hz, 2H), 0.87 (t, J = 7.3 Hz, 3H)

### Synthesis Example 97: Synthesis of LEU-1441

(E)-N-[1-(8-hydroxy-5-nitroquinolin-7-yl)-3-(pyridin-3-yl)aryl]pentanamide (LEU-1441) was obtained in the same manner as in Synthesis Example 1 by using corresponding starting material compounds. 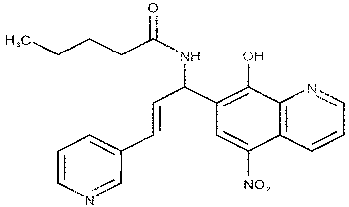
¹H-NMR (400 MHz, DMSO-d₆) δppm
9.20 (dd, J = 8.9, 1.6 Hz, 1H), 9.03 (dd, J = 4.1, 1.4 Hz, 1H), 8.80 (d, J = 8.2 Hz, 1H), 8.66 (s, 1H), 8.59 (d, J = 1.8 Hz, 1H), 8.42 (dd, J = 4.8, 1.6 Hz, 1H), 7.87-7.92 (m, 2H), 7.33 (dd, J = 8.0, 4.8 Hz, 1H), 6.62 (dd, J = 16.0, 5.5 Hz, 1H), 6.51 (d, J = 16.0 Hz, 1H), 6.21 (t, J = 6.4 Hz, 1H), 2.23 (t, J = 7.3 Hz, 2H), 1.52 (tt, J = 7.3, 7.3 Hz, 2H), 1.27 (qt, J = 7.3, 7.3 Hz, 2H), 0.86 (t, J = 7.3 Hz, 3H)

### Synthesis Example 98: Synthesis of LEU-1466

N-[1-(8-hydroxy-5-nitroquinolin-7-yl)-2-methylaryl]pentanamide (LEU-1466) was obtained in the same manner as in Synthesis Example 1 by using corresponding starting material compounds. 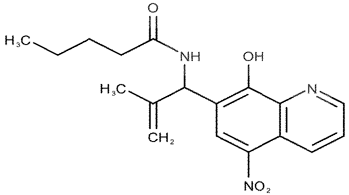
¹H-NMR (400 MHz, DMSO-d₆) δppm
9.18 (dd, J = 8.9, 1.6 Hz, 1H), 9.02 (dd, J = 4.1, 1.4 Hz, 1H), 8.56 (d, J = 9.1 Hz, 2H), 7.89 (dd, J = 8.7, 4.1 Hz, 1H), 5.98 (d, J = 8.7 Hz, 1H), 4.95 (d, J = 1.4 Hz, 1H), 4.78 (s, 1H), 2.14-2.22 (m, 2H), 1.71 (s, 3H), 1.45-1.53 (m, 2H), 1.25 (qt, J = 7.3, 7.3 Hz, 2H), 0.85 (t, J = 7.3 Hz, 3H)

### Synthesis Example 99: Synthesis of LEU-1470

3-(8-hydroxy-5-nitroquinolin-7-yl)isoindolin-1-one (LEU-1470) was obtained in the same manner as in Synthesis Example 1 by using corresponding starting material compounds. 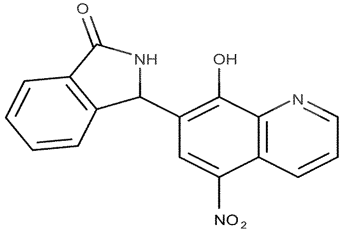
¹H-NMR (400 MHz, DMSO-d₆) δppm
9.21 (dd, J = 9.1, 1.4 Hz, 1H), 9.11 (s, 1H), 9.05 (dd, J = 4.1, 1.4 Hz, 1H), 8.19 (s, 1H), 7.93 (dd, J = 8.9, 4.3 Hz, 1H), 7.75 (dd, J = 6.4, 0.9 Hz, 1H), 7.49-7.57 (m, 3H), 6.31 (s, 1H)

### Synthesis Example 100: Synthesis of LEU-1765

N-[(8-hydroxy-5-nitroquinolin-7-yl)(4-methoxyphenyl)methyl]formamide (LEU-1765) was obtained in the same manner as in Synthesis Example 1 by using corresponding starting material compounds. 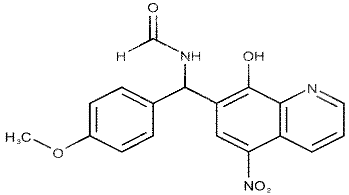
¹H-NMR (400 MHz, DMSO-d₆) δppm
9.18-9.22 (m, 2H), 9.00 (d, J = 4.1 Hz, 1H), 8.65 (s, 1H), 8.19 (s, 1H), 7.89 (dd, J = 8.9, 4.3 Hz, 1H), 7.22 (d, J = 8.7 Hz, 2H), 6.89 (d, J = 8.7 Hz, 2H), 6.64 (d, J = 8.7 Hz, 1H), 3.71 (s, 3H)

### Synthesis Example 101: Synthesis of LEU-1766

Concentrated hydrochloric acid (1 mL) was added dropwise to a suspension of LEU-1765 (120 mg, 0.34 mmol) in methanol (1 mL), and the mixture was heated at 55°C for 3 hours. After the reaction liquid was allowed to reach room temperature, a 6N aqueous sodium hydroxide solution (2 mL) was added to concentrate it, followed by neutralizing the concentrated liquid with 1N hydrochloric acid and a sodium hydrogen carbonate solution. The formed precipitate was collected by filtration, washed with water, and then dried in vacuum to obtain 7-[amino(4-methoxyphenyl)methyl]-5-nitroquinolin-8-ol (LEU-1766) (amount: 110 mg, yield: 100%). 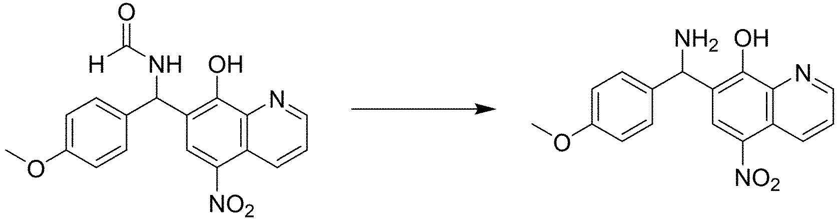
¹H-NMR (400 MHz, DMSO-d₆) δppm
9.35 (dd, J = 8.7, 1.4 Hz, 1H), 8.62 (dd, J = 4.1, 1.4 Hz, 1H), 8.60 (bs, 2H), 8.29 (s, 1H), 7.55 (dd, J = 8.7, 4.1 Hz, 1H), 7.49 (d, J = 8.7 Hz, 2H), 6.99 (d, J = 8.7 Hz, 2H), 5.60 (s, 1H), 3.76 (s, 3H)

### Example 2: Test for Measuring cMLCK Activity Value of 8-Hydroxyquinoline Compounds

The cMLCK activity of each of the 8-hydroxyquinoline compounds obtained in Synthesis Examples 1 to 101 was measured.

Additionally, the cMLCK activity of the following compounds was also measured:
LEU-1463: N-[(8-hydroxy-6-methyl-5-nitroquinolin-7-yl) (6-morpholinopyridin-3-yl)methyl]pentanamide
LEU-0944: 7-[(3-nitrophenyl) (thiazol-2-ylamino)methyl]quinolin-8-ol
LEU-0993: N-[(8-hydroxy-5-nitroquinolin-7-yl)(4-methoxyphenyl)methyl]propionamide
LEU-0997: 5-chloro-7-[(4-methoxy-3-nitrophenyl) (pyridin-2-ylamino)methyl]quinolin-8-ol
LEU-1008: 7-[phenyl(thiazol-2-ylamino)methyl]quinolin-8-ol
LEU-1220: N-[(8-hydroxy-5-nitroquinolin-7-yl)(4-methoxyphenyl)methyl]isobutyramide
LEU-0942: 7-{ (4-fluorophenyl) [(3-hydroxypyridin-2-yl)amino]methyl}quinolin-8-ol
LEU-0943: 7-{piperidin-1-yl[4-(trifluoromethyl)phenyl]methyl}quinolin-8-ol
LEU-0945: 7-[(2-hydroxyphenyl) (thiazol-2-ylamino)methyl]quinolin-8-ol
LEU-0990: N-[(8-hydroxy-5-nitroquinolin-7-yl)(4-methoxyphenyl)methyl]acetamide
LEU-0998: 5-chloro-7-[(pyridin-2-ylamino) (pyridin-3-yl)methyl]quinolin-8-ol
LEU-0999: 7-[(4-benzylpiperidin-1-yl)methyl]-5-chloroquinolin-8-ol
LEU-1018: N-[(5-chloro-8-hydroxyquinolin-7-yl) (4-methoxyphenyl)methyl]pentanamide
LEU-1128: N-[(8-hydroxyquinolin-7-yl)(4-methoxyphenyl)methyl]pentanamide

### Method for Measuring cMLCK Activity of 8-Hydroxyquinoline Compounds

The cMLCK activation action by 8-hydroxyquinoline compounds was measured by using the ADP-Glo kinase assay (Promega) according to the manufacturer's protocol.

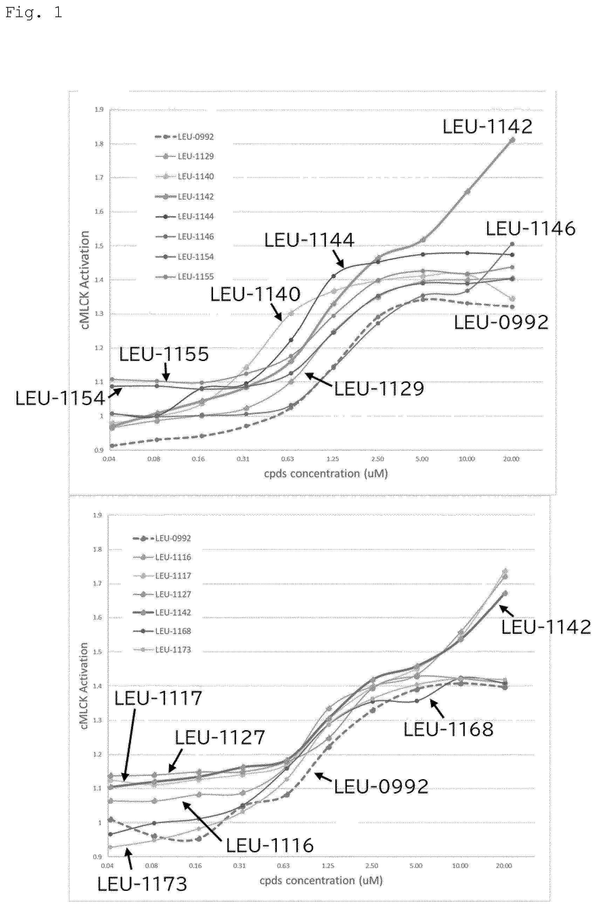

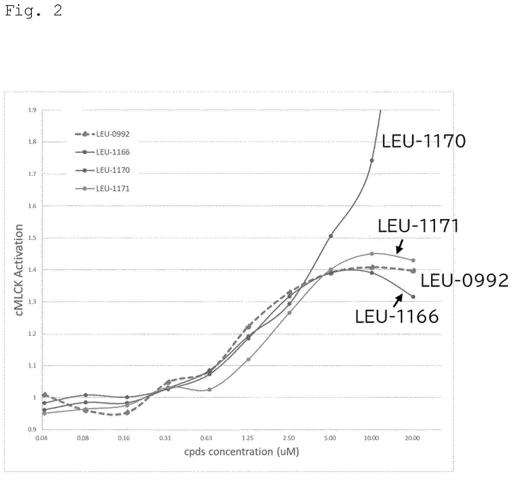

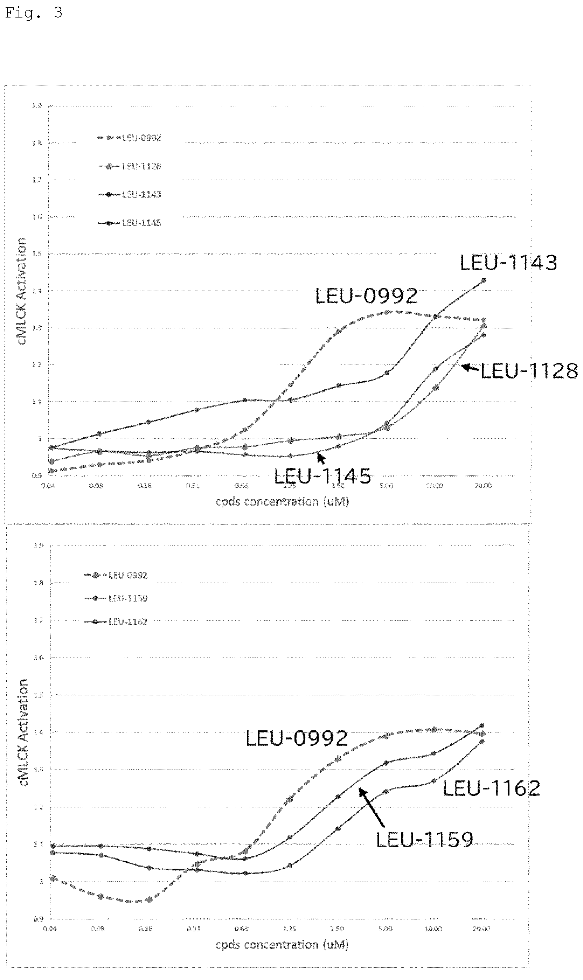

As shown in Fig. 1, the compounds of Synthesis Examples 2 to 59 exhibited stronger cMLCK activity than the compound of Synthesis Example 1. As shown in Fig. 2, the compounds of Synthesis Examples 60 to 79, LEU-0944, LEU-0993, LEU-0997, LEU-1008, and LEU-1220 exhibited cMLCK activity equivalent to that of the compound of Synthesis Example 1. As shown in Fig. 3, the compounds of Synthesis Examples 80 to 101, LEU-0942, LEU-0943, LEU-0945, LEU-0990, LEU-0998, LEU-0999, LEU-1018, and LEU-1128 showed cMLCK activity weaker than the compound of Synthesis Example 1.

### Example 3: Study of Therapeutic Effect of Compound of Present Invention on Heart Failure

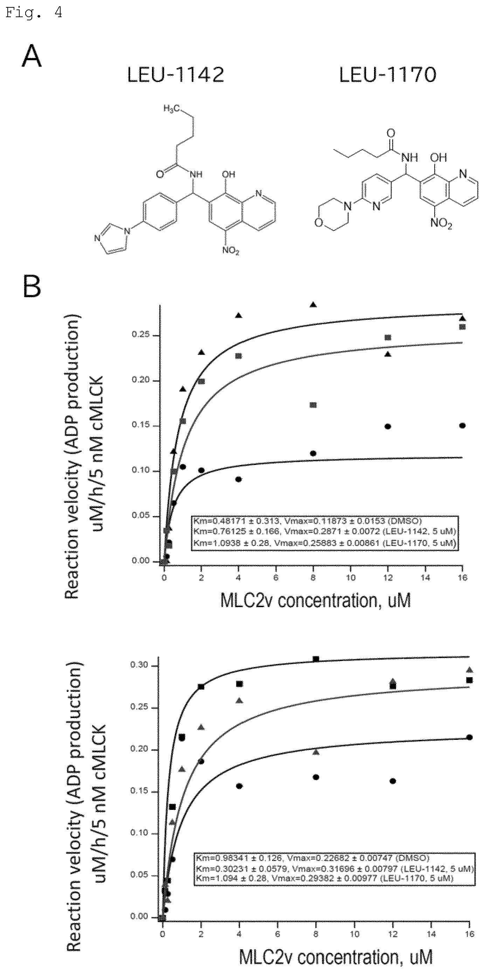

Purchased purified human cMLCK (BTN-caMLCK, #02-460-20N, Carna Bioscience) as well as in-house purified calmodulin (NM_006888) and cardiac myosin regulatory light chain (MLC2v) (NM_000432) were used to perform phosphorylation to thereby establish an in vitro kinase assay system for measuring cMLCK activity according to the ADP-Glo assay (Promega) or Kumagai method. This system was then applied to high-throughput screening (HTS). The HTS was performed by using multiple domestic compound libraries (compound concentration: 10 µM), and structural development of candidate compounds was performed, thereby developing cMLCK activators based on the 8-hydroxyquinoline (8HQ) skeleton. Fig. 4 shows the results of an enzymatic study (Michaelis-Menten kinetics) using two 8HQ compounds (LEU-1142 and LEU-1170) that were used in the early stages. These compounds did not affect Kₘ but increased Vₘₐₓ by 1.5- to 2-fold. These results suggest that the cMLCK activation effect of these compounds is due to allosteric action.

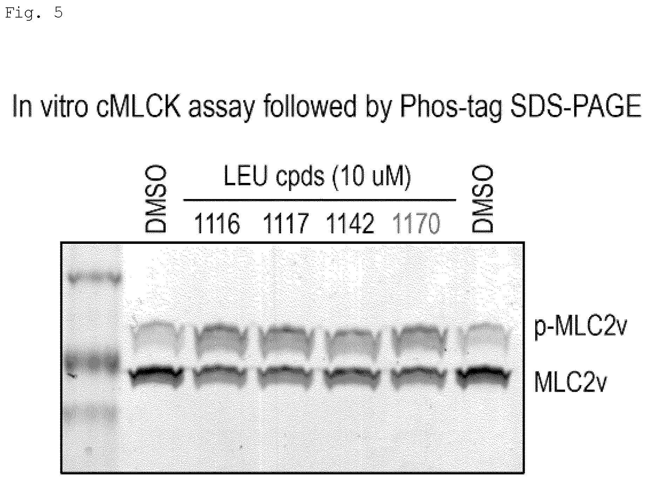

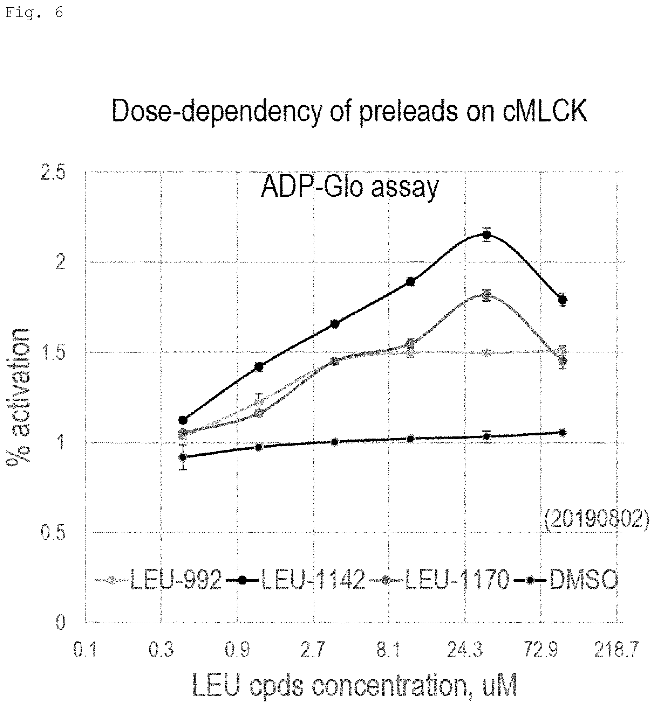

The two 8HQ compounds (LEU-1142 and LEU-1170) selected by the HTS were confirmed to increase the phosphorylation of MLC2v by using phos-tag PAGE (Phos-tag Acrylamide, Wako) (Fig. 5).
Additionally, the cMLCK activation action of these compounds was confirmed to be dose-dependent by using the ADP-Glo assay (Fig. 6).

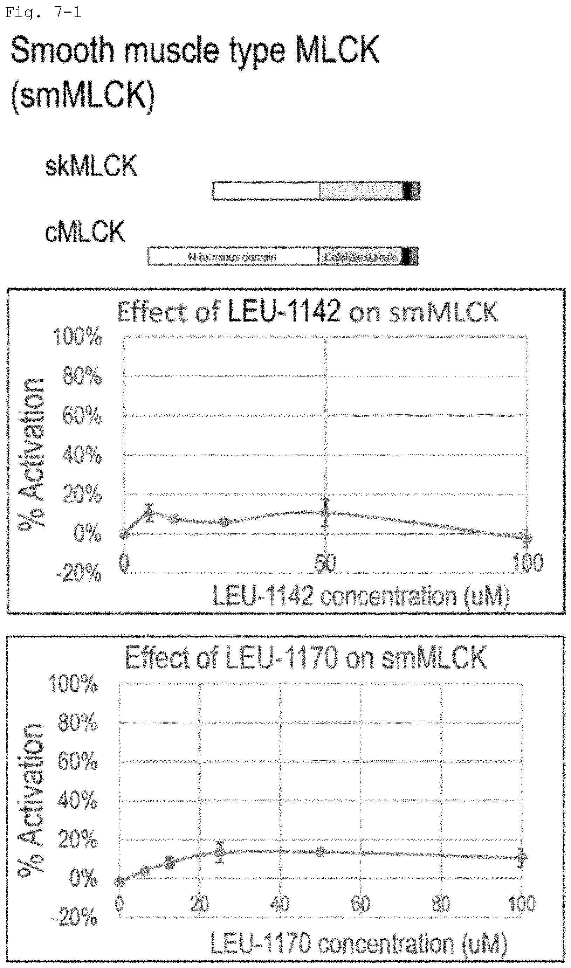

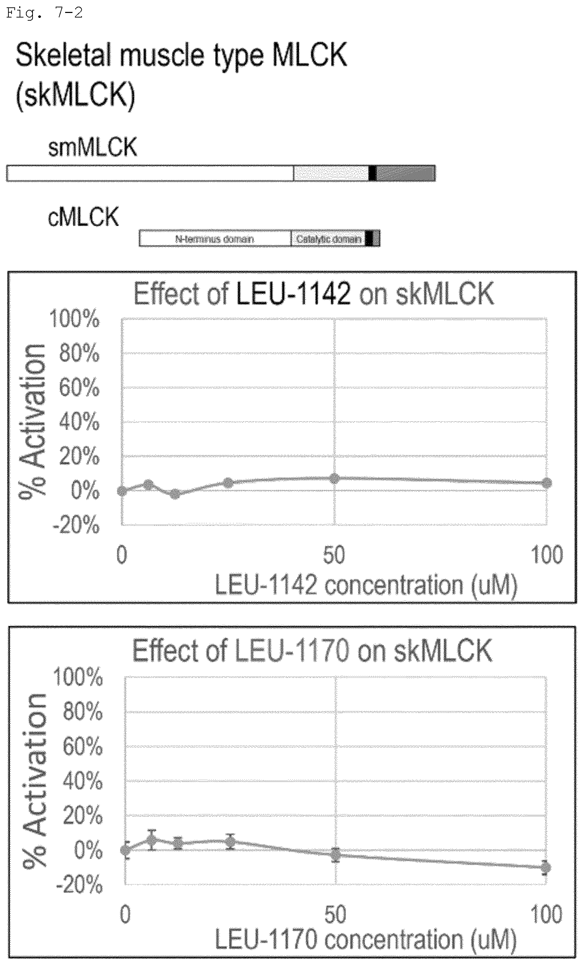

Next, the specificity of the two 8HQ compounds (LEU-1142 and LEU-1170) for human smooth muscle-type MLCK (smMLCK, NM_053025) and human skeletal muscle-type MLCK (skMLCK, NM_033118), which are cMLCK family proteins, was examined (Figs. 7-1 and 7-2). In-vitro kinase reactions were performed using smMLCK and its substrate MYL9 (NM_006097), and skMLCK and its substrate MYLPF (NM_013292), which had been all purified in-house, and their activity was measured according to the ADP-Glo assay. Neither compound showed specificity for smMLCK or skMLCK. This demonstrates that these compounds have high specificity for cMLCK.

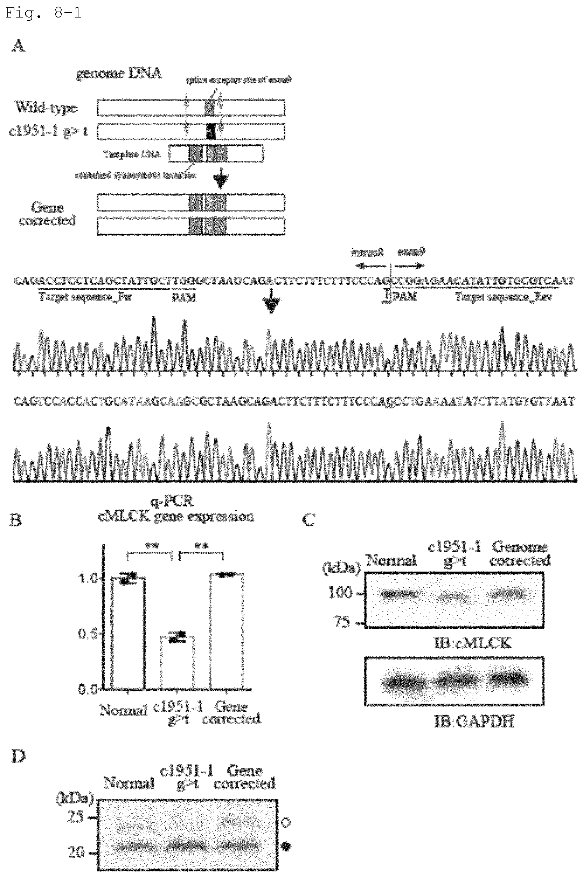

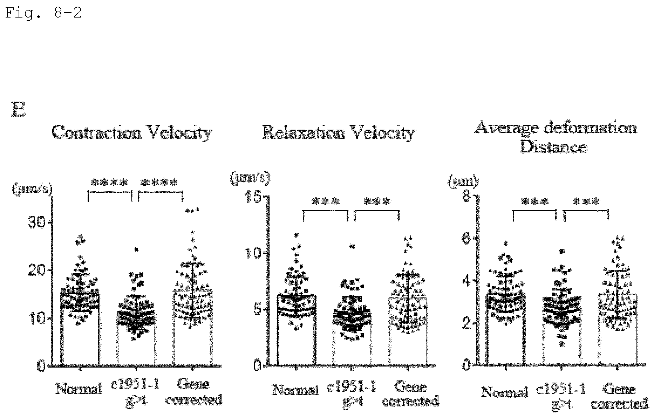

iPS cardiomyocytes were established from a patient with a heterozygous cMLCK gene (MYLK3, NM_182493.3) mutation (c.1951-1G>T; p.P639Vfs*15) (MYLK3^{+/fs}-iPSC-CMs). Additionally, iPS cardiomyocytes with this mutation normalized using the CRISPR/Cas9 system (gene-corrected) were established (MYLK3^{+/correct}-iPSC-CMs) (Fig. 8-1A). Droplet digital PCR (ddPCR) demonstrated that while mutant cells expressed only about half the level of cMLCK mRNA compared to wild-type iPS cardiomyocytes (MYLK3^{+/+}-iPSC-CMs), gene correction normalized the expression (Fig. 8-1B). Furthermore, Western blotting revealed that while mutant cells (MYLK3^{+/fs}-iPSC-CMs) expressed only about half the amount of cMLCK protein compared to wild-type iPS cardiomyocytes (MYLK3^{+/+}-iPSC-CMs), gene correction (MYLK3^{+/correct}-iPSC-CMs) normalized the expression (Fig. 8-1C). Furthermore, phos-tag SDS-PAGE revealed that while MLC2v phosphorylation was decreased in mutant cells, gene correction normalized MLC2v phosphorylation (Fig. 8-1D). Next, each type of iPSC-CMs was seeded and cultured on laminin-coated glass dishes. Then, the contraction velocity, relaxation velocity, and developed tension of the cardiomyocytes were measured by using a cell motion imaging system (SI8000, Sony) with 1 Hz pacing. The results indicated that while mutant cells showed decreased contraction velocity, relaxation velocity, and developed tension, gene correction normalized these parameters (Fig. 8-2E).

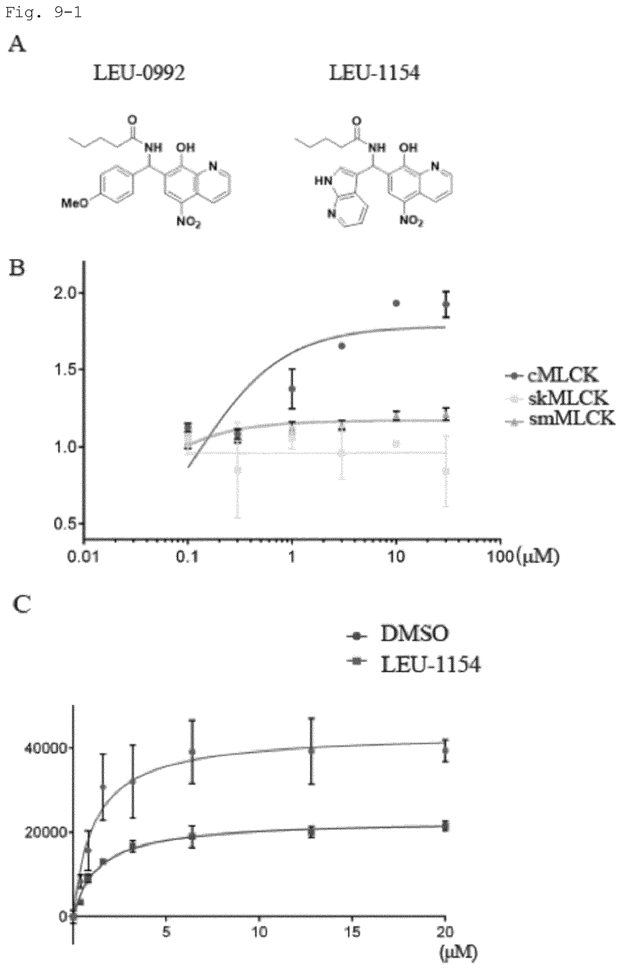

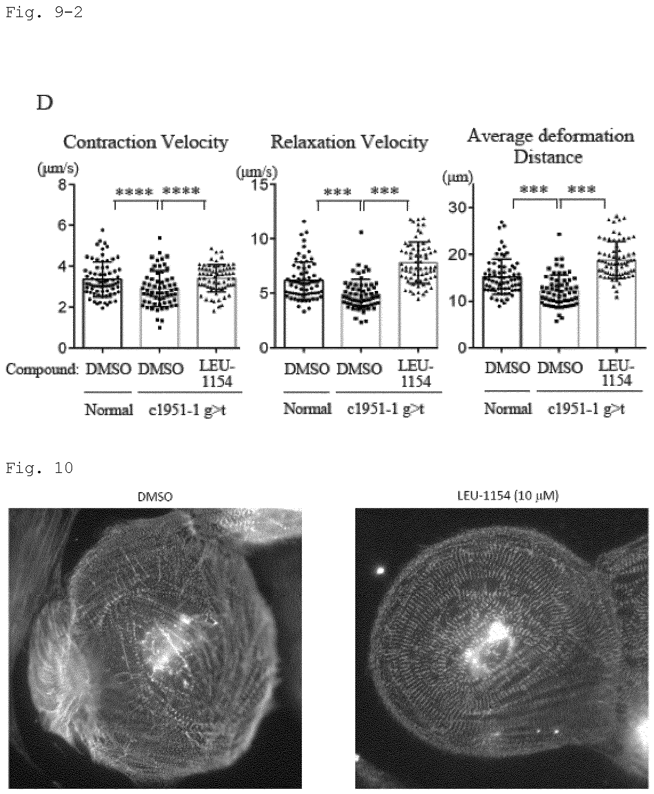

A compound (LEU-1154, Fig. 9-1A) that showed no problems on iPS cardiomyocytes in a toxicity test was selected, and the following experiment was performed using the mutant iPS cardiomyocytes (MYLK3^{+/fs}-iPSC-CMs) described above. Fig. 9-1B shows the dose-dependent activation action and specificity of LEU-1154 (activity was measured according to the ADP-Glo assay after in vitro kinase reaction was performed). These results indicate that the cMLCK activation action by LEU-1154 is dose-dependent, and that LEU-1154 has high specificity for cMLCK. Furthermore, an enzymatic study demonstrated that LEU-1154 had activity equivalent to that of the compound mentioned above (Fig. 9-1C). LEU-1154 was further administered to the mutant iPS cardiomyocytes (MYLK3^{+/fs}-iPSC-CMs), followed by measuring the contraction velocity, relaxation velocity, and developed tension with a cell motion imaging system (SI8000, Sony). Administration of LEU-1154 (10 µM) normalized the contraction velocity, relaxation velocity, and developed tension of mutant cells (Fig. 9-2D) .

Mutant iPS cardiomyocytes (MYLK3^{+/fs}-iPSC-CMs) on day 35 after the start of differentiation into cardiac muscle were treated with LEU-1154 (10 µM) for 7 days, fixed with 4% paraformaldehyde, permeabilized with 0.1% Triton X-100. Thereafter, the cells were reacted with α-actinin antibody (ab9465, Abcam) and a secondary antibody (Fluro 488), followed by observation of sarcomere structures with a ZEISS Axio Observer Z1 inverted fluorescence microscope (ZEISS Corporation, Tokyo, Japan). Fig. 10 shows the results of observing the sarcomere structure-improving action. In mutant iPS cardiomyocytes with reduced expression of cMLCK, sparse myocardial sarcomere structures were observed. However, administration of LEU-1154 (10 µM) was shown to promote the formation of sarcomere structures.

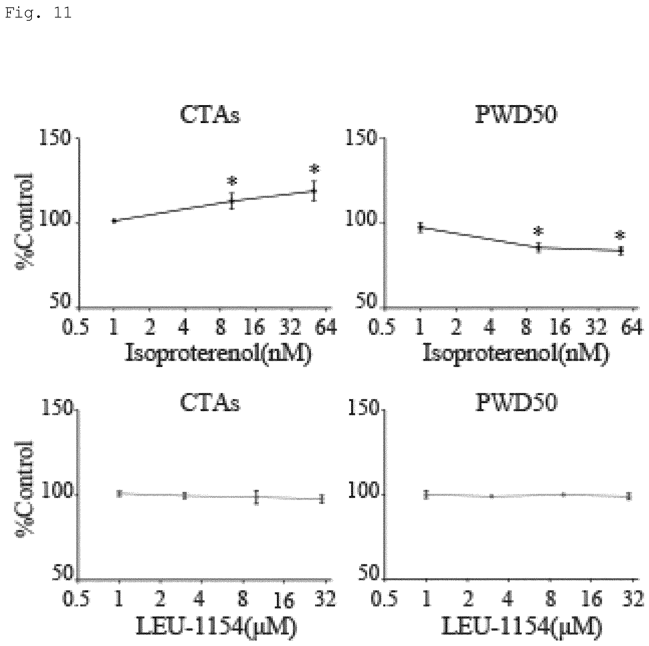

Wild-type iPSC-CMs were seeded on a 96-well plate at a density of 5 × 10⁴ cells/well and cultured in DMEM with 10% FBS for 24 hours. Subsequently, Cal-520 (ab171868, Abcam) was then added to a final concentration of 5 µM, and the cells were cultured for an additional hour. After removing the medium and washing with HHBS buffer, the buffer was replaced with FluoroBrite DMEM containing various concentrations of LEUO-1154, isoproterenol (positive control group: catecholamine stimulation), or DMSO (negative control group). Intracellular calcium dynamics was then measured with pacing by using an FDSS/µCell kinetic plate imager (Hamamatsu Photonics K.K.). Fig. 11 shows the results. While isoproterenol enhanced calcium dynamics, LEU-1154 had no effect on calcium dynamics.

The results above revealed that the developed LEU compounds (8-hydroxyquinoline compounds) allosterically activate cMLCK, enhance contractility without affecting calcium dynamics, and have the action of promoting sarcomere organization. Therefore, the 8-hydroxyquinoline compounds of the present invention can be used as a novel myotrope (for directly enhancing sarcomere contractility).

## Claims

1. A cMLCK activator comprising an 8-hydroxyquinoline compound represented by the following formula (I) or a pharmaceutically acceptable salt thereof, or a solvate thereof, 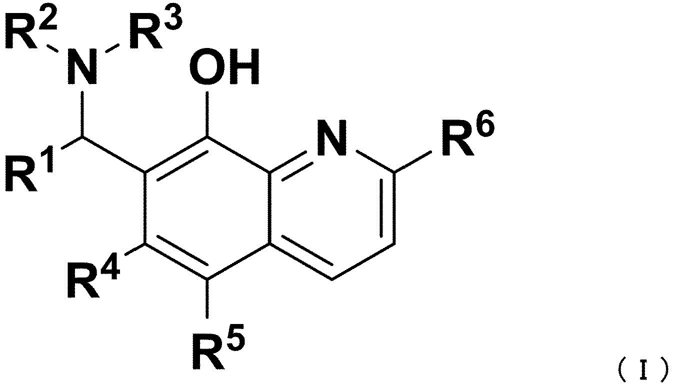 wherein
R¹ represents hydrogen, aryl, a heterocyclic group, C₁₋₆ alkyl optionally substituted with aryl, C₃₋₈ cycloalkyl, C₂₋₆ alkenyl optionally substituted with aryl or heteroaryl, C₃₋₈ cycloalkenyl, or C₂₋₆ alkynyl optionally substituted with aryl;
R² represents hydrogen, R⁷CO-, or heteroaryl;
R³ represents hydrogen;
wherein R⁷ represents hydrogen, C₁₋₆ alkyl optionally substituted with aryl or heteroaryl, C₁₋₆ alkylamino, C₃₋₈ cycloalkyl that may contain oxygen in a cycloalkyl ring, C₃₋₈ cycloalkylamino, C₁₋₆ alkoxy optionally substituted with aryl, C₃₋₈ cycloalkoxy, C₁₋₃ alkoxy-C₁₋₃ alkyl, C₁₋₄ alkoxycarbonyl C₁₋₄ alkyl, aryl, aryloxy, pyridyl, or amino;
R¹ and R², taken together with the nitrogen to which they are attached, may form an oxindole ring; and
R² and R³, taken together with the nitrogen to which they are attached, via -CH₂CH₂X(R⁸)CH₂CH₂-, may form a ring, wherein X represents -CH or nitrogen, and R⁸ represents hydrogen, aryl C₁₋₆ alkyl, or aryl C₁₋₆ alkoxycarbonyl;
R⁴ represents hydrogen, C₁₋₆ alkyl, or halogen;
R⁵ represents hydrogen, nitro, halogen, or sulfonyl; and
R⁶ represents hydrogen or C₁₋₆ alkyl.

2. A pharmaceutical composition for treating heart disease, comprising the cMLCK activator according to claim 1.

3. The pharmaceutical composition according to claim 2, for use as a therapeutic agent for heart failure.

4. The pharmaceutical composition according to claim 2, for use as an inotropic agent.

5. The pharmaceutical composition according to claim 2, for use as a drug for enhancing myocardial contractility.

6. The pharmaceutical composition according to claim 2, for use as a drug for improving cardiac function.

7. The pharmaceutical composition according to claim 2, for use in promoting organization of a sarcomere structure.

8. An 8-hydroxyquinoline compound represented by the following formula (Ia) or a pharmaceutically acceptable salt thereof, or a solvate thereof, 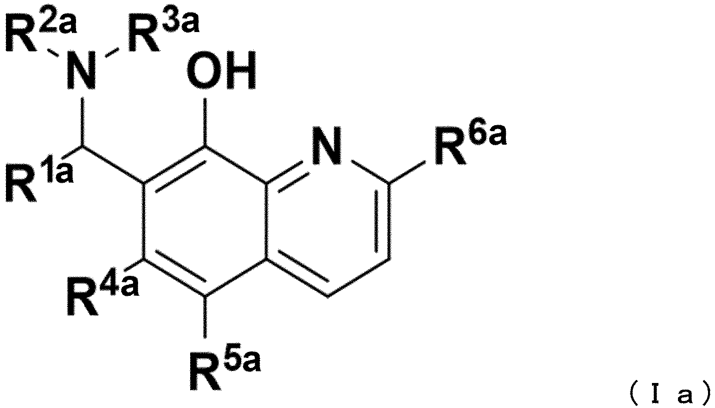 wherein
R^{1a} represents hydrogen, aryl, a heterocyclic group, aryl C₁₋₆ alkyl, C₃₋₈ cycloalkyl, C₂₋₆ alkenyl optionally substituted with aryl or heteroaryl, C₃₋₈ cycloalkenyl, or aryl C₂₋₆ alkynyl;
R^{2a} represents R^{7a}CO- or thiazolyl;
R^{3a} represents hydrogen;
wherein R^{7a} represents hydrogen, C₁₋₆ alkyl optionally substituted with aryl or heteroaryl, C₁₋₆ alkylamino, C₃₋₈ cycloalkyl that may contain oxygen in a cycloalkyl ring, C₃₋₈ cycloalkylamino, C₁₋₆ alkoxy optionally substituted with aryl, C₃₋₈ cycloalkoxy, C₁₋₃ alkoxy-C₁₋₃ alkyl, C₁₋₄ alkoxycarbonyl C₁₋₄ alkyl, aryloxy, pyridyl, or amino;
R^{1a} and R^{2a}, taken together with the nitrogen to which they are attached, may form an oxindole ring; and
R^{2a} and R^{3a}, taken together with the nitrogen to which they are attached, via -CH₂CH₂X(R⁸)CH₂CH₂-, may form a ring, wherein X represents nitrogen, and R⁸ represents aryl C₁₋₆ alkoxycarbonyl;
R^{4a} represents hydrogen, C₁₋₆ alkyl, or halogen;
R^{5a} represents hydrogen, nitro, halogen, or sulfonyl; and
R^{6a} represents hydrogen or C₁₋₆ alkyl.
